(19) Europäisches Patentamt
European Patent Office
Office européen des brevets

(11) EP 3 798 633 A1

(12) EUROPEAN PATENT APPLICATION

(43) Date of publication:
31.03.2021 Bulletin 2021/13

(51) Int Cl.:
*G01N 33/574* (2006.01)

(21) Application number: 19382839.9

(22) Date of filing: 30.09.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Fundació Institut de Recerca Biomèdica IRB (Barcelona)
  08028 Barcelona (ES)
- Universitat Pompeu Fabra
  08002 Barcelona (ES)
- Institució Catalana de Recerca I Estudis Avançats
  08010 Barcelona (ES)

(72) Inventors:
- BATLLE GÓMEZ, Eduard
  08010 Barcelona (ES)
- SANCHO SUILS, Elena
  08028 Barcelona (ES)
- ROSSELL RIBERA, David
  08002 Barcelona (ES)
- BERENGUER LLERGO, Antonio
  08028 Barcelona (ES)
- STEPHAN-OTTO ATTOLINI, Camille
  08028 Barcelona (ES)

(74) Representative: Hoffmann Eitle
Hoffmann Eitle S.L.U.
Paseo de la Castellana 140, 3a planta
Edificio LIMA
28046 Madrid (ES)

# (54) PREDICTIVE BIOMARKERS FOR TREATMENT OF A CANCER PATIENT WITH TGF-BETA SIGNALING PATHWAY INHIBITORS

(57) The present invention relates to a single non-cell specific biomarker signature useful to identify cancer patients having tumors with a TGFβ-activated microenvironment. This signature would enable to identify those cancer patients who will most likely benefit from treatment with an inhibitor of the TGFβ signaling pathway. In addition, said biomarker signature has been shown to be useful for various tumor types (a pan-tissue signature). It further provides related prognostic methods, methods of patient classification and selection for treatment with an inhibitor of the TGFβ signaling pathway, methods for patient monitoring or evaluation of response, second medical uses, diagnostic kits and uses thereof.

EP 3 798 633 A1

SEQUENCE LISTING

<110> Fundació Institut de Recerca Biomèdica (IRB Barcelona)
INSTITUCIÓ CATALANA DE RECERCA I ESTUDIS AVANÇATS
Universitat Pompeu Fabra

<120> PREDICTIVE BIOMARKERS FOR TREATMENT OF A CANCER PATIENT WITH TGF-beta SIGNALING PATHWAY INHIBITORS

<130> 901 803

<160> 24

<170> PatentIn version 3.5

<210> 1
<211> 998
<212> DNA
<213> Homo sapiens

<220>
<221> CRYAB transcript variant 1
<222> (1)..(998)

<400> 1
```
aggaggagga ggcaccaaac ggcctgggtg gatagaaggg ggacaaggag gcacacccag      60

gccggcaaag agcaggtatc agcactgcaa gcaccaagtg tgtcttgagc tcagtgagta     120

ctgggtatgt gtcacattgc caaatcccgg atcacaagtc tccatgaact gctggtgagc     180

taggataata aaacccctga catcaccatt ccagaagctt cacaagactg catatataag     240

gggctggctg tagctgcagc tgaaggagct gaccagccag ctgacccctc acactcacct     300

agccaccatg gacatcgcca tccaccaccc ctggatccgc cgcccctctt ttcctttcca     360

ctcccccagc cgcctctttg accagttctt cggagagcac ctgttggagt ctgatctttt     420

cccgacgtct acttccctga gtcccttcta ccttcggcca ccctccttcc tgcgggcacc     480

cagctggttt gacactggac tctcagagat gcgcctggag aaggacaggt tctctgtcaa     540

cctggatgtg aagcacttct ccccagagga actcaaagtt aaggtgttgg gagatgtgat     600

tgaggtgcat ggaaaacatg aagagcgcca ggatgaacat ggtttcatct ccagggagtt     660

ccacaggaaa taccggatcc cagctgatgt agaccctctc accattactt catccctgtc     720

atctgatggg gtcctcactg tgaatggacc aaggaaacag gtctctggcc ctgagcgcac     780

cattcccatc acccgtgaag agaagcctgc tgtcaccgca gcccccaaga aatagatgcc     840

ctttcttgaa ttgcattttt taaaacaaga aagtttcccc accagtgaat gaaagtcttg     900

tgactagtgc tgaagcttat taatgctaag ggcaggccca aattatcaag ctaataaaat     960

atcattcagc aacagataaa aaaaaaaaaa aaaaaaa                              998
```

<210> 2
<211> 175

<212> PRT
<213> Homo sapiens

<220>
<221> alpha crystallin B chain protein isoform 1
<222> (1)..(175)

<400> 2

```
Met Asp Ile Ala Ile His His Pro Trp Ile Arg Arg Pro Phe Phe Pro
1               5                   10                  15

Phe His Ser Pro Ser Arg Leu Phe Asp Gln Phe Phe Gly Glu His Leu
            20                  25                  30

Leu Glu Ser Asp Leu Phe Pro Thr Ser Thr Ser Leu Ser Pro Phe Tyr
        35                  40                  45

Leu Arg Pro Pro Ser Phe Leu Arg Ala Pro Ser Trp Phe Asp Thr Gly
    50                  55                  60

Leu Ser Glu Met Arg Leu Glu Lys Asp Arg Phe Ser Val Asn Leu Asp
65                  70                  75                  80

Val Lys His Phe Ser Pro Glu Glu Leu Lys Val Lys Val Leu Gly Asp
                85                  90                  95

Val Ile Glu Val His Gly Lys His Glu Glu Arg Gln Asp Glu His Gly
            100                 105                 110

Phe Ile Ser Arg Glu Phe His Arg Lys Tyr Arg Ile Pro Ala Asp Val
        115                 120                 125

Asp Pro Leu Thr Ile Thr Ser Ser Leu Ser Ser Asp Gly Val Leu Thr
    130                 135                 140

Val Asn Gly Pro Arg Lys Gln Val Ser Gly Pro Glu Arg Thr Ile Pro
145                 150                 155                 160

Ile Thr Arg Glu Glu Lys Pro Ala Val Thr Ala Ala Pro Lys Lys
                165                 170                 175
```

<210> 3
<211> 1579
<212> DNA
<213> Homo sapiens

<220>
<221> NNMT transcript
<222> (1)..(1579)

<400> 3

```
gaggaggtgc ttgccagaca ctgggtcatg gcagtggtcg gtgaagctgc agttgcctag      60
ggcagggatg gagagagagt ctgggcatga ggagagggtc tcgggatgtt tggctggact     120
agattttaca gaaagcctta tccaggcttt taaaattact ctttccagac ttcatctgag     180
actccttctt cagccaacat tccttagccc tgaatacatt tcctatcctc atctttccct     240
tctttttttt cctttctttt acatgtttaa atttaaacca ttcttcgtga ccccttttct     300
tgggagattc atggcaagaa cgagaagaat gatggtgctt gttaggggat gtcctgtctc     360
tctgaacttt ggggtcctat gcattaaata attttcctga cgagctcaag tgctccctct     420
ggtctacaat ccctggcggc tggccttcat cccttgggca agcattgcat acagctcatg     480
gccctccctc taccataccc tccacccccg ttcgcctaag ctcccttctc cgggaatttc     540
atcatttcct agaacagcca gaacatttgt ggtctatttc tctgttagtg tttaaccaac     600
catctgttct aaaagaaggg ctgaactgat ggaaggaatg ctgttagcct gagactcagg     660
aagacaactt ctgcagggtc actccctggc ttctggagga aagagaagga gggcagtgct     720
ccagtggtac agaagtgaga cataatggaa tcaggcttca cctccaagga cacctatcta     780
agccatttta accctcggga ttacctagaa aaatattaca agtttggttc taggcactct     840
gcagaaagcc agattcttaa gcaccttctg aaaaatcttt tcaagatatt ctgcctagac     900
ggtgtgaagg gagacctgct gattgacatc ggctctggcc ccactatcta tcagctcctc     960
tctgcttgtg aatcctttaa ggagatcgtc gtcactgact actcagacca gaacctgcag    1020
gagctggaga agtggctgaa gaaagagcca gaggcctttg actggtcccc agtggtgacc    1080
tatgtgtgtg atcttgaagg gaacagagtc aagggtccag agaaggagga gaagttgaga    1140
caggcggtca agcaggtgct gaagtgtgat gtgactcaga gccagccact gggggccgtc    1200
cccttacccc cggctgactg cgtgctcagc acactgtgtc tggatgccgc ctgcccagac    1260
ctccccacct actgcagggc gctcaggaac ctcggcagcc tactgaagcc aggggggcttc    1320
ctggtgatca tggatgcgct caagagcagc tactacatga ttggtgagca gaagttctcc    1380
agcctccccc tgggccggga ggcagtagag gctgctgtga aagaggctgg ctacacaatc    1440
gaatggtttg aggtgatctc gcaaagttat tcttccacca tggccaacaa cgaaggactt    1500
ttctccctgg tggcgaggaa gctgagcaga cccctgtgat gcctgtgacc tcaattaaag    1560
caattccttt gacctgtca                                                 1579
```

<210> 4
<211> 264
<212> PRT
<213> Homo sapiens

<220>
<221> NNMT protein
<222> (1)..(264)

<400> 4

Met Glu Ser Gly Phe Thr Ser Lys Asp Thr Tyr Leu Ser His Phe Asn
1 5 10 15

Pro Arg Asp Tyr Leu Glu Lys Tyr Tyr Lys Phe Gly Ser Arg His Ser
20 25 30

Ala Glu Ser Gln Ile Leu Lys His Leu Leu Lys Asn Leu Phe Lys Ile
35 40 45

Phe Cys Leu Asp Gly Val Lys Gly Asp Leu Leu Ile Asp Ile Gly Ser
50 55 60

Gly Pro Thr Ile Tyr Gln Leu Leu Ser Ala Cys Glu Ser Phe Lys Glu
65 70 75 80

Ile Val Val Thr Asp Tyr Ser Asp Gln Asn Leu Gln Glu Leu Glu Lys
85 90 95

Trp Leu Lys Lys Glu Pro Glu Ala Phe Asp Trp Ser Pro Val Val Thr
100 105 110

Tyr Val Cys Asp Leu Glu Gly Asn Arg Val Lys Gly Pro Glu Lys Glu
115 120 125

Glu Lys Leu Arg Gln Ala Val Lys Gln Val Leu Lys Cys Asp Val Thr
130 135 140

Gln Ser Gln Pro Leu Gly Ala Val Pro Leu Pro Pro Ala Asp Cys Val
145 150 155 160

Leu Ser Thr Leu Cys Leu Asp Ala Ala Cys Pro Asp Leu Pro Thr Tyr
165 170 175

Cys Arg Ala Leu Arg Asn Leu Gly Ser Leu Leu Lys Pro Gly Gly Phe
180 185 190

Leu Val Ile Met Asp Ala Leu Lys Ser Ser Tyr Tyr Met Ile Gly Glu
195 200 205

Gln Lys Phe Ser Ser Leu Pro Leu Gly Arg Glu Ala Val Glu Ala Ala
210 215 220

Val Lys Glu Ala Gly Tyr Thr Ile Glu Trp Phe Glu Val Ile Ser Gln

```
225                 230                 235                 240

Ser Tyr Ser Ser Thr Met Ala Asn Asn Glu Gly Leu Phe Ser Leu Val
                245                 250                 255

Ala Arg Lys Leu Ser Arg Pro Leu
            260


<210>  5
<211>  4941
<212>  DNA
<213>  Homo sapiens


<220>
<221>  SPRY4 transcript variant 2
<222>  (1)..(4941)

<400>  5
attcataaaa aagccatttt cccaggcagt ggttgcaaca tcgccgcgga ggtagcgagc      60

tgagctgaca gcgcggagct ggcgctgtgg agcgcaggga gccttgccgg ttcctccgac     120

cggcgtctgc gagtacagcg gcggctaacc tgccccggct tcaggattta cacagacgtg     180

gggcgatgct tgtgaccctg cagctcctca aaggccccta gaagcctgtt tctccgtaca     240

gtccaggacc tccagcccca tggagccccc gatcccacag agcgccccct tgactcccaa     300

ctcagtcatg gtccagcccc ttcttgacag ccggatgtcc cacagccggc tccagcaccc     360

actcaccatc ctacccattg accaggtgaa gaccagccat gtggagaatg actacataga     420

caaccctagc ctggccctga ccaccggccc aaagcggacc cggggcgggg ccccagagct     480

ggccccgacg cccgcccgct gtgaccagga tgtcacccac cattggatct ccttcagcgg     540

gcgccccagc tctgtgagca gcagcagcag cacatcctct gaccaacggc tcttagacca     600

catggcacca ccacccgtgg ctgaccaggc ctcaccaagg gctgtgcgca tccagcccaa     660

ggtggtccac tgccagccgc tggacctcaa gggcccggcg gtcccacccg agctggacaa     720

gcacttcttg ctgtgcgagg cctgtgggaa gtgtaaatgc aaggagtgtg catccccccg     780

gacgttgcct tcctgctggg tctgcaacca ggagtgcctg tgctcagccc agactctggt     840

caactatggc acgtgcatgt gtttggtgca gggcatcttc taccactgca cgaatgagga     900

cgatgagggc tcctgcgctg accacccctg ctcctgctcc cgctccaact gctgcgcccg     960

ctggtccttc atgggtgctc tctccgtggt gctgccctgc ctgctctgct acctgcctgc    1020

caccggctgc gtgaagctgg cccagcgtgg ctacgaccgt ctgcgccgcc ctggttgccg    1080

ctgcaagcac acgaacagcg tcatctgcaa agcagccagc ggggatgcca agaccagcag    1140

gcccgacaag cctttctgac agtttgtgtc gaagccccag tgctctgcct ggaaacctgg    1200

ttctcttctg acatctaaga agactgcagc aaggtcagag gttttagcct cctgaggctg    1260
```

```
accttgctag tctgcccact ccctaccccc agcttcggaa aatacagaga ccaccaccac    1320
gtaccctgta ttccccaagg tgatgaagaa gcactttggg gctttttttc agggtcctga    1380
aactttgtgt caaacagaca atgcaggggc agggtgtggt ttggggggaa atttttcttt    1440
ttcagaagac agaacacaga tgtggacaca tatccggaaa ctgcagctgc ttgaatgcct    1500
tcccagcccc tccttctccc tccctccctc cgcccccccc cccttcctct tttccattgt    1560
ctttggctct cacaggagct agctgcctgg gaggaattgt taactgagta ccagggtacc    1620
tttaaagaag acccttggag tcttctatac cttcttctcc ttccccatct cactccaccc    1680
cactttgtcc ctgatgtctt ggggaaggtg tagaacaccc tagcagttcc tattgtatat    1740
acttgggagc cactgagaac agaggacggc cagtgagtcc aagcctcgtt cctccttctg    1800
cctccccgga gccacaggat ggatttagga gccactgctc agtgcacttc tcccttccaa    1860
ctgcatcaac taactctcgg gggtgttctg ctcaccacac cgtccttcgg ttcttactga    1920
gtcacagact cgcctgccca ctacgtgtcc tgggttctct ctactcagat cccttccaga    1980
aactttatat gggtagagga agccagggcg gcaaatgcga gaccaaatat cattttgcca    2040
atgagtctga ggctgtggtc tctggatcca gtcattatgt ttttatagaa taattaaacc    2100
ggatgctaac ggtgttttaa aaaataataa taaaacaact tgtttccttt tggccacccc    2160
caggaagggc tgatttcaaa atctgggggc gagcaacctc aaggaacaca atttccctcc    2220
ctatcaacaa gaggatttta acagcaaaga agagaggcag cacctcccat tggcagaatg    2280
accgctgagc caggctgggt ttgggtttct tctcttctga ttctgctgct cactgtcata    2340
gccttttgtg tatagtgatg tgtctgtatc tttaatgtaa atagagagat gatgaaaaaa    2400
gagtctattt tagtgttagg aagccccagc aggggagtcg gaagagcttg gaagagctgg    2460
ggagagggta ggggaaaggt ttttccaggg gccactgggt ttgagccctg cttctgtgca    2520
cagccacacc accctctccc gacagccctc aaagacgtag caactctttc tctcaaggtg    2580
ctaaaggact cagaaggtgc agcacgtcca gtgggtaggt acttgttgca tgcaaaagct    2640
gtagtgtatc tggtccttcc tccccagctt ttgtgtgggg ttcttgcttt gtgtggtatt    2700
ttgttttccc ctctaatgag agggcatggc ctgagtcaga agagctaccc caggtgaaac    2760
tggaagtgca tgaggcagag cgtccgtagc atttccagtt tgttctgtat aggaacagag    2820
gtgcctccgg gaaggaggca gcgaggtagg tagctatgat aggcacctaa tgcttctcaa    2880
ggacttattt tttccttctt gaagactagt agtaacatct tatgatttag agtaagttga    2940
ttgtaaccat aggtatttat tgattggagg aagggagggt catattattt tcggctttat    3000
ttatgtaaca tttgctagct tgtaaaaggc gaatgtgaaa tattgcatct gcattttcca    3060
aggctgattc gtgtagctac ccttgccaca gttgtgacgg atgtatggat gttcttgaac    3120
```

atttcagaag gagtggtaga aaaaaacaca cattcagcca accacttata tgaattgaat 3180

gtatcagaag tgtactgaag ggactggaga tggttttcct cagatgaggg ggccccaaaa 3240

ttgatagtgc acatctgcac gctttctgcg aggcctcaga acttcccagg gcccctccct 3300

caaattgtct ccatgggaaa cttgacccag tggcaagttg cactttggtg atcttggtgg 3360

tctacacacc cgttctgtgg agagtcgatt tacataagct gtgtatacac acacacacac 3420

acacacacac acccctaccc cacactgact gtctaccgac aaagacccta tttcctggca 3480

aacggcctcc tgaaccctga ctttttgtgt acatacttgt aaacacggat ttttctgggt 3540

tttggtttgc tttttccttt tttccccctg ccctgttct agcttgttct tcttggtttg 3600

ctttcaacct gcttgatgga tgtctgcaga gtgctctcta agagtccacc tcagtgcctc 3660

gtgtgctcag tggtcatggg aaaggagcga aggaaccatc cttggttctc ccagcttggt 3720

tgtgtagcaa tccctcagca ttgtttttct cagcttcttg gcaaaaatta aaacaacaac 3780

aacaacaaca acaacaacaa caaacagaag gataaactgg cttgcctgtg gaccctcccc 3840

ggctctgggg ccagtcgaga gccactgagg gacccagcac tcagagacac aacacacatg 3900

tgtagctgct tctggctgag tgtgtttcct gtcaccaatg gcctgtttgg ctggacgatg 3960

cctcggcttg acctttttg aaaagtgctg gttagttccc gcccctggta aacctggggt 4020

aggtgggggt tctgtcttaa ctcgaggggc acctgggatc caggacgctt ctaggggct 4080

ctggctgccc gtgttaatga aggacagcgc ttccgcgagc accctgggaa ctgggtcttg 4140

ggtagcaaag ccctcccaga gaaaagattg ggcacaacta aggctttcct gagcaggaag 4200

ggggtgaaga ccaatccctt cctttggtcc tttggtacgc accccctcag agctgagatg 4260

gaagacatgg ctagttcttt tcagccttgt ggagcctgtc agtcgccatc atacctcgag 4320

tgaggcccag ctagataatg acttgtccaa gatggcacac gtggaaagtt gatctgcacc 4380

agaacccgga tgactgtcac cttgaagcat cctgttctcc ttctgtgctg tcccaggaag 4440

tgtctggcgg gcgtgggcag cacagctcta cactgtacga ttcactaggg catcctgcga 4500

gcctcactag ccttctggtt catgcctttg acaagcattt ttgtgccccc tctgcttact 4560

gtgacagtcg atgatgaatc ttgcgttgcc attttctgct gtgggtaact gcgtgcagtg 4620

tcttgccttg ctttctcttc ttactgtccc acagcttggt ttcatgttac aaacagaaaa 4680

gctcgaggct cccaccccgc cacatcccaa cttcatttcc ccctcactgt agcccatttc 4740

caccccacca caaagttgcc acaggttttc tttgtataga atatttattt tgaagctcta 4800

ttttaatagt atttatttta gaaagtctac tattgtaaga gttcttctgt ttgtgaagaa 4860

aaaaacaagt taaaaactga atgtactgat ttagaaaata tatataaata tatattgtta 4920

aatatacacg ggactgccgt t 4941

```
<210>  6
<211>  299
<212>  PRT
<213>  Homo sapiens


<220>
<221>  SPRY4 protein isoform 2
<222>  (1)..(299)

<400>  6

Met Glu Pro Pro Ile Pro Gln Ser Ala Pro Leu Thr Pro Asn Ser Val
1               5                   10                  15


Met Val Gln Pro Leu Leu Asp Ser Arg Met Ser His Ser Arg Leu Gln
            20                  25                  30


His Pro Leu Thr Ile Leu Pro Ile Asp Gln Val Lys Thr Ser His Val
        35                  40                  45


Glu Asn Asp Tyr Ile Asp Asn Pro Ser Leu Ala Leu Thr Thr Gly Pro
    50                  55                  60


Lys Arg Thr Arg Gly Gly Ala Pro Glu Leu Ala Pro Thr Pro Ala Arg
65                  70                  75                  80


Cys Asp Gln Asp Val Thr His His Trp Ile Ser Phe Ser Gly Arg Pro
                85                  90                  95


Ser Ser Val Ser Ser Ser Ser Ser Thr Ser Ser Asp Gln Arg Leu Leu
            100                 105                 110


Asp His Met Ala Pro Pro Pro Val Ala Asp Gln Ala Ser Pro Arg Ala
        115                 120                 125


Val Arg Ile Gln Pro Lys Val Val His Cys Gln Pro Leu Asp Leu Lys
    130                 135                 140


Gly Pro Ala Val Pro Pro Glu Leu Asp Lys His Phe Leu Leu Cys Glu
145                 150                 155                 160


Ala Cys Gly Lys Cys Lys Cys Lys Glu Cys Ala Ser Pro Arg Thr Leu
                165                 170                 175


Pro Ser Cys Trp Val Cys Asn Gln Glu Cys Leu Cys Ser Ala Gln Thr
            180                 185                 190


Leu Val Asn Tyr Gly Thr Cys Met Cys Leu Val Gln Gly Ile Phe Tyr
        195                 200                 205
```

```
His Cys Thr Asn Glu Asp Asp Glu Gly Ser Cys Ala Asp His Pro Cys
    210                 215                 220

Ser Cys Ser Arg Ser Asn Cys Cys Ala Arg Trp Ser Phe Met Gly Ala
225                 230                 235                 240

Leu Ser Val Val Leu Pro Cys Leu Leu Cys Tyr Leu Pro Ala Thr Gly
                245                 250                 255

Cys Val Lys Leu Ala Gln Arg Gly Tyr Asp Arg Leu Arg Arg Pro Gly
            260                 265                 270

Cys Arg Cys Lys His Thr Asn Ser Val Ile Cys Lys Ala Ala Ser Gly
        275                 280                 285

Asp Ala Lys Thr Ser Arg Pro Asp Lys Pro Phe
    290                 295


<210>  7
<211>  18
<212>  DNA
<213>  Artificial Sequence

<220>
<223>  CRYAB forward primer


<220>
<221>  misc_feature
<222>  (1)..(18)

<400>  7
ccttctacct tcggccac                                                    18


<210>  8
<211>  18
<212>  DNA
<213>  Artificial Sequence

<220>
<223>  CRYAB reverse primer


<220>
<221>  misc_feature
<222>  (1)..(18)

<400>  8
ttctccaggc gcatctct                                                    18


<210>  9
<211>  21
<212>  DNA
<213>  Artificial Sequence
```

<220>
<223> CRYAB probe

<400> 9
tccttcctgc gggcacccag c 21

<210> 10
<211> 20
<212> DNA
<213> Artificial Sequence

<220>
<223> NNMT forward primer

<220>
<221> misc_feature
<222> (1)..(20)

<400> 10
tgaagggaac agagtcaagg 20

<210> 11
<211> 20
<212> DNA
<213> Artificial Sequence

<220>
<223> NNMT reverse primer

<220>
<221> misc_feature
<222> (1)..(20)

<400> 11
gtcacatcac acttcagcac 20

<210> 12
<211> 27
<212> DNA
<213> Artificial Sequence

<220>
<223> NNMT probe

<220>
<221> misc_feature
<222> (1)..(27)

<400> 12
accgcctgtc tcaacttctc ctccttc 27

<210> 13
<211> 20
<212> DNA
<213> Artificial Sequence

<220>
<223> SPRY4 forward primer

<220>
<221> misc_feature
<222> (1)..(20)

<400> 13
cggcttcagg atttacacag 20

<210> 14
<211> 20
<212> DNA
<213> Artificial Sequence

<220>
<223> SPRY4 reverse primer

<220>
<221> misc_feature
<222> (1)..(20)

<400> 14
ttctaggggc ctttgaggag 20

<210> 15
<211> 22
<212> DNA
<213> Artificial Sequence

<220>
<223> SPRY4 probe

<220>
<221> misc_feature
<222> (1)..(22)

<400> 15
tgcagggtca caagcatcgc cc 22

<210> 16
<211> 20
<212> DNA
<213> Artificial Sequence

<220>
<223> TGFbeta1 forward primer

<220>
<221> misc_feature
<222> (1)..(20)

<400> 16
tcgccagagt ggttatcttt 20

<210> 17
<211> 18
<212> DNA
<213> Artificial Sequence

<220>
<223> TGFbeta1 reverse primer

<220>
<221> misc_feature
<222> (1)..(18)

<400> 17
atttcccctc cacggctc 18

<210> 18
<211> 23
<212> DNA
<213> Artificial Sequence

<220>
<223> TGFbeta1 probe

<220>
<221> misc_feature
<222> (1)..(23)

<400> 18
accactgccg cacaactccg gtg 23

<210> 19
<211> 20
<212> DNA
<213> Artificial Sequence

<220>
<223> TGFbeta2 forward primer

<220>
<221> misc_feature
<222> (1)..(20)

<400> 19
ttgctttaga aatgtgcagg 20

<210> 20
<211> 20
<212> DNA
<213> Artificial Sequence

<220>
<223> TGFbeta2 reverse primer

<220>
<221> misc_feature

<222> (1)..(20)

<400> 20
ccatttccac cctagatccc 20

<210> 21
<211> 32
<212> DNA
<213> Artificial Sequence

<220>
<223> TGFbeta2 probe

<220>
<221> misc_feature
<222> (1)..(32)

<400> 21
attgctgcct acgtccactt tacattgatt tc 32

<210> 22
<211> 20
<212> DNA
<213> Artificial Sequence

<220>
<223> TGFbeta3 forward primer

<220>
<221> misc_feature
<222> (1)..(20)

<400> 22
gctgttgaga agagagtcca 20

<210> 23
<211> 19
<212> DNA
<213> Artificial Sequence

<220>
<223> TGFbeta3 reverse primer

<220>
<221> misc_feature
<222> (1)..(19)

<400> 23
cattgggctg aaaggtgtg 19

<210> 24
<211> 33
<212> DNA
<213> Artificial Sequence

<220>

```
<223>  TGFbeta3 probe

<220>
<221>  misc_feature
<222>  (1)..(33)

<400>  24
cttaggtcta gaaatcagca ttcactgtcc atg                              33
```

**Description**

**FIELD OF THE INVENTION**

The present invention relates to a single non-cell specific biomarker signature useful to identify cancer patients having tumors with a TGFβ-activated microenvironment. This signature would enable to identify those cancer patients who will most likely benefit from treatment with an inhibitor of the TGFβ signaling pathway. In addition, said biomarker signature has been shown to be useful for various tumor types (a pan-tissue signature). It further provides related prognostic methods, methods of patient classification and selection for treatment with an inhibitor of the TGFβ signaling pathway, methods for patient monitoring or evaluation of response, second medical uses, diagnostic kits and uses thereof.

**BACKGROUND OF THE INVENTION**

Colorectal cancer kills around 700.000 patients every year worldwide. At the time of diagnosis, the majority of patients will present loco-regional disease that can be effectively resected by surgery. This intervention is sufficient to cure the primary disease in most cases. Yet, over the course of the following months or years, around 40% of the patients that underwent resection of the primary tumor with curative intention will relapse, generally in the form of metastatic disease. As these metastases eventually interfere with the function of vital organs such as the liver and lungs, patients that undergo relapse have poor prognosis.

The transforming growth factor-beta (TGFβ) signaling inhibition is an emerging strategy for cancer therapy. The role of the TGFβ pathway as a tumor promoter or suppressor at the cancer cell level is still a matter of debate, due to its differential effects at the early and late stages of carcinogenesis. Conversely, the TGFβ signaling pathway has been described to exert pro-tumoral activities at the microenvironment level. Thus, TGFβ pathway inhibition strategies in cancer are primarily targeting the tumor microenvironment (TME).

The TME is a complex structure composed of extracellular matrix proteins (mainly, type I collagen) and various cell types including mesenchymal cells (cancer-associated fibroblasts [CAF]), endothelial cells and pericytes, nerve cells, immune cells, and bone marrow-derived stem cells. These cell types can express TGFβ receptors and respond to elevated TGFβ levels present in the TME. TGFβ has been reported to regulate fibrosis, angiogenesis, and immune cell function in the context of tumors (Neuzillet et al., Pharmacology & Therapeutics 147 (2015) 22-31; Batlle and Massague, Immunity 50 (2019), 924-940)

Furthermore, poor prognosis in CRC has been associated to the presence of a TGFβ-activated TME. The inventors previously described that the response of cells of the TME to TGFβ is associated with CRC progression and disease relapse (Calon et al., Cancer Cell 22 (2012) 571-584; Calon et al., Nature Genetics 47:4 (2015) 320-332). In particular, they described the use of the gene expression programs induced by addition of TGFβ1 (from here onwards named as TGFβ response signatures or TBRS) in cultures of normal tissue derived T cells (T-TBRS), macrophages (Ma-TBRS) or fibroblasts (F-TBRS) as surrogates of the degree of activation of the most abundant cell types in the TME of CRCs by TGFβ. It was selected as TBRS the full set of genes upregulated by TGFβ signaling in these cell cultures (>2 fold, $p<0.05$). F-TBRS is composed of 165 genes, T-TBRS of 76 and Ma-TBRS of 1125 genes. Each signature reflects the transcriptional activity of TGFβ signaling pathway in fibroblasts, macrophages and T cells respectively. Authors followed to show that the average expression of each of these TBRS predict very significantly poor prognosis in CRC patient transcriptomic cohorts (Calon et al., Cancer Cell 22 (2012) 571-584; Calon et al., Nature Genetics 47:4 (2015) 320-332).

In parallel, a European consortium comprising clinicians and researchers re-classified CRC patients depending on molecular signatures and mutated pathways. This work gave rise to the definition of 4 consensus molecular subtypes (CMS) (Guinney et al. Nat Med. 2015, 21(11), 1350-6). In particular, in the consensus classification published by Guinney et al., the group with the worst prognosis (CMS4) was reported to be characterized by activation of pathways involved in EMT and the TGF-beta program -angiogenesis, remodeling of the extracellular matrix, inflammatory system.

There are many clinical challenges to developing inhibitors of TGFβ signaling pathway, notably timing of treatment and predictive biomarkers for patient selection, in order to define in what kind of tumor microenvironment TGFβ signaling inhibition may be more beneficial (Neuzillet et al., Pharmacology & Therapeutics 147 (2015) 22-31). As pointed out in deGramont et al. (Oncoimmunology 6:1 (2017) e1257453), due to the complex nature of the TGFβ pathway, its role in cell fate and their differential activity in tumor cells and their microenvironment, predictive biomarkers may be challenging to identify.

Recently, increased TGFβ activity in the TME was found to be associated with lack of response to PD-1-PD-L1 immune checkpoint inhibitors in CRC (Tauriello et al., Nature, Vol. 554 (2018) 539 - 543) and in human metastatic urothelial cancer (mUC) samples (Mariathasan et al., Nature, Vol. 554 (2018) 544 - 548).

Despite recent advances, there is still a need to find simple, accurate and reproducible methods which are easy to apply in the clinical practice for predicting the outcome of cancer and/or help clinicians in the selection of the more appropriate treatment strategies and the follow-up of patients. In particular, there is a need for identifying new predictive biomarkers determining those cancer patients having tumors with a TGFβ-activated TME and thus presenting a higher likelihood to respond to a TGFβ pathway inhibition therapy. These patients are also likely not to respond to checkpoint immunotherapies. To this end, the assessment of F-TBRS, T-TBRS and Ma-TBRS described above represents an unfeasible approach as each TBRS is composed of a very large number of genes (>1000 in total). Therefore, a simple method to identify tumors that exhibit an overall TGF-β-activated TME could be of great utility for personalized medicine and cancer treatment, especially when this method is of general application to a variety of tumor types.

The present invention addresses these needs.

## SUMMARY OF THE INVENTION

In a first aspect, the present invention provides a method for determining whether a patient's tumor has a TGFβ-activated microenvironment (TME), wherein said method comprises determining the expression levels of a group of genes comprising or consisting of CRYAB (Crystallin Alpha B), NNMT (Nicotinamide N-Methyltransferase), and SPRY4 (Sprouty RTK Signaling Antagonist 4) genes (also referred herein as "the MOMTGFB genes") in tumor samples and using the obtained expression levels to calculate a score (also referred herein as "MOMTGFB score") which has been found by the inventors to correlate with overall TGFβ levels and with a TGFβ-activated TME.

As described in Example 2, the inventors found that a mathematical combination of the levels of expression of the three MOMTGFB genes was associated with overall TGFβ levels measured by qRT-PCR on RNA extracted from paraffin embedded samples from a colorectal cancer patient cohort (n=270).

Moreover, the inventors found that the MOMTGFB three gene signature can be used as surrogate of the overall degree of activation of TME by TGFβ, including TGFβ activity in fibroblasts, T cells and macrophages.

TGFβ is secreted as a pro-hormone that is stored in the extracellular matrix in an inactive form and that can be subsequently mobilized and activated by sophisticated mechanisms (Lyons et al. J.Cell.Biol. (1990) 110(4), 1361-1367, (Batlle and Massagué. Immunity 50 (2019), 924-940). In addition, each cell type present in the TME may display different degrees of TGFβ activity depending on the expression of TGFβ receptors, presence of inhibitory molecules and other contextual molecules that modify their susceptibility to TGFβ accumulated in the TME (David and Massagué. Nat Rev Mol Cell Biol. 2018). The degree of TGFβ-activated TME has been previously measured in transcriptomic cancer datasets using as surrogate the fibroblast TGFβ response signature (F-TBRS), the T-cell (T-TBRS) and the macrophage (Ma-TBRS) response signatures described in Calon et al. 2012 (Cancer Cell 22, (2012) 571-584). In the present application the inventors showed that the group of cancer patient samples with highest levels of MOMTGFB scores also exhibited elevated levels of the three cell type-specific TBRS signatures previously disclosed in Calon et al. 2012 (Cancer Cell 22, (2012) 571-584) .

Therefore, the inventors have identified a gene signature which associates with general non-cell-specific activation of the tumor microenvironment by TGFβ, more specifically, it associates with a tumor microenvironment comprising TGFβ activated fibroblasts, T-cells and macrophages. Accordingly, this signature enables to capture the activation of the microenvironment by TGFβ using a single biomarker signature instead of three distinct signatures (i.e., F-TBRS, T-TBRS, Ma-TBRS). This association was found in several tumors when analyzing the transcriptomic cohorts of various cancer types (Example 3), namely in colorectal carcinoma, stomach adenocarcinoma, bladder cancer, pancreas adenocarcinoma, prostate carcinoma, lung cancer and breast cancer.

Interestingly, a high MOMTGFB score was shown to identify 96% of CRC tumors classified under CMS4, a CRC molecular subtype associated with prominent TGFβ activation according to the consensus molecular subtypes (CMSs) classification (Guinney et al. Nat Med. 2015, 21(11), 1350-6). Importantly, it was also able to capture tumors with a TGFβ-activated TME classified under other CMS groups (Example 3.1, **Figures 4** and **Figure 5**). The provision of a method identifying tumors presenting a TGFβ-activated TME enables to select for treatment with an inhibitor of the TGFβ signaling pathway, tumors belonging to CMS1-3 subgroups (typically not associated with a TGFβ activated TME) which would thus otherwise likely not had been selected for treatment with a TGFβ signaling pathway inhibitor.

In addition, the inventors showed that the signature of the invention was capable of identifying tumors presenting a TGFβ-activated TME, and thus likely to benefit from a therapy inhibiting the TGFβ signaling pathway, independently of microsatellite stability status, that is, both in microsatellite stable (MSS) and microsatellite instable (MSI) phenotypes (Example 3, **Figure 6** and **Figure 8**).

MSI-high (MSI-H) status has been previously associated with better prognosis in early-stage CRC and has emerged as a predictor of sensitivity to check-point inhibitors immunotherapy treatments (Battaglin et al., Advances in Hematology and Oncology 2018, 16(11), 735-747). Nevertheless, as above-mentioned, immune checkpoint inhibitors were found by Tauriello et al. (Tauriello et al., Nature, Vol. 554 (2018) 539 - 543) to lack efficacy in CRC tumors with a TGFβ activated microenvironment. Thus, the signature of the invention enables to identify from patients having a MSI tumor, those which would likely not benefit from an immune check point inhibitor treatment alone and select the same for a combination treatment with an inhibitor of the TGFβ signaling pathway. Thus, the method of the invention further enables selecting patients having tumors which would likely benefit from a TGFβ inhibition therapy for which such therapy would not had been selected on the basis of MSI status or CMS molecular classification methods.

TGFβ signaling in the TME has been reported to operate as the main mechanism of immune evasion during metastasis formation. Elevated TGFβ triggers T cell exclusion, a phenomenon associated with poor outcome in CRC and other tumor types, and blocks antitumor Th1 effector phenotype (Tauriello et al., Nature, Vol. 554 (2018) 539 - 543; Galon et al., Science 313 (2006) 1960-1964; Mariathasan et al., Nature, Vol. 554 (2018) 544 - 548). In Tauriello et al., Galunisertib (GAL), which is a small molecule TGFBR1 inhibitor, was used to treat mice with TGFβ high metastatic CRCs. GAL treatment unleashed the immune system against CRC, which exerted a potent therapeutic response that prevented the formation of metastasis. In mice bearing overt metastatic disease, the combination of GAL with anti-PD-L1 treatment eradicated most metastases and prolonged recurrence-free survival for over a year after treatment cessation. This striking response was characterized by disruption of the T-cell exclusion phenotype characteristic of progressed metastatic disease, and by prominent Th1 immune activation (Tauriello et al., Nature, Vol. 554 (2018) 539 - 543). When these same tumors were allowed to develop established metastasis, GAL alone had no effect. Yet, a combination of GAL plus immune checkpoint therapies cured overt metastatic disease (data from Tauriello et al., Nature, Vol. 554 (2018) 539 - 543). Similar observations were made in models of urothelial and breast carcinomas (Mariathasan et al., Nature, Vol. 554 (2018) 544 - 548).

Tauriello et al. (Nature, Vol. 554 (2018) 539 - 543) showed that treatment with the TGFβ inhibitor Galunisertib (GAL) of mice orthotopically transplanted with LAKTP mutant mice organoids was associated to a strong reduction/prevention of liver metastasis, as well as a reduction of primary tumor (and local carcinomatosis) size (Tauriello et al., Nature, Vol. 554 (2018) 539 - 543). As described in Example 4, the inventors measured MOMTGFB score in the developed primary CRC tumors, which resemble the metastatic intestinal tumors of origin and exhibit a TGFβ-activated TME, before and after treatment of mice with GAL. As shown in **Figure 14,** MOMTGFB score was found to be lower after therapy with GAL, thus supporting the utility of the MOMTGFB gene signature as predictive biomarker of response to TGFβ inhibition therapies and as biomarker for monitoring or evaluating response to treatment with TGFβ inhibitors.

Accordingly, in a second aspect, the invention also pertains to a method for predicting the efficacy of (or the likelihood of response to) a treatment with an inhibitor of the TGFβ signaling pathway in a cancer patient, wherein said method comprises:

i. determining whether a patient's tumor has a TGFβ-activated microenvironment according to a method as described herein; and

ii. predicting a higher efficacy or likelihood of response to a treatment with an inhibitor of the TGFβ signaling pathway when said patient's tumor has a TGFβ-activated microenvironment.

In a third aspect, the present invention relates to a method for selecting a cancer patient which is likely to benefit from a treatment with an inhibitor of the TGFβ signaling pathway, wherein said method comprises:

i. determining whether a patient's tumor has a TGFβ-activated microenvironment according to a method as described herein; and

ii. selecting said cancer patient for the treatment with an inhibitor of the TGFβ signaling pathway when said patient's tumor has a TGFβ-activated microenvironment.

In a fourth aspect, the invention pertains to an inhibitor of the TGFβ signaling pathway for use in a method for treatment of a cancer patient, wherein said patient's tumor has been determined to have a TGFβ-activated microenvironment according to a method as described herein.

In an alternative aspect, the invention provides the use of an inhibitor of the TGFβ signaling pathway in the manufacturing of a medicament for the treatment of a cancer patient, wherein said cancer patient has been selected as having a tumor with a TGFβ-activated microenvironment by a method as described herein.

In a further alternative aspect, the invention relates to a method of treating a cancer patient by administering a therapeutically effective amount of an inhibitor of the TGFβ signaling pathway, wherein said patient has been selected as having a tumor with a TGFβ-activated microenvironment by a method as described herein.

In a fifth aspect, the present invention relates to an anti-cancer agent other than an inhibitor of the TGFβ signaling pathway (e.g., cytotoxic agent or immune checkpoint inhibitor) or radiotherapy for use in a method for treatment of a cancer patient in combination with an inhibitor of the TGFβ signaling pathway, wherein said patient's tumor has a TGFβ-activated microenvironment according to a method as described herein.

In an alternative aspect, the invention provides the use of an anti-cancer agent other than an inhibitor of the TGFβ signaling pathway (e.g., cytotoxic agent or immune checkpoint inhibitor) or radiotherapy in combination with an inhibitor of the TGFβ signaling pathway in the manufacturing of a medicament for the treatment of a cancer patient, wherein said cancer patient has been selected by a method as described herein as having a tumor with a TGFβ-activated microenvironment.

In a further alternative aspect, the invention relates to a method of treating a cancer patient by administering a therapeutically effective amount of an anti-cancer agent other than an inhibitor of the TGFβ signaling pathway (e.g., cytotoxic agent or immune checkpoint inhibitor) or radiotherapy in combination with a therapeutically effective amount of an inhibitor of the TGFβ signaling pathway, wherein said patient has been selected by a method as described herein as having a tumor with a TGFβ-activated microenvironment.

In a sixth aspect, the present invention relates to an anti-cancer agent other than an inhibitor of the TGFβ signaling pathway (e.g., cytotoxic agent or immune checkpoint inhibitor) or radiotherapy for use in a method for treatment of a cancer patient selected by a method as described herein as failing to have a tumor with a TGFβ-activated microenvironment, wherein said other anti-cancer agent or radiotherapy is not administered in combination with an inhibitor of the TGFβ signaling pathway.

In an alternative aspect, the invention provides the use of an anti-cancer agent other than an inhibitor of the TGFβ signaling pathway (e.g., cytotoxic agent or immune checkpoint inhibitor) or radiotherapy in the manufacturing of a medicament for the treatment of a cancer patient selected by a method as described herein as failing to have a tumor with a TGFβ-activated microenvironment, wherein said other anti-cancer agent or radiotherapy is not administered in combination with an inhibitor of the TGFβ signaling pathway.

In a further alternative aspect, the invention relates to a method of treating a cancer patient selected by a method as described herein as failing to have a tumor with a TGFβ-activated microenvironment, by administering a therapeutically effective amount of an anti-cancer agent other than an inhibitor of the TGFβ signaling pathway (e.g., cytotoxic agent or immune checkpoint inhibitor) or radiotherapy, wherein said other anti-cancer agent or radiotherapy is not administered in combination with an inhibitor of the TGFβ signaling pathway.

In a particular embodiment of any thereof, said other anti-cancer agent or radiotherapy is administered as single agent or therapy.

In a seventh aspect, the invention relates to a method for determining the prognosis of a cancer patient, wherein said method comprises:

i. determining whether a patient's tumor has a TGFβ-activated microenvironment according to a method as described herein; and
ii. classifying the patient as having poor prognosis when said patient's tumor has a TGFβ-activated microenvironment.

In an eighth aspect, the present invention relates to a method for monitoring or evaluating the response to treatment with an inhibitor of the TGFβ signaling pathway in a cancer patient, wherein said method comprises determining whether a patient's tumor has a TGFβ-activated microenvironment as described herein, wherein a TGFβ-activated microenvironment is associated to lack of response.

In an ninth aspect, the invention concerns a kit suitable for determining the expression levels of the genes CRYAB, NNMT, and SPRY4 in an isolated tumor sample, wherein said kit comprises:

i. a reagent for the quantification of the CRYAB gene expression levels;
ii. a reagent for the quantification of the NNMT gene expression levels;
iii. a reagent for the quantification of the SPRY4 gene expression levels; and
iv. optionally, a reagent for the quantification of a control gene expression levels;
v. optionally, further comprising instructions for the use of said reagents in determining the expression levels of said genes in a tumor sample isolated from a cancer patient.

In a tenth aspect, the invention refers to the use of a kit as described herein, in a method for determining whether a patient's tumor has a TGFβ-activated microenvironment according to a method as described herein, in a method for selecting a cancer patient which is likely to benefit from a treatment with an inhibitor of the TGFβ signaling pathway as described herein, in a method for monitoring or evaluating the response to an inhibitor of the TGFβ signaling pathway as described herein, or in a method for determining the prognosis of a cancer patient as described herein.

## BRIEF DESCRIPTION OF THE DRAWINGS

Figure 1. Association between MOMTGFB score and overall TGFβ levels measured by qRT-PCR in formalin fixed paraffin embedded samples from a cohort of colorectal cancer patients (FFPE cohort, n=270). Every dot corresponds to one tumor sample. The expression of MOMTGFB genes and TGFβ1, -2, and 3 in each sample was normalized to overall average expression of 12 normalizers in that sample.

Figure 2. Smoothed estimates of log-Hazard Ratio and Kaplan-Meier graphs showing disease free survival (DFS) in years in patients classified in two groups according to the MOMTGFB score (Low vs High) in the FFPE cohort in all patients (stage 1-3), in stage 2 and in stage 3 patients. Patients with high MOMTGFB score bear tumors with a TGFβ-activated TME and are therefore likely to benefit from treatments aimed to inhibit TGFβ signaling.

Figure 3. Box-Plot graphs depicting overall TGFβ, F-TBRS, T-TBRS and Ma-TRBRS expression levels in 3 groups of patients according to their MOMTGFB score (Low, Medium and High). Transcriptomic data from the GEO + TCGA cohort of CRC patients (n=1705). Results are shown for all samples. Each dot is one sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated stroma using as surrogates F-, T- and Ma-TBRSs.

Figure 4. **A.** Patients with CMS4 tumors show overall worse prognosis compared to (CMS1-3). Kaplan Meier (K-M) curves showing disease free survival for patients with associated CMS data in the pooled transcriptomic cohort (GEO +TCGA); K-M curves only plot stage 1-3 patients with no metastasis at the time of diagnosis and only use patients for whom there are clinical follow up data. **B.** CMS4 tumors show a high degree of TGFβ-activated stroma according to their high overall expression levels of F-, T-, and Ma-TBRS. Accordingly, most patients fall into the category with high MOMTGFB score. Sample groups of low, medium and high MOMTGFB expression levels were defined using the mean and -1 standard deviation as cutoffs. Tukey box plots have whiskers of maximum 1.5 times the interquartile range; the boxes represent first, second (median) and third quartiles. There are no patients with low MOMTGFB scores in the CMS4 category. **C.** MOMTGFB captures 96.1% of CMS4 patients. These patients are likely to benefit from therapies directed towards inhibition of TGFβ signaling. In addition MOMTGFB captures patients with high activated stroma in the other CMS subtypes (see figure 5).

Figure 5. **A** Patients from the pooled transcriptomic cohort (GEO +TCGA) with tumors classified in each CMS1, CMS2 and CMS3 subtypes have been divided in three subgroups (i.e., Low / Medium / High) according to the MOMTGFB score. Box Plots show association with overall expression levels of TGFβ-activated stromal signatures for each CMS subtype of patient's tumors. Results are shown for all samples. Each dot is one sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Those patients with high MOMTGFB score present tumors characterized by a higher degree of TGFβ-activated stroma using as surrogates F-, T- and Ma-TBRSs. **B.** Kaplan-Meier graphs show disease free survival (DFS) in years per CMS type (i.e., CMS1, CMS2, CMS3) in the above subgroups (i.e., Low / Medium / High) according to the MOMTGFB score. Kaplan Meier analysis demonstrates that patients with high MOMTGFB levels within CMS1 and CMS3 have clearly worse prognosis and therefore these patients may also benefit from therapies that inhibit TGFβ signaling. Patients bearing tumors with a TGFβ-activated microenvironment represent 51% from the CMS1 subtype according to the MOMTGFB scores (see also Figure 3C). According to current stratification methods, these patients will be misclassified as good prognosis patients, likely to benefit from checkpoint immunotherapies.

Figure 6. MOMTGFB genes identify patients susceptible for treatment with TGF-beta inhibitors in previously used classifications for CRC prognosis such as MSS and MSI patients. As shown, within patients with MSI, considered as patients with good prognosis, the combined use of these three genes can identify patients with a TGFβ-activated TME, that have bad prognosis, and that are therefore susceptible of benefitting from therapies that inhibit TGFβ signaling pathway.

Figure 7. Heatmaps illustrating the correlations between MOMTGFB score with average gene expression of TGFβ, F-TBRS, T-TBRS, Ma-TBRS and distribution of Consensus Molecular Subtypes (CMS) in CRC transcriptomic cohorts (TCGA+GEO: 1705 patients), including patients from stages I to IV. There is an inverse correlation with the expression of a signature of CD45 positive cells that captures the relative abundance of leukocytes compared to the other cell types present in the TME. Furthermore, as previously showed (Figure 4), a majority of CMS2 samples show Low to Medium levels of MOMTGFB, while most patients classified as CMS4 cluster in the MOMTGFB-High area. CMS1 and MSI patients are evenly distributed throughout the range of MOMTGFB scores values indicating, as previously described, that a high % of these theoretically good prognosis patients exhibit a high degree of TGFβ-activated TME and, therefore, should be considered as bad prognosis patients, likely candidates to receive TGFβ signaling inhibitory therapies, and unlikely to respond to immunecheckpoint therapies alone.

Figure 8: Box-Plot graphs depicting overall TGFβ, F-TBRS, T-TBRS and Ma-TRBRS expression levels in 3 groups of patients according to their MOMTGFB score (Low, Medium and High) in stomach adenocarcinoma (STAD) patients from the TCGA cohort. Results are presented for all samples, and according to the MSI/MSS status of the patients (second and third rows). Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 9. Box-Plot graphs depicting overall TGFβ, F-TBRS, T-TBRS and Ma-TRBRS expression levels in 3 groups of patients according to their MOMTGFB score (Low, Medium and High) in bladder cancer patients from the TCGA cohort. Results are shown for all samples. Each dot is a sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 10. Box-Plot graphs depicting overall TGFβ, F-TBRS, T-TBRS and Ma-TRBRS expression levels in 3 groups of patients according to their MOMTGFB score (Low, Medium and High) in pancreas adenocarcinoma patients from the TCGA cohort. Results are shown for all samples. Each dot is a sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 11. Box-Plot graphs depicting overall TGFβ, F-TBRS, T-TBRS and Ma-TRBRS expression levels in 3 groups of patients according to their MOMTGFB score (Low, Medium and High) in prostate carcinoma patients from the GSE21034 cohort. Results are shown for all samples. Each dot is a sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 12. Box-Plot graphs depicting overall TGFβ, F-TBRS, T-TBRS and Ma-TRBRS expression levels in 3 groups of patients according to their MOMTGFB score (Low, Medium and High) in lung cancer patients from the GSE31210 cohort. Results are shown for all samples. Each dot is a sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 13. Box-Plot graphs depicting overall TGFβ, F-TBRS, T-TBRS and Ma-TRBRS expression levels in 3 groups of patients according to their MOMTGFB score (Low, Medium and High) in various subtypes of breast cancer patients (LumA; LumB; Basal; Her2) in the Metabric cohort. Results are shown for all samples in each subtype. Each dot is a sample. Sample groups of low, medium and high expression levels were defined using the mean and -1 standard deviation as cutoffs. Tukey box plots have whiskers of maximum 1.5 times the interquartile range; the boxes represent first, second (median) and third quartiles. Patients with high MOMTGFB score present tumors characterized by a higher degree of TGFβ-activated TME using as surrogates F-, T- and Ma-TBRSs.

Figure 14. MOMTGFB genes identify the microenvironment of tumors that respond to TGFβ inhibitory therapies, and is also a biomarker of response. Tumors were generated in immunocompetent mice through implantation in the caecum of mouse tumor organoids (MTOs) mutant in the 4 main pathways driving colorectal cancer progression. MOMTGFB scores are high in tumors before treatment, indicative that they are likely to respond to TGFβ inhibitory therapies. Upon treatment with small molecule TGFβ receptor 1 inhibitor LY2157299 (Galunisertib) MOMTGFB scores are reduced in the treated tumors , further indicating there has been a response to the treatment. Tukey box plots have whiskers that cover the maximum and minimum values of the scores; the boxes represent first, second (median) and third quartiles.

## DETAILED DESCRIPTION OF THE INVENTION

### Definitions

The terms "subject", or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "cancer patient" and "subject suffering from cancer" are used herein interchangeably. It may refer to those subjects diagnosed after a confirmatory test (e.g., biopsy and/or histology) and subjects suspected of having cancer. The term "subject suspected of having cancer" as used herein, refers to a subject that presents one or more signs or symptoms indicative of a cancer and is being screened for cancer. A subject suspected of having cancer encompasses for instance an individual who has received a preliminary diagnosis *(e.g.,* an X-ray computed tomography scan showing a mass) but for whom a confirmatory test *(e.g.,* biopsy and/or histology) has not been done or for whom the stage of cancer is not known.

The terms "cancer" and "cancerous" refer to or describe the physiological condition in mammals that is typically characterized by unregulated cell growth. Included in this definition are benign and malignant cancers or tumors, as well as dormant tumors or micrometastases. Examples of cancer include but are not limited to, carcinoma, lymphoma, blastoma, sarcoma, and leukemia. More particular examples of such cancers include breast cancer, squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, colon cancer, colorectal cancer, rectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

The term "tumor" is an abnormal mass of tissue that may be benign, premalignant, or cancerous. Preferably, this "tumor" is a cancerous tumor.

The term "metastasis" as used herein refers to distant metastasis affecting organs other than the primary tumor site. Metastasis may be defined as the process by which cancer spreads or transfers from the primary site to other regions of the body with the development of a similar cancerous lesion at the new location (see for instance: Chambers AF et al., Nat Rev Cancer 2002; 2: 563-72). For instance, in colorectal cancer, metastasis in another organ (e.g., the liver) typically shows an enteroid adenocarcinoma pattern. A "metastatic" or "metastasizing" cell is typically one that loses adhesive contacts with neighboring cells and migrates via the bloodstream or lymph from the primary site of disease to invade neighboring body structures.

The term "tumor microenvironment" or "TME" as used herein refers to a complex structure composed of extracellular matrix proteins (mainly, type I collagen) and various cell types including mesenchymal cells (cancer-associated fibroblasts [CAFs]), endothelial cells and pericytes, nerve cells, immune cells, and bone marrow-derived stem cells that generally surround and feed a tumor. A tumor can change its microenvironment, and the microenvironment can affect how a tumor grows and spreads.

The term "TGFβ-activated microenvironment" or "TGFβ-activated TME" as used herein, refers to a tumor microenvironment wherein the cells surrounding the tumor express TGFβ receptors and respond to elevated TGFβ levels present in the TME. TGFβ has been reported to regulate fibrosis, angiogenesis, and immune cell function in the context of tumors (Neuzillet et al., Pharmacology & Therapeutics 147 (2015) 22-31; Batlle and Massague, Immunity 50 (2019), 924-940).

The term "TGFβ response signature" or "TBRS" as used herein refers to the gene expression program induced by addition of TGFβ1 in cultures of normal tissue derived T cells (T-TBRS), macrophages (Ma-TBRS) or fibroblasts (F-TBRS) It was selected as TBRS the full set of genes upregulated by TGFβ signaling in these cell cultures (>2 fold, $p<0.05$). F-TBRS is composed of 165 genes, T-TBRS of 76 and Ma-TBRS of 1125 genes. Each signature reflects the transcriptional activity of TGFβ signaling in fibroblasts, macrophages and T cells respectively. F-TBRS, T-TBRS and Ma-TBRS were previously shown by the inventors to act as surrogates of the degree of activation of the most abundant cell types in the TME of CRCs by TGFβ (Calon et al., Cancer Cell 22 (2012) 571-584; Calon et al., Nature Genetics 47:4 (2015) 320-332). Accordingly, TGFβ activation of fibroblasts, T-cells and macrophages can be assessed by determining expression of F-TBRS, T-TBRS and Ma-TBRS, respectively.

The term "organoid" or "cancer organoid" as used herein refers to small, self-organized three dimensional tissue cultures derived from primary tumors or metastasis. They can be maintained indefinitely in the appropriate cell culture conditions. When inoculated in experimental models, they faithfully recapitulate many of the traits of the tumor of origin.

The term "treating", as used herein, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse). The term "treatment", as used herein, unless otherwise indicated, refers to the act of "treating".

The term "therapeutically effective amount" as used herein refers to an amount that is effective, upon single or multiple dose administration to a subject (such as a human patient) in the treatment of a disease, disorder or pathological condition.

The term "combination therapy" or "combination treatment" are used herein indistinctively, and is meant to comprise the administration of the referred therapeutic agents to a subject suffering from cancer, in the same or separate pharmaceutical formulations, and at the same time or at different times. If the therapeutic agents are administered at different times they should be administered sufficiently close in time to provide for the combined effect (e.g. potentiating or synergistic response) to occur. The particular combination of therapies to employ in a combination regimen will take into account compatibility of the desired therapeutics and/or procedures and/or the desired therapeutic effect to be achieved. It will be appreciated that the therapies employed may achieve a desired effect for the same disorder (for example, anti-cancer effects), and/or they may achieve different effects (e.g., control of any adverse effects).

The term "single agent" as used herein relates to the use of an active ingredient sufficiently separate in time from another active ingredient to prevent for the potentiating or synergistic response to occur. More specifically, the use as "single agent" does not encompass the use as a "combination therapy".

The term "anti-cancer treatment" as used herein may include any treatment to stop or prevent cancer, including but not limited to surgery, radiotherapy, anti-cancer agents and any other existing therapies or to be developed.

The term "anti-cancer agent" as used herein refers to any therapeutic agents useful in treating cancer. Examples of anti-cancer therapeutic agents include, but are limited to, e.g., an inhibitor of the TGFβ signaling pathway, chemotherapeutic agents, growth inhibitory agents, cytotoxic agents, anti-hormonal agents, agents used in radiation therapy, anti-angiogenesis agents, apoptotic agents, anti-tubulin agents, and other agents to treat cancer, such as anti-HER-2 antibodies (e.g., Herceptin®), anti-CD20 antibodies, an epidermal growth factor receptor (EGFR) antagonist (e.g., a tyrosine kinase inhibitor), HER1/EGFR inhibitor (e.g., erlotinib (Tarceva<®>)), platelet derived growth factor inhibitors (e.g., Gleevec™ (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

The terms "TGFβ inhibitor" or "TGFβ (signaling) pathway inhibitor" or "inhibitor of the TGFβ signaling pathway" are used herein indistinctively and refer to an agent inhibiting TGFβ signaling by any means. Illustrative, non-limiting examples of "TGFβ inhibitors" are those inhibiting TGFβ signaling pathway at any of the (i) ligand level, (ii) the ligand-receptor level or the (ii) intracellular level.

(i) Agents inhibiting the TGF β pathway at the ligand level include antisense oligonucleotides which may be delivered directly intravenously or engineered into immune cells to prevent TGFβ synthesis (for example, trabedersen [AP12009], an antisense oligonucleotide targeting TGFβ2; and Lucanix® [belagenpumatucel-L], a TGFβ2 antisense gene-modified allogeneic cancer cell vaccine);

(ii) Agents inhibiting the TGFβ pathway at the ligand-receptor level include *ligand-traps* (e.g., TGFβ-neutralizing affinity ligands [e.g., monoclonal antibodies] and soluble receptors), *antibodies or molecules that prevent TGFβ release from latent complexes,* like antibodies against GARP (Cuende et al., Science Translational Medicine 7 (2015), issue 284, pp. 284ra56) or antibodies against αvβ8 integrin that blocks the release of active TGFβ by cancer cells (N. Takasaka et al., JCI Insight. 3 (2018), doi:10.1172/jci.insight.122591); and *anti-TGFβ-receptor affinity ligands* to prevent ligand-receptor interaction (for example, fresolimumab, a pan-TGFβ antibody; disitertide [P144], a peptidic TGFβ1 inhibitor specifically designed to block the interaction with its receptor; and IMCTR1 [LY3022859], a monoclonal antibody against TGFβRII) ; and

(iii) Agents inhibiting the TGFβ pathway intracellular level include TGFβ receptor kinase inhibitors to prevent signal transduction (for example, galunisertib [LY2157299], a small molecule inhibitor of TGFβRI.

Particular examples of TGFβ pathway inhibitors in development in cancer are provided in Table 1 of Neuzillet et al. (Pharmacology & Therapeutics 147 (2015) 22-31):

TGFβ pathway inhibitors in development in cancer.

| Name | Targets | Trial identifier | Current status |
|---|---|---|---|
| *TGFβ ligand inhibitors* | | | |
| Lerdelimumab (CAT-152) Genzyme® | TGFβ2 | | Development stopped |
| Metelimumab Genzyme® | TGFβ1 | | Development stopped |
| Fresolimumab (GC1008) Genzyme®/Aventis® | TGFβ1, -β2, -β3 | NCT00356460<br>NCT00923169<br>NCT01472731<br>NCT01112293<br>NCT01401062 | Results in RCC, melanoma, mesothelioma and glioma; combination phase I/II in progress in breast cancer |
| LY2382770 Eli Lilly® | TGFβ1 | | In progress outside oncology |
| Trabedersen (AP12009) Antisens Pharma® | TGFβ2 | NCT00844064<br>NCT00431561<br>NCT00761280 | Results in glioma, PDAC, CRC, melanoma and glioblastoma |
| Lucanix (Belagenpumatucel-L) NovaRx Corporation® | TGFβ2 | NCT01058785<br>NCT00676507 | Results in glioma and NSCLC; combination phase I in progress |
| FANG™ Vaccine (rhGMCSF/shRNAfurin) Gradalis® | TGFβ1, -β2 | NCT01061840<br>NCT01309230<br>NCT01505166<br>NCT01453361 | In progress in melanoma, CRC and ovarian cancer |
| Disitertide (P144) Digna Biotech® | TGFβ1 | | In progress outside oncology |
| *TGFβ receptor inhibitors* | | | |
| Galunisertib (LY2157299) Eli Lilly® | TGFβRI | NCT01246986<br>NCT01373164<br>NCT01220271<br>NCT02178358<br>NCT01582269 | Phase II in progress in PDAC, HCC, glioma and glioblastoma |
| TEW-7197 MedPacto® | TGFβRI | NCT02160106 | Phase I in progress |
| PF-03446962 Pfizer® | ALK-1 (TGFβRI) | NCT00557856<br>NCT01337050<br>NCT01911273<br>NCT01486368<br>NCT01620970<br>NCT02116894 | Results of phase I; phase II results pending in HCC and in progress in malignant pleural mesothelioma and refractory urothelial carcinoma; combination phase I in progress with regorafenib in CRC |
| IMC-TR1 (LY3022859) Eli Lilly® | TGFβRII | NCT01646203 | Phase I in progress |

CRC: colorectal carcinoma; HCC: hepatocellular carcinoma; NSCLC: non-small cell lung carcinoma; PDAC: pancreatic ductal adenocarcinoma; RCC:rRenal cell carcinoma.

and in Table 1 of Batlle and Massagué (Immunity (2019) 50(4):924-940):

| Therapy | Target | Drug | Progress to clinic |
|---|---|---|---|
| Small molecule inhibitors | TGFBR1 kinase | Galunisertib (GAL) | Multiple completed safety and efficacy phase 1 clinical trials in HCC, glioblastoma and other tumor types.<br>Several on-going phase 1/2 trials:<br>- Metastatic breast cancer (GAL + Radiation)<br>- Metastatic pancreatic cancer (GAL + Durvalumab)<br>- Advanced Hepatocellular Carcinoma (GAL + Stereotactic radiotherapy).<br>- Metastatic Androgen Receptor Negative Triple<br>- Negative Breast Cancer (GAL and Paclitaxel)<br>- Colorectal cancer (GAL + Capecitabine)<br>- Rectal cancer (GAL + chemoradiation)<br>- Advanced refractory solid tumors (GAL + Nivolumab)<br>- Metastatic Prostate cancer (GAL + Enzalutamide) |
| | | Vactosertib (VAC) | Safety and efficacy phase trials in advanced stage solid tumors<br>Several on-going phase 1/2 trials:<br>- Metastatic Gastric Cancer (VAC + Paclitaxel)<br>- Advanced NSCLC (VAC + Durvalumab)<br>- Metastatic Colorectal and Gastric cancer (VAC + Pembrolizumab)<br>- Advanced desmoid tumors (VAC + Imatinib) |
| | | LY3200882 | Phase 1: safety and dose escalation in solid tumors |
| | | PF-06952229 | Phase 1: safety and dose escalation in breast cancer and prostate cancer in monotherapy or combination with several drugs (palbociclib, Letrozole, Enzalutamide) |
| | | AZ12601011<br>AZ12799734 | Preclinical development (Spender et al., 2019) |

(continued)

| Therapy | Target | Drug | Progress to clinic |
|---|---|---|---|
| Antibodies | Blocking pan-TGF-β (TGF-β1, TGF-β2, TGF-β3) | Fresolimumab | Completed safety and efficacy phase 1 trials in renal cell carcinoma, melanoma and glioma.<br>On-going phase 1/2 trials:<br>- Relapsed malignant mesothelioma<br>- Advanced renal cell carcinoma and melanoma<br>- Early stage NSCLC (FRESO + stereotactic ablative radiotherapy).<br>- Metastatic breast cancer (FRESO + Radiation) |
| | Blocking pan-TGF-β | SAR439459 | Phase 1 (safety and dose escalation) for advanced solid tumors in monotherapy or combination with anti-PD1 antibodies. |
| | Blocking pan-TGF-β | NIS793 | Phase 1 in combination with anti-PD-1 antibodies for patients with advanced malignancies (breast, lung, HCC, CRC, Pancreatic cancer, Renal cancer) |
| | Blocking TGF-β1 & TGF-β2 specific | XPA-42-089 | Preclinical cancer models (Dodagatta-Marri et al., 2019) |
| | Chimeric antibody-TGF-β traps | CTLA4-TGFβRII | Preclinical cancer models (Ravi et al., 2018) |
| | | PDL1-TGFβRII (M7824) | Ongoing Phase 1/2:<br>- NSCLC (compared to Pembrolizumab)<br>- Triple negative breast cancer (M7824 + Eribulin)<br>- Prostate cancer<br>- Metastatic colorectal cancer<br>- Cholangiocarcinoma and Gallbladder |
| | GARP | ABBV151 | Phase 1 for advanced solid tumors as monotherapy or in combination with PD-1 antibodies. |
| | αvβ8 Integrins | | Preclinical cancer models (Takasaka et al., 2018) |
| | LAP | | Preclinical cancer models (Gabriely et al., 2017) |
| Receptor-based TGF-β traps | | AVID200 | Phase 1 (safety and dose escalation) for advanced and metastatic solid tumors. |
| Adoptive cell transfer | | Autologous CD8+ T cells expressing a dnTGFR2 | Phase 1 trial in chemorefractory Epstein Barr Hodgkin lymphoma |

The term "reduce or inhibit" as used herein may refer to the ability to cause an overall decrease preferably of 20% or greater, more preferably of 50% or greater, and most preferably of 75%, 85%, 90%, 95%, or greater. Reduce or inhibit can refer for instance, to the biological activity of an active ingredient or a ligand (e.g. TGF β activity), to the symptoms of the disorder being treated, the presence or size of metastases, the size of the primary tumor, etc.

The term "cytotoxic agent" as used herein refers to a substance that inhibits or prevents the function of cells and/or causes destruction of cells. The term is intended to include radioactive isotopes (e.g. At<211>, I<131>, I<125>, Y<90>, Re<186>, Re<188>, Sm<153>, Bi<212>, P<32>and radioactive isotopes of Lu), chemotherapeutic agents, and toxins such as small molecule toxins or enzymatically active toxins of bacterial, fungal, plant or animal origin, including fragments and/or variants thereof.

The term "chemotherapeutic agent" as used herein refers to a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1 and calicheamicin omegal1; dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel, ABRAXANE® Cremophor-free, albumin-engineered nanoparticle formulation of paclitaxel, and TAXOTERE® doxetaxel; chloranbucil; GEMZAR® gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluorometlhylornithine (DMFO); retinoids such as retinoic acid; capecitabine; combretastatin; leucovorin (LV); oxaliplatin, including the oxaliplatin treatment regimen (FOLFOX); lapatinib (Tykerb®); inhibitors of PKC-alpha, Raf, H-Ras, EGFR (e.g., erlotinib (Tarceva<®>)) and VEGF-A that reduce cell proliferation and pharmaceutically acceptable salts, acids or derivatives of any of the above.

The term "anti-hormonal agents" as used herein refers to agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY 117018, onapristone, and FARESTON· toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); epidermal growth factor; hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha and TGF-$\beta$ ; insulin-like growth factor-I and -II; erythropoietin (EPO); osteoinductive factors; interferons such as interferon-alpha, -beta and -gamma colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

The term "growth inhibitory agent" as used herein refers to a compound or composition which inhibits growth of a cell in vitro and/or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13 .

The term "radiation therapy" or "radiotherapy" as used herein refers to the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

The term "marker" or "biomarker" as used herein may refer to markers of disease, prognostic or predictive markers which are typically substances found in a bodily sample that can be easily measured. Said bodily sample can be for instance a blood, plasma or feces sample. The term biomarker encompasses biophysical and biochemical determinations, including genetic and serological markers.

The term "prognosis" as used herein refers to predicting disease progression or outcome. More specifically, "prognostic markers" may refer to patient or tumor characteristics that predict outcome (usually survival) independent of the treatment. Thus, they are usually identified and validated in patients who receive no therapy or surgical therapy only. The goal of identifying prognostic markers is to define patient subpopulations with significantly different anticipated outcomes, which might benefit from different therapies. Good prognostic patients may not require additional treatment beyond the primary surgical resection, while poor prognostic patients may derive improved survival benefit from adjuvant therapy or other closer clinical follow up or therapeutic strategy.

"Predictive markers", on the other hand, may refer to patient or tumor characteristics that predict benefit from specific treatments (either in terms of tumor shrinkage or survival). In other words, the differences in tumor response or survival benefit between treated versus untreated patients will be significantly different in those positive or negative for the predictive marker (Zhu CQ and Tsao MS, 2014).

The term "substantially identical" sequence as used herein refers to a sequence which is at least about 95%, preferably at least about 96%, 97%, 98%, or 99% identical to a reference sequence. Identity percentage between the two sequences can be determined by any means known in the art, for example the Needleman and Wunsch global alignment algorithm.

The term "probe" as used herein refers to synthetic or biologically produced nucleic acids, between 10 and 285 base pairs in length which contain specific nucleotide sequences that allow specific and preferential hybridization under predetermined conditions to target nucleic acid sequences, and optionally contain a moiety for detection or for enhancing assay performance. A minimum of ten nucleotides is generally necessary in order to statistically obtain specificity and to form stable hybridization products, and a maximum of 285 nucleotides generally represents an upper limit for length in which reaction parameters can be easily adjusted to determine mismatched sequences and preferential hybridization. Probes may optionally contain certain constituents that contribute to their proper or optimal functioning under certain assay conditions. For example, probes may be modified to improve their resistance to nuclease degradation (e.g., by end capping), to carry detection ligands (e.g., fluorescein), to carry ligands for purification or enrichment purposes (e.g. biotin) or to facilitate their capture onto a solid support (e.g., poly-deoxyadenosine "tails").

The term "primers" as used herein refers to oligonucleotides that can be used in an amplification method, such as a polymerase chain reaction ("PCR"), to amplify a nucleotide sequence. Primers are designed based on the polynucleotide sequence of a particular target sequence.

The term "specific" as used herein in connection with a nucleotide sequence means that a nucleotide sequence will hybridize to/amplify a predetermined target sequence and will not substantially hybridize to/amplify a non-target sequence under the assay conditions, generally stringent conditions are used.

The term "hybridization" as used herein refers to a process by which, under predetermined reaction conditions, two partially or completely complementary strands of nucleic acid are allowed to come together in an antiparallel fashion to form a double-stranded nucleic acid with specific and stable hydrogen bonds, following explicit rules pertaining to which nucleic acid bases may pair with one another.

The term "substantial hybridization" means that the amount of hybridization observed will be such that one observing the results would consider the result positive with respect to hybridization data in positive and negative controls. Data which is considered "background noise" is not substantial hybridization.

The term "stringent hybridization conditions" means approximately 35°C to 65°C in a salt solution of approximately 0.9 molar NaCl. Stringency may also be governed by such reaction parameters as the concentration and type of ionic species present in the hybridization solution, the types and concentrations of denaturing agents present, and the temperature of hybridization. Generally as hybridization conditions become more stringent, longer probes are preferred if stable hybrids are to be formed. As a rule, the stringency of the conditions under which hybridization is to take place will dictate certain characteristics of the preferred probes to be employed.

The term "affinity reagent" may refer to a ligand (e.g., antibody, peptide, protein, nucleic acid or small molecule) that selectively captures (binds to) a target molecule through specific molecular recognition, typically with a binding affinity in the nanomolar to sub-nanomolar range. For example, the affinity reagent may be an aptamer, antibody or antibody-mimetic.

The term "affinity" as used herein may refer to the equilibrium constant for the dissociation of an antigen with an antigen-binding molecule (KD), and is considered a measure for the binding strength between an antigenic determinant and an antigen-binding site on the antigen -binding molecule: the lesser the value of the KD, the stronger the binding strength between an antigenic determinant and the antigen-binding molecule (alternatively, the affinity can also be expressed as the association constant (KA), which is 1/KD). It will be clear to the skilled person that the dissociation constant may be the actual or apparent dissociation constant.

The term "aptamer" or "nucleic acid aptamer" as used herein may refer to an isolated or purified single-stranded nucleic acid (RNA or DNA) that binds with high specificity and affinity to a target through interactions other than Watson-Crick base pairing. An aptamer has a three dimensional structure that provides chemical contacts to specifically bind to a target. Unlike traditional nucleic acid binding, aptamer binding is not dependent upon a conserved linear base sequence, but rather a particular secondary or tertiary structure. That is, the nucleic acid sequences of aptamers are non-coding sequences. Any coding potential that an aptamer may possess is entirely fortuitous and plays no role whatsoever in the binding of an aptamer to a target. A typical minimized aptamer is 5-15 kDa in size (15-45 nucleotides), binds to a target with nanomolar to sub-nanomolar affinity, and discriminates against closely related targets (e.g., aptamers will typically not bind to other proteins from the same gene or functional family).

The term "antibody" as used herein may refer to an immunoglobulin or an antigen-binding fragment thereof. Unless otherwise specified, the term includes, but is not limited to, polyclonal, monoclonal, monospecific, multispecific, humanized, human, chimeric, synthetic, recombinant, hybrid, mutated, grafted, and *in vitro* generated antibodies. The antibody can include a constant region, or a portion thereof, such as the kappa, lambda, alpha, gamma, delta, epsilon and mu constant region genes. For example, heavy chain constant regions of the various isotypes can be used, including: $IgG_1$, $IgG_2$, $IgG_3$, $IgG_4$, IgM, $IgA_1$, $IgA_2$, IgD, and IgE. By way of example, the light chain constant region can be kappa or lambda. In certain embodiments, the term "antibody" may also refer to antibody derivatives, such as antibody-based fusion proteins or antibodies further modified to contain additional non-proteinaceous moieties, such as water-soluble polymers, e.g. polyethylene glycol (PEG).

The terms "antigen-binding domain" and "antigen-binding fragment" refer to a part of an antibody molecule that comprises amino acids responsible for the specific binding between antibody and antigen. For certain antigens, the antigen-binding domain or antigen-binding fragment may only bind to a part of the antigen. The part of the antigen that is specifically recognized and bound by the antibody is referred to as the "epitope" or "antigenic determinant." Antigen-binding domains and antigen-binding fragments include Fab; a $F(ab')_2$ fragment (a bivalent fragment having two Fab fragments linked by a disulfide bridge at the hinge region); a Fv fragment; a single chain Fv fragment (scFv); a Fd fragment (having the two $V_H$ and $C_H1$ domains); single domain antibodies (sdAbs; consisting of a single $V_H$ domain), and other antibody fragments that retain antigen-binding function. The Fab fragment has $V_H$-$C_H1$ and $V_L$-$C_L$ domains covalently linked by a disulfide bond between the constant regions. The $F_v$ fragment is smaller and has $V_H$ and $V_L$ domains non-covalently linked. The $scF_v$ contains a flexible polypeptide that links (1) the C-terminus of $V_H$ to the N-terminus of $V_L$, or (2) the C-terminus of $V_L$ to the N-terminus of $V_H$. The sdAbs include heavy chain antibodies naturally devoid of light chains and single-domain antibodies derived from conventional four chain antibodies. These antigen-binding domains and fragments are obtained using conventional techniques known to those with skill in the art, and are evaluated for function in the same manner as are intact immunoglobulins.

The term "recombinant antibody" as used herein refers to an antibody produced or expressed using a recombinant expression vector, where the expression vector comprises a nucleic acid encoding the recombinant antibody, such that introduction of the expression vector into an appropriate host cell results in the production or expression of the recombinant antibody. Recombinant antibodies may be chimeric or humanized antibodies, mono- or multi-specific antibodies.

The term "an antibody mimetic" (AbM) as used herein refers to single-domain scaffolds, which have been engineered to bind therapeutic targets with affinity and specificity that match that of natural antibodies. Antibody mimetics have been developed utilizing an immunoglobulin-like fold, for example, fibronectin type III, NCAM and CTLA-4. Other mimetics scaffolds bearing no similarity to immunoglobulin folds have also been obtained. Non-limiting examples of said scaffolds are DARPins, anticalins, affibodies, adnectins, fynomers, etc. (see for instance, Weidle et al. Cancer Genomics & Proteomics. 2013, 10:1-18; Lofblom, J. et al., Curr. Opin. Biotechnol. 2011, 22: 843-848; Banta, S. et al., Annu. Rev. Biomed. Eng., 2010, 15: 93-113).

## Method for identifying tumors with a TGFβ-activated TME

In a first aspect, the present invention provides a method for determining whether a patient's tumor has a TGFβ-activated microenvironment, wherein said method comprises or consists of:

a) determining the expression levels of a group of genes comprising or consisting of CRYAB, NNMT, and SPRY4 in a biological sample isolated from said patient;
b) calculating a score from the expression levels of the genes determined in step a); and
c) determining whether a patient's tumor has a TGFβ-activated microenvironment according to the score obtained in b).

**Step (a)** of the methods of the invention comprises or consists of determining in said biological sample the expression levels of the genes defined above.

The **CRYAB** (crystallin alpha B) gene has been described in humans (NCBI Gene ID: 1410). Mammalian lens crystallins are divided into alpha, beta, and gamma families. Alpha crystallins are composed of two gene products: alpha-A and alpha-B, for acidic and basic, respectively. Alpha crystallins can be induced by heat shock and are members of the small heat shock protein (HSP20) family. They act as molecular chaperones although they do not renature proteins and release them in the fashion of a true chaperone; instead they hold them in large soluble aggregates. Post-translational modifications decrease the ability to chaperone. These heterogeneous aggregates consist of 30-40 subunits; the alpha-A and alpha-B subunits have a 3:1 ratio, respectively. Two additional functions of alpha crystallins are an autokinase activity and participation in the intracellular architecture. The encoded protein has been identified as a moonlighting protein based on its ability to perform mechanistically distinct functions. Alpha-A and alpha-B gene products are differentially expressed; alpha-A is preferentially restricted to the lens and alpha-B is expressed widely in many tissues and organs. Elevated expression of alpha-B crystallin occurs in many neurological diseases; a missense mutation co-segregated in a family with a desmin-related myopathy.

CRYAB gene is located in chromosome 11 (11q23.1; Reference GRCh38.p12 Primary Assembly) and has 5 exons. Alternative splicing results in multiple transcript variants; currently, the following gene-expression product variants have been described:

| **mRNA - NCBI Reference Sequence:** | **Protein - NCBI Reference Sequence/ UniProtKB/Swiss-Prot:** |
|---|---|
| NM_001289807.1 transcript variant 2, mRNA | NP_001276736.1/P02511 alpha crystallin B chain isoform 1 |
| NM_001289808.1 transcript variant 3, mRNA. | NP_001276737.1/P02511 alpha crystallin B chain isoform 1 |
| NM_001330379.1 transcript variant 4, mRNA | NP_001317308.1/A0A024R3B9 alpha crystallin B chain isoform 2 |
| NM_001885.2 transcript variant 1, mRNA. | NP_001876.1/P02511 alpha crystallin B chain isoform 1 |

The canonical sequence of the mRNA expression product of human CRYAB gene corresponds to NM 001885.2 transcript variant 1, mRNA and is referred as SEQ ID NO:1:

```
  1 aggaggagga ggcaccaaac ggcctgggtg gatagaaggg ggacaaggag gcacacccag
 61 gccggcaaag agcaggtatc agcactgcaa gcaccaagtg tgtcttgagc tcagtgagta
121 ctgggtatgt gtcacattgc caaatcccgg atcacaagtc tccatgaact gctggtgagc
181 taggataata aaacccctga catcaccatt ccagaagctt cacaagactg catatataag
241 gggctggctg tagctgcagc tgaaggagct gaccagccag ctgacccctc acactcacct
301 agccaccatg gacatcgcca tccaccaccc ctggatccgc cgccccttct ttcctttcca
361 ctcccccagc cgcctctttg accagttctt cggagagcac ctgttggagt ctgatctttt
421 cccgacgtct acttccctga gtcccttcta ccttcggcca ccctccttcc tgcgggcacc
481 cagctggttt gacactggac tctcagagat gcgcctggag aaggacaggt tctctgtcaa
541 cctggatgtg aagcacttct ccccagagga actcaaagtt aaggtgttgg gagatgtgat
601 tgaggtgcat ggaaaacatg aagagcgcca ggatgaacat ggtttcatct ccagggagtt
661 ccacaggaaa taccggatcc cagctgatgt agaccctctc accattactt catccctgtc
721 atctgatggg gtcctcactg tgaatggacc aaggaaacag gtctctggcc ctgagcgcac
781 cattcccatc acccgtgaag agaagcctgc tgtcaccgca gcccccaaga aatagatgcc
841 ctttcttgaa ttgcattttt taaaacaaga aagtttcccc accagtgaat gaaagtcttg
901 tgactagtgc tgaagcttat taatgctaag ggcaggccca aattatcaag ctaataaaat
961 atcattcagc aacagataaa aaaaaaaaaa aaaaaaaa
```

The term "CRYAB mRNA" as used herein may refer to any of CRYAB gene transcript variants, preferably, to any transcript variants corresponding to isoform 1 (e.g., transcript variants 1, 2 or 3), more preferably, said sequence is SEQ ID NO: 1.

The canonical sequence of the protein expression product of human CRYAB gene corresponds to alpha crystallin B chain isoform 1 (UniProtKB P02511; January 1, 1990 - v2) and is referred as SEQ ID NO:2:

```
MDIAIHHPWIRRPFFPFHSPSRLFDQFFGEHLLESDLFPTSTSLSPFYLRPPSFLRAPSW
FDTGLSEMRLEKDRFSVNLDVKHFSPEELKVKVLGDVIEVHGKHEERQDEHGFISREFHR
KYRIPADVDPLTITSSLSSDGVLTVNGPRKQVSGPERTIPITREEKPAVTAAPKK
```

The term "CRYAB protein" as used herein may refer to any of the protein isoforms, preferably it refers to SEQ ID NO: 2.

The **NNMT** (nicotinamide N-methyltransferase) gene has been described in humans (NCBI Gene ID: 4837). N-methylation is one method by which drug and other xenobiotic compounds are metabolized by the liver. This gene encodes the protein responsible for this enzymatic activity which uses S-adenosyl methionine as the methyl donor.

The NNMT gene is located in chromosome 11 (11q23.2; Reference GRCh38.p12 Primary Assembly) and has 3 exons. The canonical sequence of the mRNA expression product of human NNMT gene corresponds to NCBI Reference Sequence: NM_006169.2 and is referred as SEQ ID NO:3:

```
   1 gaggaggtgc ttgccagaca ctgggtcatg gcagtggtcg gtgaagctgc agttgcctag
  61 ggcagggatg gagagagagt ctgggcatga ggagagggtc tcgggatgtt tggctggact
 121 agattttaca gaaagcctta tccaggcttt taaaattact ctttccagac ttcatctgag
 181 actccttctt cagccaacat tccttagccc tgaatacatt tcctatcctc atctttccct
 241 tctttttttt cctttctttt acatgtttaa atttaaacca ttcttcgtga ccccttttct
 301 tgggagattc atggcaagaa cgagaagaat gatggtgctt gttaggggat gtcctgtctc
 361 tctgaacttt ggggtcctat gcattaaata attttcctga cgagctcaag tgctccctct
 421 ggtctacaat ccctggcggc tggccttcat cccttgggca agcattgcat acagctcatg
 481 gccctccctc taccataccc tccacccccg ttcgcctaag ctcccttctc cgggaatttc
 541 atcatttcct agaacagcca gaacatttgt ggtctatttc tctgttagtg tttaaccaac
 601 catctgttct aaaagaaggg ctgaactgat ggaaggaatg ctgttagcct gagactcagg
 661 aagacaactt ctgcagggtc actccctggc ttctggagga aagagaagga gggcagtgct
 721 ccagtggtac agaagtgaga cataatggaa tcaggcttca cctccaagga cacctatcta
 781 agccatttta accctcggga ttacctagaa aaatattaca agtttggttc taggcactct
 841 gcagaaagcc agattcttaa gcaccttctg aaaaatcttt tcaagatatt ctgcctagac
 901 ggtgtgaagg gagacctgct gattgacatc ggctctggcc ccactatcta tcagctcctc

 961 tctgcttgtg aatcctttaa ggagatcgtc gtcactgact actcagacca gaacctgcag
1021 gagctggaga agtggctgaa gaaagagcca gaggcctttg actggtcccc agtggtgacc
1081 tatgtgtgtg atcttgaagg gaacagagtc aagggtccag agaaggagga gaagttgaga
1141 caggcggtca agcaggtgct gaagtgtgat gtgactcaga gccagccact gggggccgtc
1201 cccttacccc cggctgactg cgtgctcagc acactgtgtc tggatgccgc ctgcccagac
1261 ctccccacct actgcagggc gctcaggaac ctcggcagcc tactgaagcc aggggggcttc
1321 ctggtgatca tggatgcgct caagagcagc tactacatga ttggtgagca gaagttctcc
1381 agcctccccc tgggccggga ggcagtagag gctgctgtga aagaggctgg ctacacaatc
1441 gaatggtttg aggtgatctc gcaaagttat tcttccacca tggccaacaa cgaaggactt
1501 ttctccctgg tggcgaggaa gctgagcaga cccctgtgat gcctgtgacc tcaattaaag
1561 caattccttt gacctgtca
```

The term "NNMT mRNA" as used herein may refer to any NNMT gene transcript variants, more preferably, said sequence is SEQ ID NO: 3.

The canonical sequence of the protein expression product of human NNMT gene corresponds to UniProtKB P40261-1; February 1, 1995 - v1) and is referred as SEQ ID NO:4:

```
MESGFTSKDTYLSHFNPRDYLEKYYKFGSRHSAESQILKHLLKNLFKIFCLDGVKGDLLI
DIGSGPTIYQLLSACESFKEIVVTDYSDQNLQELEKWLKKEPEAFDWSPVVTYVCDLEGN
RVKGPEKEEKLRQAVKQVLKCDVTQSQPLGAVPLPPADCVLSTLCLDAACPDLPTYCRAL
RNLGSLLKPGGFLVIMDALKSSYYMIGEQKFSSLPLGREAVEAAVKEAGYTIEWFEVISQ
SYSSTMANNEGLFSLVARKLSRPL
```

The term "NNMT protein" as used herein may refer to any of the protein isoforms, more preferably, said sequence is SEQ ID NO: 4.

**SPRY4** (Sprouty RTK Signaling Antagonist 4) gene has been described in humans (NCBI Gene ID: 81848). This gene encodes a member of a family of cysteine- and proline-rich proteins. The encoded protein is an inhibitor of the receptor-transduced mitogen-activated protein kinase (MAPK) signaling pathway. Activity of this protein impairs the formation of active GTP-RAS. Nucleotide variation in this gene has been associated with hypogonadotropic hypogonadism 17 with or without anosmia.

The SPRY4 gene is located in chromosome 5 (5q31.3; Reference GRCh38.p12 Primary Assembly) and has 5 exons. Alternative splicing results in a multiple transcript variants; currently, the following gene-expression product variants have been described:

| **mRNA - NCBI Reference Sequence:** | **Description** | **Protein - NCBI Reference Sequence/ UniProtKB/ Swiss-Prot:** |
|---|---|---|
| NM_001127496.1 transcript variant 2, mRNA. | This variant (2) differs in the 5' UTR, lacks a portion of the 5' coding region, and initiates translation at a downstream in-frame start codon, compared to variant 1. The encoded isoform (2) has a shorter N-terminus than isoform 1. | NP_001120968.1/Q9C004 protein sprouty homolog 4 isoform 2 |
| NM_001293289.1 transcript variant 3, mRNA. | This variant (3) contains multiple differences in the 5' region and initiates translation at a downstream in-frame start codon, compared to variant 1. The encoded isoform (2) has a shorter N-terminus than isoform 1. | NP001280218.1/Q9C004 protein sprouty homolog 4 isoform 2 |
| NM_001293290.1 transcript variant 4, mRNA. | This variant (4) differs in the 5' UTR, lacks a portion of the 5' coding region, and initiates translation at a downstream in-frame start codon, compared to variant 1. The encoded isoform (2) has a shorter N-terminus than isoform 1. | NP_001280219.1/Q9C004 protein sprouty homolog 4 isoform 2 |
| NM_030964.3 transcript variant 1, mRNA. | This variant (1) encodes the longer isoform (1). | NP_112226.2/Q9C004 protein sprouty homolog 4 isoform 1 |

Variants 2, 3, and 4 encode the same isoform (2).

The canonical sequence of the mRNA expression product of human SPRY4 gene corresponds to transcript variant 2 with NCBI Reference Sequence NM_001127496.1 and is referred as SEQ ID NO:5:

```
  1 attcataaaa aagccatttt cccaggcagt ggttgcaaca tcgccgcgga ggtagcgagc
 61 tgagctgaca gcgcggagct ggcgctgtgg agcgcaggga gccttgccgg ttcctccgac
121 cggcgtctgc gagtacagcg gcggctaacc tgccccggct tcaggattta cacagacgtg
181 gggcgatgct tgtgaccctg cagctcctca aaggccccta gaagcctgtt tctccgtaca
241 gtccaggacc tccagcccca tggagccccc gatcccacag agcgccccct tgactcccaa
301 ctcagtcatg gtccagcccc ttcttgacag ccggatgtcc cacagccggc tccagcaccc
361 actcaccatc ctacccattg accaggtgaa gaccagccat gtggagaatg actacataga
421 caaccctagc ctggccctga ccaccggccc aaagcggacc cggggcgggg ccccagagct
481 ggccccgacg cccgcccgct gtgaccagga tgtcacccac cattggatct ccttcagcgg
541 gcgccccagc tctgtgagca gcagcagcag cacatcctct gaccaacggc tcttagacca
601 catggcacca ccacccgtgg ctgaccaggc ctcaccaagg gctgtgcgca tccagcccaa
661 ggtggtccac tgccagccgc tggacctcaa gggcccggcg gtcccacccg agctggacaa
```

```
721 gcacttcttg ctgtgcgagg cctgtgggaa gtgtaaatgc aaggagtgtg catccccccg
781 gacgttgcct tcctgctggg tctgcaacca ggagtgcctg tgctcagccc agactctggt
841 caactatggc acgtgcatgt gtttggtgca gggcatcttc taccactgca cgaatgagga
901 cgatgagggc tcctgcgctg accacccctg ctcctgctcc cgctccaact gctgcgcccg
961 ctggtccttc atgggtgctc tctccgtggt gctgccctgc ctgctctgct acctgcctgc
1021 caccggctgc gtgaagctgg cccagcgtgg ctacgaccgt ctgcgccgcc ctggttgccg
1081 ctgcaagcac acgaacagcg tcatctgcaa agcagccagc ggggatgcca agaccagcag
1141 gcccgacaag cctttctgac agtttgtgtc gaagccccag tgctctgcct ggaaacctgg
1201 ttctcttctg acatctaaga agactgcagc aaggtcagag gttttagcct cctgaggctg
1261 accttgctag tctgcccact ccctaccccc agcttcggaa aatacagaga ccaccaccac
1321 gtaccctgta ttccccaagg tgatgaagaa gcactttggg gctttttttc agggtcctga
1381 aactttgtgt caaacagaca atgcaggggc agggtgtggt ttggggggaa atttttcttt
1441 ttcagaagac agaacacaga tgtggacaca tatccggaaa ctgcagctgc ttgaatgcct
1501 tcccagcccc tccttctccc tccctccctc cgcccccccc cccttcctct tttccattgt
1561 ctttggctct cacaggagct agctgcctgg gaggaattgt taactgagta ccagggtacc
1621 tttaaagaag acccttggag tcttctatac cttcttctcc ttccccatct cactccaccc
1681 cactttgtcc ctgatgtctt ggggaaggtg tagaacaccc tagcagttcc tattgtatat
1741 acttgggagc cactgagaac agaggacggc cagtgagtcc aagcctcgtt cctccttctg
1801 cctccccgga gccacaggat ggatttagga gccactgctc agtgcacttc tcccttccaa
1861 ctgcatcaac taactctcgg gggtgttctg ctcaccacac cgtccttcgg ttcttactga
1921 gtcacagact cgcctgccca ctacgtgtcc tgggttctct ctactcagat cccttccaga
1981 aactttatat gggtagagga agccagggcg gcaaatgcga gaccaaatat cattttgcca
2041 atgagtctga ggctgtggtc tctggatcca gtcattatgt ttttatagaa taattaaacc
2101 ggatgctaac ggtgttttaa aaaataataa taaaacaact tgtttccttt tggccacccc
2161 caggaagggc tgatttcaaa atctgggggc gagcaacctc aaggaacaca atttccctcc
2221 ctatcaacaa gaggatttta acagcaaaga agagaggcag cacctcccat tggcagaatg
2281 accgctgagc caggctgggt ttgggtttct tctcttctga ttctgctgct cactgtcata
2341 gccttttgtg tatagtgatg tgtctgtatc tttaatgtaa atagagagat gatgaaaaaa
2401 gagtctattt tagtgttagg aagccccagc aggggagtcg gaagagcttg gaagagctgg
2461 ggagagggta ggggaaaggt ttttccaggg gccactgggt ttgagccctg cttctgtgca
2521 cagccacacc accctctccc gacagccctc aaagacgtag caactctttc tctcaaggtg
2581 ctaaaggact cagaaggtgc agcacgtcca gtgggtaggt acttgttgca tgcaaaagct
2641 gtagtgtatc tggtccttcc tccccagctt ttgtgtgggg ttcttgcttt gtgtggtatt
2701 ttgttttccc ctctaatgag agggcatggc ctgagtcaga agagctaccc caggtgaaac
2761 tggaagtgca tgaggcagag cgtccgtagc atttccagtt tgttctgtat aggaacagag
2821 gtgcctccgg gaaggaggca gcgaggtagg tagctatgat aggcacctaa tgcttctcaa
2881 ggacttattt tttccttctt gaagactagt agtaacatct tatgatttag agtaagttga
2941 ttgtaaccat aggtatttat tgattggagg aagggagggt catattattt tcggctttat
3001 ttatgtaaca tttgctagct tgtaaaaggc gaatgtgaaa tattgcatct gcattttcca
3061 aggctgattc gtgtagctac ccttgccaca gttgtgacgg atgtatggat gttcttgaac
3121 atttcagaag gagtggtaga aaaaaacaca cattcagcca accacttata tgaattgaat
```

```
3181 gtatcagaag tgtactgaag ggactggaga tggttttcct cagatgaggg ggccccaaaa
3241 ttgatagtgc acatctgcac gctttctgcg aggcctcaga acttcccagg gcccctccct
3301 caaattgtct ccatgggaaa cttgacccag tggcaagttg cactttggtg atcttggtgg
3361 tctacacacc cgttctgtgg agagtcgatt tacataagct gtgtatacac acacacacac
3421 acacacacac acccctaccc cacactgact gtctaccgac aaagacccta tttcctggca
3481 aacggcctcc tgaaccctga ctttttgtgt acatacttgt aaacacggat ttttctgggt
3541 tttggtttgc tttttccttt tttcccccctg cccctgttct agcttgttct tcttggtttg
3601 ctttcaacct gcttgatgga tgtctgcaga gtgctctcta agagtccacc tcagtgcctc
3661 gtgtgctcag tggtcatggg aaaggagcga aggaaccatc cttggttctc ccagcttggt
3721 tgtgtagcaa tccctcagca ttgtttttct cagcttcttg gcaaaaatta aaacaacaac
3781 aacaacaaca acaacaacaa caaacagaag gataaactgg cttgcctgtg gaccctcccc
3841 ggctctgggg ccagtcgaga gccactgagg gacccagcac tcagagacac aacacacatg
3901 tgtagctgct tctggctgag tgtgtttcct gtcaccaatg gcctgtttgg ctggacgatg
3961 cctcggcttg accttttttg aaaagtgctg gttagttccc gcccctggta aacctggggt
4021 aggtgggggt tctgtcttaa ctcgaggggc acctgggatc caggacgctt ctaggggggct
4081 ctggctgccc gtgttaatga aggacagcgc ttccgcgagc accctgggaa ctgggtcttg
4141 ggtagcaaag ccctcccaga gaaaagattg ggcacaacta aggctttcct gagcaggaag
4201 ggggtgaaga ccaatccctt cctttggtcc tttggtacgc accccctcag agctgagatg
4261 gaagacatgg ctagttcttt tcagccttgt ggagcctgtc agtcgccatc atacctcgag
4321 tgaggcccag ctagataatg acttgtccaa gatggcacac gtggaaagtt gatctgcacc
4381 agaacccgga tgactgtcac cttgaagcat cctgttctcc ttctgtgctg tcccaggaag
4441 tgtctggcgg gcgtgggcag cacagctcta cactgtacga ttcactaggg catcctgcga
4501 gcctcactag ccttctggtt catgcctttg acaagcattt ttgtgccccc tctgcttact
4561 gtgacagtcg atgatgaatc ttgcgttgcc attttctgct gtgggtaact gcgtgcagtg
4621 tcttgccttg ctttctcttc ttactgtccc acagcttggt ttcatgttac aaacagaaaa
4681 gctcgaggct cccaccccgc cacatcccaa cttcatttcc ccctcactgt agcccatttc
4741 caccccacca caaagttgcc acaggttttc tttgtataga atatttattt tgaagctcta
4801 ttttaatagt atttatttta gaaagtctac tattgtaaga gttcttctgt ttgtgaagaa
4861 aaaaacaagt taaaaactga atgtactgat ttagaaaata tatataaata tatattgtta
4921 aatatacacg ggactgccgt t
```

The term "SPRY4 mRNA" as used herein may refer to any of SPRY4 gene transcript variants, preferably to any transcript variants corresponding to isoform 2 (e.g., transcript variants 2, 3 and 4), more preferably, said sequence is SEQ ID NO: 5 (variant 2, isoform 2).

The canonical sequence of the protein expression product of human SPRY4 gene corresponds to isoform 2 (NP_001120968.1, 18-FEB-2019) and is referred as SEQ ID NO:6:

>NP_001120968.1 protein sprouty homolog 4 isoform 2 [Homo sapiens]

```
MEPPIPQSAPLTPNSVMVQPLLDSRMSHSRLQHPLTILPIDQVKTSHVENDYIDNPSLALTTGPKRTRGG
```

APELAPTPARCDQDVTHHWISFSGRPSSVSSSSSTSSDQRLLDHMAPPPVADQASPRAVRIQPKVVHCQP
LDLKGPAVPPELDKHFLLCEACGKCKCKECASPRTLPSCWVCNQECLCSAQTLVNYGTCMCLVQGIFYHC
TNEDDEGSCADHPCSCSRSNCCARWSFMGALSVVLPCLLCYLPATGCVKLAQRGYDRLRRPGCRCKHTNS
VICKAASGDAKTSRPDKPF

The term "SPRY4 protein" as used herein may refer to any of the protein isoforms, preferably it refers to SEQ ID NO: 6.

Step (a) of the methods of the invention requires the determination of the expression levels of CRYAB, NNMT, and SPRY4 genes in a biological sample isolated from a subject suffering from cancer also referred herein as a "cancer patient". It may comprise the determination of the expression levels of 50 or less genes, 30 or less genes, 20 or less genes, 10 or less genes and 5 or less genes. In preferred embodiments, the group of genes which expression is determined in step a) consists of less than 19 genes, preferably these genes consist of CRYAB, NNMT, and SPRY4.

The methods of the invention can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In performing the method of the present invention, said biological sample from the cancer patient is preferably a sample containing tumor cells. Tumors or portions thereof may be surgically resected from the patient or obtained by routine biopsy. Preferably, a tumor sample is obtained from the primary tumor. In a particular embodiment, optionally in combination with any of the features or embodiments described above or below, said biological sample isolated from the subject is a tumor biopsy sample, preferably obtained from a resected tumor.

These types of samples are routinely used in the clinical practice and a person skilled in the art will know how to identify the most appropriate means for their obtaining and preservation. Once a sample has been obtained, it may be used fresh, it may be frozen or preserved using appropriate means (e.g., as a formalin-fixed, paraffin-embedded tissue sample). Such biological samples can be taken around the time of diagnosis, before, during or after treatment (e.g. surgical resection).

Methods for quantifying gene expression are well known in the art. Any current or future means for quantifying gene expression can be used in the methods of the invention.

In a particular embodiment, optionally in combination with one or more of the features or embodiments described herein, the mRNA expression level of these genes is determined. Molecular biology methods for measuring quantities of target nucleic acid sequences are well known in the art. These methods include but are not limited to end point PCR, competitive PCR, reverse transcriptase-PCR (RT-PCR), quantitative PCR (qPCR), reverse transcriptase qPCR (RT-qPCR), PCR-pyrosequencing, PCR-ELISA, DNA microarrays, gene expression panels (e.g. nanoString™), nucleic acid sequencing, such as next generation sequencing methods, in situ hybridization assays (such as dot-blot, Fluorescence In Situ Hybridization assay (FISH), RNA-ISH, automated quantitative RNA ISH (RNAscope®)), mass spectrometry, branched DNA (Nolte, Adv. Clin. Chem. 1998,33:201-235) and to multiplex versions of said methods (see for instance, Andoh et al., Current Pharmaceutical Design, 2009;15,2066-2073) and the next generation of any of the techniques listed and combinations thereof, all of which are within the scope of the present invention. Such methods may also include the pre-conversion of mRNA into cDNA by the reaction with a reverse transcriptase (RT), for example the PCR or qPCR reaction is usually preceded by conversion of mRNA into cDNA and referred to as RT-PCR or RT-qPCR, respectively.

Diverse next-generation sequencing methods have been described and are well known to a person skilled in the art. These include for instance sequencing by synthesis with cyclic reversible termination approaches (e.g., Illumina, SEQLL, Qiagen), sequencing by synthesis with single-nucleotide addition approaches (e.g., Roche-454, Thermo Fisher-Ion Torrent), sequencing by ligation (e.g., Thermo Fisher SOLiD and BGI-Complete Genomics), real-time long-read sequencing (e.g., Pacific Biosciences, Oxford Nanopore Technologies), synthetic long-read sequencing (e.g., Illumina, 10X Genomics, iGenomeX), see for instance Goodwin S, et al., Nat Rev Genet. 2016, 17(6):333-51).

In some embodiments, said molecular biology quantification methods are based on sequence specific amplification. Such an amplification based assay comprises an amplification step which comprises contacting a sample (preferably an isolated DNA sample) with two or more amplification oligonucleotides specific for a target sequence in a target nucleic acid to produce an amplified product if the target nucleic sequence is present in the sample. Suitable amplification methods include for example, replicase-mediated amplification, ligase chain reaction (LCR), strand-displacement amplification (SDA), transcription mediated amplification (TMA) and polymerase chain reaction (PCR), which includes quantitative PCR.

One particularly preferred quantification method is quantitative PCR (qPCR), also known as real-time PCR. It relates to a type of PCR that amplifies and simultaneously quantifies a target DNA molecule. Its key feature is that the amplified DNA is detected as the reaction progresses in real time. Unless otherwise provided, the term qPCR as used herein encompasses reverse transcriptase (RT)-qPCR. Different instruments are available, such as ABI Prism 7700 SDS, GeneAmp 5700 SDS, ABI Prism 7900 HT SDS from Applied Biosystems; iCycler iQ from Bio-Rad; Smart Cycler from Cepheid; Rotor-Gene from Corbett Research; LightCycler from Roche Molecular Biochemicals and Mx4000 Multiplex from Stratagene. The qPCR process enables accurate quantification of the PCR product in real-time by measuring PCR product accumulation very early in the exponential phase of the reaction, thus reducing bias in the quantification linked to the PCR amplification efficiency occurring in end-point PCR. Real-time PCR is well known in the art and is thus not described in detail herein. Technology overview and protocols for qPCR are available for instance from the above-mentioned vendors, e.g., http://www.sigmaaldrich.com/technical-documents/protocols/biology/sybr-green-qpcr.html or http://www.sigmaaldrich.com/life-science/molecular-biology/pcr/quantitative-pcr/qpcr-technical-guide.html. For a review of qPCR methods see Wong ML y Medrano JF, Biotechniques 2005, 39(1):75-85. In a particular embodiment, the quantification method is a multiplex qPCR.

Different detecting chemistries are available for qPCR. All of them can be used with the above-mentioned qPCR instruments. The term "detection chemistry" refers to a method to report amplification of specific PCR product in real-time PCR. These detecting chemistries may be classified into two main groups; the first group comprises double-stranded DNA intercalating molecules, such as SYBR Green I and EvaGreen, whereas the second includes fluorophore-labeled oligonucleotides. The latter, in turn, has been divided into three subgroups according to the type of fluorescent molecules used in the PCR reaction: (i) primer-probes (Scorpions, Amplifluor®, LUX™, Cyclicons, Angler®); (ii) probes; hydrolysis (TaqMan, MGB-TaqMan, Snake assay) and hybridization (Hybprobe or FRET, Molecular Beacons, HyBeacon™, MGB-Pleiades, MGB-Eclipse, ResonSense®, Yin-Yang or displacing); and (iii) analogues of nucleic acids (PNA, LNA®, ZNA™, non-natural bases: Plexor™ primer, Tiny-Molecular Beacon) see E. Navarro eta l.,Clinica Chimica Acta, Volume 439, 15 January 2015, Pages 231-250.

Said probes may be dual-labeled oligonucleotides, such as hydrolysis probes or molecular beacons. The 5' end of the oligonucleotide is typically labelled with a fluorescent reporter molecule while the 3' end is labeled with a quencher molecule. The sequence of the probe is specific for a region of interest in the amplified target molecule. In a more preferred embodiment, said probe is a hydrolysis probe which is designed so that the length of the sequence places the 5' fluorophore and the 3' quencher in close enough proximity so as to suppress fluorescence. Several reporter molecules and quenchers for use in qPCR probes are well known in the art.

Generally, for the quantification of nucleotide sequences specific oligonucleotides, such as probes and / or primers are used. The term "a primer and / or a probe" specifically includes "primers and / or probes". Both expressions are used interchangeably herein and encompass for example a primer; a probe; a primer and a probe; a pair of primers; and a pair of primers and a probe. Design and validation of primers and probes is well known in the art. For the design of primers and probes in quantitative real-time PCR methods, see for instance Rodriguez A et al. (Methods Mol Biol., 2015, 1275:31-56). Preferred primers and/or probes which may be used in the methods of the invention are described herein below under the kits of the invention.

Preferably, oligonucleotides useful in the methods of the invention are about 5 to about 50 nucleotides in length, about 10 to about 30 nucleotides in length, or about 20 to about 25 nucleotides in length. In certain embodiments, oligonucleotides specifically hybridizing with the target sequence are about 19 to about 21 nucleotides in length. In a particular embodiment, said oligonucleotides have been modified for detection or to enhance assay performance.

These oligonucleotides may be ribonucleotides or deoxyribonucleotides. In particular embodiments, the oligonucleotides may have at least one chemical modification. For instance, suitable oligonucleotides may be comprised of one or more "conformationally constrained" or bicyclic sugar nucleoside modifications, for example, "locked nucleic acids." "Locked nucleic acids" (LNAs) are modified ribonucleotides that contain an extra bridge between the 2' and 4' carbons of the ribose sugar moiety resulting in a "locked" conformation that confers enhanced thermal stability to oligonucleotides containing the LNAs. In other embodiments, the oligonucleotides may comprise peptide nucleic acids (PNAs), which contain a peptide-based backbone rather than a sugar-phosphate backbone. Other chemical modifications that the oligonucleotides may contain include, but are not limited to, sugar modifications, such as 2'-O-alkyl (*e.g.* 2'-O-methyl, 2'-O-methoxyethyl), 2'-fluoro, and 4' thio modifications, and backbone modifications, such as one or more phosphorothioate, morpholino, or phosphonocarboxylate linkages. For instance, these oligonucleotides, particularly those of shorter lengths (*e.g.,* less than 15 nucleotides) can comprise one or more affinity enhancing modifications, such as, but not limited to, LNAs, bicyclic nucleosides, phosphonoformates, 2' O-alkyl and the like. In some embodiments, the oligonucleotides may be chemically modified, for instance to improve their resistance to nuclease degradation (e.g., by end capping), to carry detection ligands (e.g., fluorescein) or to facilitate their capture onto a solid support (e.g., poly-deoxyadenosine "tails").

For mRNA determination, RNA isolated from frozen or fresh samples is extracted from the cells by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process. In a particular embodiment, the expression level is determined using mRNA obtained from a formalin-fixed, paraffin-embedded tissue sample. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinised and rehydrated. The sample is then lysed and RNA is extracted from the sample. Commercially available kits may be used for RNA extraction from paraffin samples, such as PureLink™ FFPE Total RNA Isolation Kit (Thermofisher Scientific Inc., US).

In another embodiment, the determination of the expression of the CRYAB, NNMT, and SPRY4 genes is carried out at protein level. Suitable methods for determining the levels of a given protein include, without limitation, those described herein below. Preferred methods for determining the protein expression levels in the methods of the present invention are immunoassays. Various types of immunoassays are known to one skilled in the art for the quantitation of proteins of interest. These methods are based on the use of affinity reagents, which may be any antibody or ligand specifically binding to the target protein or to a fragment thereof, wherein said affinity reagent is preferably labeled. Illustrative, but non-exclusive, examples of labels that can be used include radioactive isotopes, enzymes, fluorophores, chemoluminescent reagents, enzyme cofactors or substrates, enzyme inhibitors, particles, dyes, etc.

Affinity reagents may be any antibody or ligand specifically binding to the target protein or to a fragment thereof. Affinity ligands may include proteins, peptides, peptide aptamers, affimers and other target specific protein scaffolds, like antibody-mimetics.

Specific antibodies against the protein markers used in the methods of the invention may be produced for example by immunizing a host with a protein of the present invention or a fragment thereof. Likewise, peptides specific against the protein markers used in the methods of the invention may be produced by screening synthetic peptide libraries.

Western blot or immunoblotting techniques allow comparison of relative abundance of proteins separated by an electrophoretic gel (e.g., native proteins by 3-D structure or denatured proteins by the length of the polypeptide). Immunoblotting techniques use antibodies (or other specific ligands in related techniques) to identify target proteins among a number of unrelated protein species. They involve identification of protein target via antigen-antibody (or protein-ligand) specific reactions. Proteins are typically separated by electrophoresis and transferred onto a sheet of polymeric material (generally nitrocellulose, nylon, or polyvinylidene difluoride). Dot and slot blots are simplified procedures in which protein samples are not separated by electrophoresis but immobilized directly onto a membrane.

Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay. Multiplex and any next generation versions of any of the above, such as bead-based flow-cytometry immunoassays (e.g., based on the Luminex xMAP technology) are specifically encompassed. In a particular embodiment, said immunoassay is an ELISA assay or any multiplex version thereof.

Other methods that can be used for quantification of proteins in solution are techniques based on mass spectrometry (MS) such as liquid chromatography coupled to mass spectrometry (LC / MS), described for example in US2010/0173786, or tandem LC-MS / MS (WO2012/155019, US2011/0039287, M. Rauh, J Chromatogr B Analyt Technol Biomed Life Sci 2012 February 1, 883-884. 59-67) and multiplex versions of the above techniques, as well as the next generation of such techniques and combinations thereof.

For determining protein expression and location, immunohistochemical and in-situ hybridization analysis are usually preferred.

Immunohistochemistry (IHC) analysis is typically conducted using thin sections of the biological sample immobilized on coated slides. These sections, when derived from paraffin-embedded tissue samples, are deparaffinised and preferably treated so as to retrieve the antigen. The detection can be carried out in individual samples or in tissue microarrays. This procedure, although is subjectively determined by the pathologist, is the standard method of measurement of IHC results, and well known in the art.

Generally, the use of this technique entails the determination of the Histological score value. For instance, the Histological score (H-Score) value may be determined per biological sample according to (i) staining intensity and (ii) the percentage of positive staining tumor cells by using the following formula:

$$\text{H-Score} = \Sigma \text{ (intensity grade x \% stained cells)}$$

The staining intensity of tumor cells may be scored in different intensity grades, for example the following 4 grades:

1. no staining or very weak staining (intensity = 0);
2. positive weak staining (intensity= 1 or +);

3. positive moderate staining (intensity=, 2 or ++); and
4. positive strong staining (intensity=3 or +++).

The percentage of positive staining cells for each intensity grade may be scored from 0 to 100.
The final score, called "H-Score", is preferably calculated by adding the products of the percentage cells stained with a given intensity grade (0-100) by the corresponding staining intensity grade value (0-3). The following formula may be applied:

H-Score = Σ intensity grade x % stained cells = 1 x (% of cells with weak staining) + 2 x (% of cells with moderate staining) + 3 x (% of cells with strong staining)

The assessment of the staining intensity and the percentage of positive staining tumor cells can be determined by any means known to the skilled person including but not limited to a panel of at least two independent pathologists with no knowledge about clinical data scoring all immunohistochemical stainings. In case, the panel of pathologist were to disagree in the scores it is convenient to expand the panel of independent pathologists to at least 3, 4, or 5.
Once the staining intensity and the percentage of positive staining tumor cells have been determined, the value of the H-Score may be obtained by applying the above formula. The resulting value of the H-Score determines the level of expression of the protein marker.
The expression "determining the expression levels" as used herein, refers to ascertaining the absolute or relative amount or concentration of the biomarker in the sample. Techniques to assay levels of individual biomarkers from test samples are well known to the skilled technician, and the invention is not limited by the means by which the components are assessed.
Expression levels may be absolute or relative. When the expression levels are normalized, normalization can be performed with respect to different measures in the sample. These procedures are well known to one skilled in the art. Typically, expression levels are normalized with respect to an "endogenous control". An "endogenous control" as used herein may relate to a gene expression product whose expression levels do not change or change only in limited amounts in tumor cells with respect to non-tumorigenic cells.
"Endogenous control", also referred herein as "control gene" or "normalizing gene" is usually the expression product from a housekeeping gene and which codes for a protein which is constitutively expressed and carries out essential cellular functions. Housekeeping genes that can be used as endogenous control include for example β-2-microglobulin, ubiquitin, 18-S ribosomal protein, cyclophilin, GAPDH, actin and HPRT. In a particular embodiment, "endogenous controls" used in the present invention are selected from the group consisting of 18S, ACTB, B2M, GAPDH, GUSB2, HPRT, PPIA, RPLP, TBP, TUBB, UBC, and YWHAZ.
Also, the number of control genes which can be used for normalization purposes is not particularly limited. For illustrative, but not limiting, purposes, this may be conducted with one, two, three, four, five, six, seven eight, nine, ten, eleven, twelve, fifteen, twenty, or as many genes as desired. For instance, when next generation sequencing methods are used to quantify transcript expression, normalization may be conducted against the whole genome.
**Step (b)** of the methods of the invention comprises calculating a score. The score is a value obtained according to a given mathematical algorithm wherein the expression values of each of the gene markers used in the methods of the invention are variables of said mathematical algorithm. Preferably, said score is calculated as the sum of the product between the gene expression values and their estimated regression coefficients obtained in a regression analysis. In a particular embodiment, said score calculated in step b) is obtained by the following formula:

MOMTGFB score = a * CRYAB + b* NNMT + c * SPRY4;

wherein the terms "CRYAB", "NNMT" and "SPRY4" correspond to the expression values of the respective gene products.
According to this formula, said coefficients (a, b and c) are positive for markers CRYAB, NNMT, and SPRY4 and the score is proportional to the expression levels of CRYAB, NNMT, and SPRY4; wherein the higher the score, the higher the TGFβ activation of the TME. The value of the coefficients (a, b and c) is not particularly limited, it may range for instance from 0.0001 to 1000, preferably from 0.001 to 100, more preferably from 0.01 to 10 or even more preferably from 0.1 to 1, such as 0.15, 0.20, 0.25, 0.30, 0.35, 0.40, 0.45, 0.50, 0.55, 0.60, 0.65, 0.70, 0.75, 0.80, 0,85, 0.90 or 0.95. In a particular embodiment, all these coefficients equal 1; this is equivalent to not using any coefficient and simply relying in the levels of each gene expression product in the calculation of the score. In another particular embodiment, the coefficient of CRYAB is from 0.20 to 0.40, the coefficient for NNMT is from 0.26 to 0.46 and the coefficient of SPRY4 is from 0.09 to 0.29. In preferred embodiments, the coefficient of CRYAB is from 0.25 to 0.35, the coefficient for NNMT is from 0.31 to 0.41 and the coefficient of SPRY4 is from 0.14 to 0.24.

In a further preferred embodiment, the score is obtained by using the following formula:

$$\text{MOMTGFB score} = 0.30 * \text{CRYAB} + 0.36 * \text{NNMT} + 0.19 * \text{SPRY4}.$$

In preferred embodiments, the gene product expression values have been normalized with respect to the expression values of control gene expression products.

Furthermore, **step (c)** of the methods of the invention comprises classifying/selecting the cancer patient as having a TGFβ activated microenvironment based on the score obtained in b).

Typically, in step c) said method comprises comparing the score in the subject sample with a reference value; and an increase of the score in the subject sample with regard to said reference value is indicative of a TGFβ activated microenvironment; whereas a decrease of the score in the subject sample with regard to said reference value is indicative of a tumor which fails to have a TGFβ activated microenvironment.

As described above, the method of the invention enables to identify non-cell-specific TGFβ activation of the microenvironment. In preferred embodiments, a TGFβ activated microenvironment is defined as a tumor microenvironment comprising TGFβ activated fibroblasts, T-cells and macrophages.

The term "reference value", as used herein, relates to a predetermined criteria used as a reference for evaluating the values or data obtained from the samples collected from a subject. This "reference value" may also be referred as "cut-off value" or "threshold value".

The reference value can be an absolute value, a relative value, a value that has an upper or a lower limit, a range of values, an average value, a median value, a mean value, a z-score (e.g. mean value + or - 1 standard deviation (SD)), a tertile value, or a value as compared to a particular control or baseline value. In a particular embodiment, optionally in combination with one or more of the embodiments or features described above or below, said reference value is the mean value or the tertile value.

In addition, it is further noted that a variety of statistical and mathematical methods for establishing the threshold or cut-off level of expression are known in the prior art. A threshold or cut-off expression level for a particular biomarker may be selected, for example, based on data from Receiver Operating Characteristic (ROC) plots. One of skill in the art will appreciate that these threshold or cut-off expression levels can be varied, for example, by moving along the ROC plot for a particular biomarker or combinations thereof, to obtain different values for sensitivity or specificity thereby affecting overall assay performance.

Sensitivity, specificity, and/or accuracy are parameters typically used to describe the validity or performance of a test. In particular, they are used to quantify how good and reliable the discrimination method is. A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

In preferred embodiments, the methods of the invention have sensitivity, specificity and/or accuracy values of at least about 60 %, preferably of at least about 70 %, and can be, for example, at least 75 %, at least 80 %, at least 85 %, at least 90 %, at least 95 %, at least 96 %, at least 97 %, at least 98 %, at least 99 % or 100% in at least 60 % of the group or population assayed, or preferably in at least 65 %, 70 %, 75 %, 80 %, 85 %, 90 %, 95 % or 100 % of the group or population assayed.

A reference value can be based on an individual sample value but is generally based on a large number of samples, including or excluding the sample to be tested. For instance, this reference value may be derived from a collection of tumor tissue samples from a reference cancer patients' population for whom historical information relating to the actual clinical outcome for the corresponding cancer patient is available. Said reference cancer patient's population may for instance be from subjects suffering from one or more of the cancer types referred herein, including particular subgroups therefrom (e.g. patients belonging to a particular tumor-node-metastasis (TNM) stage(s)).

In the methods of the invention, the score obtained in step b) is considered "decreased" when said score is lower than a reference value. Preferably, the score is considered to be lower than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference value.

Likewise, in the context of the methods of the invention, the score obtained in step b) is considered "increased" when said score is higher than a reference value. Preferably, the score is considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Alternatively or in addition, subjects having more than about 1.1,1.2, 1.3, 1.4, 1.5, 2, 3, 4, 5, 6, 7, 8, 9, 10, 15 or 20 fold levels deviation (i.e., increase or decrease) than the reference value as described herein.

The method of the invention, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical significance measures obtained by statistical tests; illustrative, non-limiting examples of said statistical significance measures include determining confidence intervals, determining the p-value, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

It is further noted that the accuracy of the method of the invention can be further increased by additionally considering other gene markers, biochemical parameters and/or clinical characteristics of the patients (e.g. age, sex, tobacco and/or other risk factors). Determination of these other markers, parameters and/or characteristics (including the characterization of the tumor subtype according to these) can be sequential or simultaneous to any or all of steps a) to c) as described herein above.

In some embodiments, the methods of the invention may be applied or common to several cancer types, including, but not limited to, carcinoma, lymphoma, blastoma, sarcoma, and leukemia. More particular examples of such cancers include breast cancer, squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, colon cancer, colorectal cancer, endometrial or uterine carcinoma, salivary gland carcinoma, kidney cancer, liver cancer, prostate cancer, renal cancer, vulval cancer, thyroid cancer, hepatic carcinoma, gastric cancer, melanoma, and various types of head and neck cancer.

In a particular embodiment, said method is not tissue-specific. In related embodiments, said method is useful in the determination of a TGFβ-activated microenvironment in at least two, preferably three, four, five, six or seven different cancer types. Preferably, this cancer is selected from the group consisting of colorectal cancer, stomach adenocarcinoma, bladder cancer, pancreas carcinoma, prostate cancer, lung cancer, breast cancer, kidney cancer, liver cancer, and melanoma. In other embodiments, the cancer is colorectal cancer.

In some embodiments of the methods of the invention the subject has a solid tumor. This solid may be previously treated or untreated. In preferred embodiments, the subject has a previously untreated solid tumor.

Tumors can be further stratified according to its stage of development. Nowadays, the tumor-node-metastasis (TNM) staging system is the standard method used for treatment selection and clinically predicting survival of patients with cancer. The TNM staging system was developed and is maintained by the American Joint Committee on Cancer (AJCC) and the Union for International Cancer Control (UICC). It was developed as a tool for doctors to stage different types of cancer based on certain, standardized criteria (www.cancerstaging.org). In particular, the TNM staging system is based on the extent of the tumor (T), the extent of spread to the lymph nodes (N), and the presence of metastasis (M).

The T category describes the original (primary) tumor.

| | |
|---|---|
| **TX** | Primary tumor cannot be evaluated |
| **T0** | No evidence of primary tumor |
| **Tis** | Carcinoma in situ (early cancer that has not spread to neighboring tissue) |
| **T1-T4** | Size and/or extent of the primary tumor |

The **N category** describes whether or not the cancer has reached nearby lymph nodes

| | |
|---|---|
| **NX** | Regional lymph nodes cannot be evaluated |
| **N0** | No regional lymph node involvement (no cancer found in the lymph nodes) |
| **N1-N3** | Involvement of regional lymph nodes (number and/or extent of spread) |

The **M category** tells whether there are distant metastases (spread of cancer to other parts of the body).

| | |
|---|---|
| **M0** | No distant metastasis (cancer has not spread to other parts of the body) |
| **M1** | Distant metastasis (cancer has spread to distant parts of the body) |

Because each cancer type has its own classification system, letters and numbers do not always mean the same thing for every kind of cancer. Once the T, N, and M are determined, they are combined, and an overall *stage* of 0, I, II, III, IV is assigned. Sometimes these stages are subdivided as well, using letters such as IIIA and IIIB.

In some cancer types, non-anatomic factors are required for assigning the anatomic stage/prognostic group. These are clearly defined in each chapter of the AJCC Cancer Staging Manual (e.g. Gleason Score in Prostate). These factors are collected separately from T, N, and M, which remain purely anatomic and are used to assign stage groups.

Where non-anatomic factors are used in groupings, there is a definition of the groupings provided for cases where the non-anatomic factor is not available (X) or where it is desired to assign a group ignoring the non-anatomic factor.

Stage I cancers are the least advanced and often have a better prognosis. Higher stage cancers are often more advanced but, in many cases, can still be treated successfully.

For instance, for CRC the following stage classification may be used:

| AJCC stage | TNM stage | TNM stage criteria for colorectal cancer |
|---|---|---|
| Stage 0 | Tis N0 M0 | Tis: Tumor confined to mucosa; cancer- in situ |
| Stage I | T1 N0 M0 | T1l: Tumor invades submucosa |
| Stage I | T2 N0 M0 | T2: Tumor invades muscularis propria |
| Stage II-A | T3 N0 M0 | T3: Tumor invades subserosa or beyond (without other organs involved) |
| Stage II-B | T4 N0 M0 | T4: Tumor invades adjacent organs or perforates the visceral peritoneum |
| Stage III-A | T1-2 N1 M0 | N1: Metastasis to 1 to 3 regional lymph nodes. T1 or T2. |
| Stage III-B | T3-4 N1 M0 | N1: Metastasis to 1 to 3 regional lymph nodes. T3 or T4. |
| Stage III-C | any T, N2 M0 | N2: Metastasis to 4 or more regional lymph nodes. Any T. |
| Stage IV | any T, any N,M1 | M1: Distant metastases present. Any T, any N. |

With the continuous flow of new data and the increasing knowledge of the disease, the staging system requires continuous adjustment. The eight edition of the TNM classification of malignant tumors (James D. Brierley, Mary K. Gospodarowicz, Christian Wittekind, December 2016) provides the latest, internationally agreed-upon standards to describe and categorize cancer stages and progression.

In a particular embodiment, optionally in combination with any of the embodiments or features described herein, the methods of the invention comprise further to step

a):
A1) determining in said sample the stage according to the TNM classification of tumors; and

b) calculating a score from the expression levels of the markers determined in the biological sample as defined in step a) and the stage of TNM classification as defined in step A1); and

c) determining whether a patient's tumor has a TGFβ-activated microenvironment according to the score obtained in b).

In other embodiments, optionally in combination with any of the embodiments or features described herein, the methods of the invention do not comprise determining the stage according to the TNM classification of tumors as defined in stage A1) above.

Alternatively, or in addition, other markers may be used for tumor stratification. It is currently accepted that tumors (e.g. CRC, gastric cancer, etc.) can be classified according to their global genomic status into two main types: *microsatellite instable tumors* (MSI) and *microsatellite stable* (MSS) tumors (also known as tumors with chromosomal instability), see Umar, A. et al. J. Natl. Cancer Inst. (2004) 96, 261-268; Ratti, M et al., Cell Mol Life Sci. (2018) 75(22): 4151-4162. This taxonomy may play a significant role in determining pathologic, clinical and biological characteristics of tumors: MSS tumors are characterized by changes in chromosomal copy number and show worse prognosis, on the contrary the less common MSI tumors (about 15% in colon tumors) are characterized by the accumulation of a high number of mutations and show predominance in females, proximal colonic localization, poor differentiation, tumor-infiltrating lymphocytes and better prognosis. In addition, these subtypes have been described to exhibit different responses to chemotherapeutic agents, and it is also well established that they arise from a distinctive molecular mechanism.

Whereas MSS tumors generally follow the classical adenoma-to-carcinoma progression described by Vogelstein and Fearon (Cell 1 (1990) 61(5):759-767), MSI tumors result from the inactivation of DNA mismatch repair genes like MLH-1. In clinical practice, MSI status is generally determined by immunohistochemistry (IHC) methods that detect protein MLH1, MSH2, MSH6 and/or PMS2 (Manavis J., et al. Appl Immunohistochem Mol Morphol. 2003 Mar;11(1):73-7). Nevertheless, other techniques such as molecular biology techniques to evaluate microsatellites or even newly developed Next generation sequencing methods can also be used (Nowak J.A., et al. J Mol Diagn. (2017) 19(1): 84-91). Bethesda guidelines are typically followed to determine MSI/MSH status (Umar, A. et al. J. Natl. Cancer Inst. (2004) 96, 261-268).

The biomarker signature described herein was shown by the inventors to enable identifying tumors presenting a TGFβ-activated microenvironment independently of microsatellite stability status, that is, both in microsatellite stable (MSS) and microsatellite instable (MSI) phenotypes (Example 3.1 and **Figures 6** and **8**).

Accordingly, in a particular embodiment, the method of the invention is characterized by identifying a TGFβ-activated microenvironment in any of microsatellite instable (MSI) or microsatellite stable (MSS) tumors.

MSI-high (MSI-H) status has been associated with a better prognosis in early-stage CRC, and has emerged as a predictor of sensitivity to immunotherapy treatments with checkpoint inhibitors (Battaglin et al., Advances in Hematology and Oncology 2018, 16(11), 735-747). Nevertheless, as above-mentioned, immune checkpoint inhibitors were found by Tauriello et al. (Nature, Vol. 554 (2018) 539 - 543) to lack efficacy in CRC tumors with a TGFβ activated microenvironment. Thus, the signature of the invention enables to identify from patients having a MSI tumor those which would likely not benefit from an immune check point inhibitor treatment alone and select the same for a combination treatment with an inhibitor of the TGFβ signaling pathway.

In some embodiments, optionally in combination with any of the embodiments or features described herein, the methods of the invention do not comprise determining the tumor MSI/MSS status.

In other embodiments, optionally in combination with any of the embodiments or features described herein, the methods of the invention comprise further to step

a): A2) determining in said sample the tumor MSI/MSS status; and

b) calculating a score from the expression levels of the markers determined in the biological sample as defined in step a) and the tumor MSI/MSS status as defined in step A2), optionally the stage of TNM classification as defined in step A1); and

c) determining whether a patient’s tumor has a TGFβ-activated microenvironment according to the score obtained in b).

A further classification system which has been described for tumor subtype determination and may be used in the methods of the invention is the consensus molecular subtype (CMS) classification system described by Guinney et al. (Nat Med. 2015, 21(11), 1350-6). The CMS classification comprises 4 CMSs with the following characterizing features:

- **CMS1** (microsatellite instability immune, 14%), hypermutated, microsatellite unstable and strong immune activation;
- **CMS2** (canonical, 37%), epithelial, marked WNT and MYC signaling activation;
- **CMS3** (metabolic, 13%), epithelial and evident metabolic dysregulation; and
- **CMS4** (mesenchymal, 23%), prominent transforming growth factor-β activation, stromal invasion and angiogenesis.

Interestingly, a high MOMTGFB score was shown to identify 96.1% of CRC tumors classified under CMS4 (associated with prominent TGFβ activation) according to the consensus molecular subtypes (CMSs) classification. It thus provides a valuable tool for clinicians to identify tumors presenting CMS4 characteristics with a much simpler test (only requires determining the expression levels of three MOMSTGFB genes).

In addition, the method of the invention was also able to capture tumors with a TGFβ activated TME classified under other CMS groups (Example 2.1), which would otherwise likely not had been selected for treatment with an inhibitor of the TGFβ signaling pathway.

Accordingly, the method of the invention is capable of identifying a tumor with a TGFβ-activated microenvironment in any of CMS1, CMS2, CMS3 or CMS4 molecular subtype tumors, preferably in any of CMS1, CMS3 or CMS4. Thus, in some embodiments, optionally in combination with any of the embodiments or features described herein, the methods of the invention do not comprise determining the tumor CMS subtype.

In other embodiments, optionally in combination with any of the embodiments or features described herein, the methods of the invention comprise further to step

a):
A3) determining in said sample the tumor CMS subtype; and

b) calculating a score from the expression levels of the markers determined in the biological sample as defined in step a) and the tumor CMS subtype as defined in A3), optionally the stage of TNM classification as defined in step A1) and/or the tumor MSI/MSS status defined in step A2); and

c) determining whether a patient's tumor has a TGFβ-activated microenvironment according to the score obtained in b).

**Predictive methods of the invention**

In a second aspect, the invention also pertains to a method for predicting the efficacy of (or the likelihood of response to) a treatment with an inhibitor of the TGFβ signaling pathway in a cancer patient, wherein said method comprises:

i. determining whether a patient's tumor has a TGFβ-activated microenvironment according to a method as described herein; and

ii. predicting a higher efficacy or likelihood of response to a treatment with an inhibitor of the TGFβ signaling pathway when said patient's tumor has a TGFβ-activated microenvironment.

In a third aspect, the present invention also relates to a method for selecting a cancer patient which is likely to benefit from a treatment with an inhibitor of the TGFβ signaling pathway, wherein said method comprises:

i. determining whether a patient's tumor has a TGFβ-activated microenvironment according to a method as described herein; and

ii. selecting said cancer patient for the treatment with an inhibitor of the TGFβ signaling pathway when said patient's tumor has a TGFβ-activated microenvironment.

This treatment with an inhibitor of the TGFβ signaling pathway may be a neoadjuvant treatment administered prior to the surgical removal of the tumor and/or an adjuvant treatment after the surgical intervention. Preferably, said treatment is an adjuvant treatment. A treatment with an inhibitor of the TGFβ signaling pathway may be as described herein below.

The methods of the present invention or any of the steps thereof might be implemented by a computer. In a particular embodiment, optionally in combination with any of the features or embodiments as described herein, the score of step b) is calculated using a computer; and/or the selection, classification and/or determination of step c) is conducted using a computer.

Therefore, a further aspect of the invention refers to a computer implemented method, wherein the method is any of the methods disclosed herein or any combination thereof.

It is noted that any computer program capable of implementing any of the methods of the present invention or used to implement any of these methods or any combination thereof, also forms part of the present invention.

This computer program is typically directly loadable into the internal memory of a digital computer, comprising software code portions for performing the steps of comparing the score (e.g., obtained from the level of one or more of the target markers as described in the invention), from the one or more biological samples of a subject with a reference value and determining the prognosis of said subject or whether it would benefit from adjuvant therapy, when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. The present invention further relates to a computer-readable storage medium having stored thereon a computer program of the invention or the results of any of the methods of the invention.

As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

**Methods for treatment, related medical uses and compositions**

In a fourth aspect, the invention pertains to an inhibitor of the TGFβ signaling pathway for use in a method for treatment of a cancer patient, wherein said patient's tumor has been determined to have a TGFβ-activated microenvironment according to a method as described herein.

In an alternative aspect, the invention provides the use of an inhibitor of the TGFβ signaling pathway in the manufacturing of a medicament for the treatment of a cancer patient, wherein said cancer patient has been selected as having a tumor with a TGFβ-activated microenvironment by a method as described herein.

In a further alternative aspect, the invention relates to a method of treating a cancer patient by administering a therapeutically effective amount of an inhibitor of the TGFβ signaling pathway, wherein said patient has been selected as having a tumor with a TGFβ-activated microenvironment by a method as described herein.

In a related aspect, the present invention further provides any of the methods as described herein above, which further comprises the step of administering to said patient a therapeutically effective amount of a TGFβ signaling pathway inhibitor.

Said TGFβ signaling pathway inhibitor may be administered as single agent or in combination with another therapy or drug. In a particular embodiment of any of these aspects, the inhibitor of the TGFβ signaling pathway is administered to said patient in combination (i.e., as a combination treatment) with another anti-cancer treatment, preferably with another anti-cancer agent or radiotherapy.

The term anti-cancer agent has been defined herein above and may include but is not limited to chemotherapeutic agents, growth inhibitory agents, cytotoxic agents, anti-hormonal agents, agents used in radiation therapy, anti-angiogenesis agents, apoptotic agents, anti-tubulin agents, etc. and any combinations thereof. Said anti-cancer agent may be administered prior, concomitantly or after the TGFβ inhibitor administration. The two drugs may form part of the same composition or be provided as a separate composition for administration at the same time or at a different time.

Preferably, said TGFβ signaling pathway inhibitor is administered to said patient in combination with a cytotoxic agent or immune checkpoint inhibitor.

In a fifth aspect, the present invention relates to an anti-cancer agent other than a TGFβ signaling pathway inhibitor (e.g., cytotoxic agent or immune checkpoint inhibitor) or radiotherapy for use in a method for treatment of a cancer patient in combination with a TGFβ signaling pathway inhibitor, wherein said patient's tumor has a TGFβ-activated microenvironment according to a method as described herein.

In an alternative aspect, the invention provides the use of an anti-cancer agent other than a TGFβ signaling pathway inhibitor (e.g., cytotoxic agent or immune checkpoint inhibitor) or radiotherapy in combination with a TGFβ signaling pathway inhibitor in the manufacturing of a medicament for the treatment of a cancer patient, wherein said cancer patient has been selected by a method as described herein as having a tumor with a TGFβ-activated microenvironment.

In a further alternative aspect, the invention relates to a method of treating a cancer patient by administering a therapeutically effective amount of an anti-cancer agent other than a TGFβ signaling pathway inhibitor (e.g., cytotoxic agent or immune checkpoint inhibitor) or radiotherapy in combination with a therapeutically effective amount of a TGFβ signaling pathway inhibitor, wherein said patient has been selected by a method as described herein as having a tumor with a TGFβ-activated microenvironment.

In preferred embodiments of any thereof, said other anti-cancer agent is a cytotoxic agent or immune checkpoint inhibitor.

Immune checkpoints regulate T cell function in the immune system. T cells play a central role in cell-mediated immunity. Checkpoint proteins interact with specific ligands which send a signal into the T cell and essentially switch off or inhibit T cell function. Cancer cells take advantage of this system by driving high levels of expression of checkpoint proteins on their surface which results in control of the T cells expressing checkpoint proteins on the surface of T cells that enter the tumor microenvironment, thus suppressing the anti-cancer immune response. As such, inhibition of checkpoint proteins would result in restoration of T cell function and an immune response to the cancer cells. Examples of checkpoint proteins include, but are not limited to CTLA-4, PDL1, PDL2, PD1, B7-H3, B7- H4, BTLA, HVEM, TIM3, GAL9, LAG3, VISTA, KIR, 2B4 (belongs to the CD2 family of molecules and is expressed on all NK, γδ, and memory $CD8^+$ (αβ) T cells), CD 160 (also referred to as BY55), CGEN-15049, CHK 1 and CHK2 kinases, A2aR and various B-7 family ligands.

Programmed cell death protein 1 (PD-1) is a 288 amino acid cell surface protein molecule is expressed on T cells and pro-B cells and plays a role in their fate/ differentiation. PD-1 has two ligands, PD-L1 and PD-L2, which are members of the B7 family. PD-L1 protein is upregulated on macrophages and dendritic cells (DC) in response to LPS and GM- CSF treatment, and on T cells and B cells upon TCR and B cell receptor signaling, whereas in resting mice, PD-L1 mRNA can be detected in the heart, lung, thymus, spleen, and kidney. PD-1 negatively regulates T cell responses.

PD-1 has been shown to play a role in tumor-specific escape from immune surveillance. It has been demonstrated that PD-1 is highly expressed in tumor-specific cytotoxic T lymphocytes (CTLs) in both chronic myelogenous leukemia (CML) and acute myelogenous leukemia (AML). PD-1 is also up-regulated in melanoma infiltrating T lymphocytes (TILs) (Dotti, Blood (2009) 114 (8): 1457-58). Tumors have been found to express the PD-1 ligand (PDL-1 and PDL-2) which, when combined with the up-regulation of PD-1 in CTLs, may be a contributory factor in the loss in T cell functionality and the inability of CTLs to mediate an effective anti-tumor response. Researchers have shown that in mice chronically infected with lymphocytic choriomeningitis virus (LCMV), administration of anti-PD-1 antibodies blocked PD-1-PDL interaction and was able to restore some T cell functionality (proliferation and cytokine secretion), and lead to a decrease in viral load (Barber et al (2006) Nature 439 (9): 682-687).

Accordingly, in a particular embodiment, optionally in combination with one or more of the embodiments of features described herein, said method comprises administering to the cancer patient an inhibitor of the TGFβ signaling pathway in combination with an agent that is an immune checkpoint inhibitor. This checkpoint inhibitor may be a biologic therapeutic or a small molecule. Preferably, the checkpoint inhibitor is an antibody. In preferred embodiments, the checkpoint inhibitor inhibits a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, B-7 family ligands or a combination thereof. In a particular embodiment, this immune-check point inhibitor is an anti-PD1/PDL1 inhibitor, including combinations of any thereof. Illustrative, non-limiting examples of anti-PD1/PDL1 inhibitors are the following PD-1 inhibitors currently being tested in clinical trials: CT-011 is a humanized IgG1 monoclonal antibody against PD-1. A phase II clinical trial in subjects with diffuse large B-cell lymphoma (DLBCL) who have undergone autologous stem cell transplantation was recently completed. Preliminary results demonstrated that 70% of subjects were progression-free at the end of the follow-up period, compared with 47% in the control group, and 82% of subjects were alive, compared with 62% in the control group. This trial determined that CT-011 not only blocks PD-1 function, but it also augments the activity of natural killer cells, thus intensifying the antitumor immune response.

BMS 936558 is a fully human IgG4 monoclonal antibody targeting PD-1 agents under a phase I trial, biweekly administration of BMS-936558 in subjects with advanced, treatment-refractory malignancies showed durable partial or complete regressions. The most significant response rate was observed in subjects with melanoma (28%) and renal cell carcinoma (27%), but substantial clinical activity was also observed in subjects with non-small cell lung cancer (NSCLC), and some responses persisted for more than a year. It was also relatively well tolerated; grade >3 adverse events occurred in 14% of subjects.

BMS 936559 is a fully human IgG4 monoclonal antibody that targets the PD-1 ligand PD-L1. Phase I results showed that biweekly administration of this drug led to durable responses, especially in subjects with melanoma. Objective response rates ranged from 6% to 17%) depending on the cancer type in subjects with advanced-stage NSCLC, melanoma, RCC, or ovarian cancer, with some subjects experiencing responses lasting a year or longer.

MK 3475 is a humanized IgG4 anti-PD-1 monoclonal antibody in phase I development in a five-part study evaluating the dosing, safety, and tolerability of the drug in subjects with progressive, locally advanced, or metastatic carcinoma, melanoma, or NSCLC.

MPDL 3280A is a monoclonal antibody, which also targets PD-L1, undergoing phase I testing in combination with the BRAF inhibitor vemurafenib in subjects with BRAF V600-mutant metastatic melanoma and in combination with bevacizumab, which targets vascular endothelial growth factor receptor (VEGFR), with or without chemotherapy in subjects with advanced solid tumors.

AMP 224 is a fusion protein of the extracellular domain of the second PD-1 ligand, PD-L2, and IgGI, which has the potential to block the PD-L2/PD-1 interaction. AMP-224 is currently undergoing phase I testing as monotherapy in subjects with advanced cancer.

Medi 4736 is an anti-PD-L1 antibody in phase I clinical testing in subjects with advanced malignant melanoma, renal cell carcinoma, NSCLC, and colorectal cancer.

CTLA4 (cytotoxic T-lymphocyte-associated protein), is a protein receptor that down regulates the immune system. CTLA4 is found on the surface of T cells, which lead the cellular immune attack on antigens. The T cell attack can be turned on by stimulating the CD28 receptor on the T cell. The T cell attack can be turned off by stimulating the CTLA4 receptor. In another particular embodiment, this immune-check point inhibitor is an anti-CTLA4 inhibitor, such as ipilimumab (Yervoy™), or any combination thereof.

The inhibitor of the TGFβ signaling pathway alone or in combination with an anti-cancer agent may be formulated as a pharmaceutical composition, wherein said pharmaceutical composition further comprises a pharmaceutically acceptable excipient, vehicle or carrier. Said pharmaceutical composition may be a "dosage form" devised to enable administration of the drug medication in the prescribed dosage amounts. Depending on the method/route of administration different dosage forms will be used.

Still in a sixth aspect, the present invention relates to an anti-cancer agent (e.g., cytotoxic agent or immune checkpoint inhibitor) or radiotherapy for use in a method for treatment of a cancer patient selected by a method as described herein as failing to have a tumor with a TGFβ-activated microenvironment, wherein said anti-cancer agent or radiotherapy is not administered in combination with a TGFβ signaling pathway inhibitor.

In an alternative aspect, the invention provides the use of an anti-cancer agent (e.g., cytotoxic agent or immune checkpoint inhibitor) or radiotherapy in the manufacturing of a medicament for the treatment of a cancer patient selected by a method as described herein as failing to have a tumor with a TGFβ-activated microenvironment, wherein said anti-cancer agent or radiotherapy is not administered in combination with a TGFβ signaling pathway inhibitor.

In a further alternative aspect, the invention relates to a method of treating a cancer patient selected by a method as described herein as failing to have a tumor with a TGFβ-activated microenvironment, by administering a therapeutically effective amount of an anti-cancer agent (e.g., cytotoxic agent or immune checkpoint inhibitor) or radiotherapy, wherein said anti-cancer agent or radiotherapy is not administered in combination with an inhibitor of the TGFβ signaling pathway.

## Additional methods of the invention

In a seventh aspect, the invention relates to a method for determining the prognosis of a cancer patient, wherein said method comprises:

i. determining whether a patient's tumor has a TGFβ-activated microenvironment according to a method as described herein; and
ii. classifying the patient as having poor prognosis when said patient's tumor has a TGFβ-activated microenvironment.

In a related aspect, it refers to a method for determining the prognosis of a cancer patient, wherein said method comprises:

a) determining the expression levels of a group of genes comprising CRYAB, NNMT, and SPRY4 in a tumor sample isolated from said patient;
b) calculating a score from the expression levels of the genes determined in step a); and
c) comparing the score in the patient's sample with a reference value; wherein an increase of the score in the patient's sample with regard to said reference value is indicative of poor prognosis.

Poor prognosis in CRC has been associated to the presence of a TGFβ-activated stroma (Calon et al., Cancer cell 22 (2012) 571-584; Calon et al., Nature genetics 47:4 (2015) 320-332). As shown in **Figures 5A & 5B,** from those cancer patients having tumors classified in CMS1, CMS2 or CMS3 subgroups the high MOMTGFB population is the one presenting tumors with TGFβ-activated stroma and a worse prognosis.

In preferred embodiments, the method of the invention is used to determine those patients with poor prognosis/outcome which have been classified under CMS1, CMS2 or CMS3 subgroups when using the consensus molecular classification (Guinney et al. 2015). Similarly, MOMSTGFB score enables to identify patients with poor prognosis independently from their MSS/MSI status (**Fig. 6**). In preferred embodiments, the method of the invention is used to determine those patients with poor prognosis/outcome which have been classified as MSI (typically associated with better prognosis) further to microsatellite stability determination.

Disease progression or outcome may be measured using different parameters, including but not limited to, tumor growth, tumor growth delay, increase/decrease of tumor size, increase/decrease in tumor markers, and patient's survival.

Preferably, in the context of the present invention, the clinical outcome of a subject, is expressed as overall survival and/or disease-free survival. Survival of cancer patients is generally suitably expressed by Kaplan-Meier curves, named after Edward L. Kaplan and Paul Meier who first described it (Kaplan, Meier: Amer. Statist. Assn. 53:457-481). The Kaplan-Meier estimator is also known as the product limit estimator. It serves for estimating the survival function from life-time data. A plot of the Kaplan-Meier estimate of the survival function is a series of horizontal steps of declining magnitude which, when a large enough sample is taken, approaches the true survival function for that population. The value of the survival function between successive distinct sampled observations is assumed to be constant. With respect to the present invention, the Kaplan-Meier estimator may be used to measure the fraction of patients living for a certain amount of time after beginning a therapy (e.g. after tumor resection). The clinical outcome predicted may be the (overall/disease-free) survival in months/years from the time point of taking the sample. It may be survival for a certain period from taking the sample, such as of six months or more, one year or more, two years or more, three years or more, four years or more, five years or more, six years or more. In each case, "survival" may refer to "overall survival" or "disease free survival".

The term "disease free survival", or "DFS" as used herein, is defined as the interval of time from start of treatment (e.g., date of surgery) to the first measurement of cancer growth. The term "overall survival" or "OS" as used herein, is defined as the interval of time from the start of treatment (e.g., date of surgery) to death from any cause.

The term "poor prognosis" as used herein may refer to a high risk of recurrence, relapse, metastasis and/or death. In preferred embodiments, the term "poor prognosis" means a survival (i.e. DFS and/or OS) of six months or less, one year or less, two years or less, three years or less, four years or less, five years or less, six years or less, etc. In one embodiment, the term poor prognosis refers to a DFS and/or OS of less than 5 years.

In an eighth aspect, the present invention relates to a method for monitoring or evaluating the response to treatment with an inhibitor of the TGFβ signaling pathway in a cancer patient, wherein said method comprises determining whether a patient's tumor has a TGFβ-activated microenvironment as described herein, wherein a TGFβ-activated microenvironment is associated to lack of response.

In a related aspect, it concerns a method for monitoring or evaluating the response to treatment with a TGFβ signaling pathway inhibitor in a cancer patient, wherein said method comprises:

a) determining the expression levels of a group of genes comprising CRYAB, NNMT, and SPRY4 in a tumor sample isolated from said patient;
b) calculating a score from the expression levels of the genes determined in step a); and
c) comparing the score in the patient's sample with a reference value; wherein a decrease of the score in the patient's sample with regard to said reference value is indicative of response to the treatment.

Preferably said treatment is an adjuvant treatment administered to the patient after surgical resection. In preferred embodiments, said reference value is a score value in this patient at an earlier time point.

**Kit and use of a kit in the methods of the invention**

In a ninth aspect, the invention concerns a kit suitable for determining the expression levels of the CRYAB, NNMT, and SPRY4 genes in an isolated tumor sample, wherein said kit comprises:

i. a reagent for the quantification of the CRYAB gene expression levels;
ii. a reagent for the quantification of the NNMT gene expression levels;
iii. a reagent for the quantification of the SPRY4 gene expression levels; and
iv. optionally, a reagent for the quantification of a control gene expression levels;
v. optionally, further comprising instructions for the use of said reagents in determining the expression levels of said genes in a tumor sample isolated from a cancer patient.

In preferred embodiments, said kit comprises:

i. an oligonucleotide specific to CRYAB mRNA or an affinity reagent for the CRYAB gene encoded protein;
ii. an oligonucleotide specific to NNMT mRNA or an affinity reagent for NNMT gene encoded protein;
iii. an oligonucleotide specific to SPRY4 mRNA or an affinity reagent for the SPRY4 gene encoded protein; and
iv. optionally, an oligonucleotide specific to a normalizing gene or an affinity reagent for the control gene encoded protein;
v. optionally, further comprising instructions for the use of said reagents in determining the expression levels of said genes in a tumor sample isolated from a cancer patient.

In further preferred embodiments, said kit comprises:

i. an oligonucleotide specific to SEQ ID NO:1 or an affinity reagent for SEQ ID NO:2;
ii. an oligonucleotide specific to SEQ ID NO:3 or an affinity reagent for SEQ ID NO: 4;
iii. an oligonucleotide specific to SEQ ID NO:5 or an affinity reagent for SEQ ID NO:6; and
iv. optionally, an oligonucleotide specific to a normalizing gene or an affinity reagent for the control gene encoded protein;
v. optionally, further comprising instructions for the use of said reagents in determining the expression levels of said genes in a tumor sample isolated from a cancer patient.

Said reagents in points i) to iv) may be as described herein above for the methods of nucleic acid and protein quantification in step (a) of the methods of the invention. In one embodiment, said oligonucleotide specific to CRYAB mRNA in i) is a primer and/or probe specific to CRYAB mRNA, said oligonucleotide specific to NNMT mRNA in ii) is a primer and/or probe specific to NNMT mRNA, and/or said oligonucleotide specific to SPRY4 mRNA in iii) is a primer and/or probe specific for SPRY4 mRNA.

In a preferred embodiment, said oligonucleotide specific to CRYAB mRNA, NNMT mRNA and SPRY4 mRNA, respectively, is a pair of primers (one forward and one reverse) and a hydrolysis probe (e.g. a Taqman probe) that aligns within the fragment amplified by the two primers.

For instance, these oligonucleotides specific to CRYAB mRNA, NNMT mRNA and SPRY4 mRNA, respectively, can comprise or consist of any of the primers and probes shown in the table below or an oligonucleotide sequence having at least 30% identity, preferably at least 35%, 40%, 45%, 50%, 55%, 60%, 65%, 70%, or at least 75% identity, more preferably at least 80%, 85%, 90%, 95%, 96%, 97%, 98% or at least 99% identity to any thereof:

| Gene | Forward primer | Reverse primer | Probe |
|---|---|---|---|
| CRYAB | SEQ ID NO: 7: CCTTCTACCTTCGGCCAC | SEQ ID NO: 8: TTCTCCAGGCGCATCTCT | SEQ ID NO: 9: TCCTTCCTGCGGGCACCCAGC |
| NNMT | SEQ ID NO: 10: TGAAGGGAACAGAGTCAAGG | SEQ ID NO: 11: GTCACATCACACTTCAGCAC | SEQ ID NO: 12: ACCGCCTGTCTCAACTTCTCCTCCTTC |
| SPRY4 | SEQ ID NO: 13: CGGCTTCAGGATTTACACAG | SEQ ID NO: 14: TTCTAGGGGCCTTTGAGGAG | SEQ ID NO: 15: TGCAGGGTCACAAGCATCGCCC |

In preferred embodiments, said kit comprises:

i. a pair of primers comprising or consisting of SEQ ID NO: 7 and SEQ ID NO: 8, or an oligonucleotide sequence having at least 75% identity to any thereof; and a probe consisting of SEQ ID NO: 9, or an oligonucleotide sequence having at least 75% identity thereof;
ii. a pair of primers comprising or consisting of SEQ ID NO: 10 and SEQ ID NO: 11, or an oligonucleotide sequence having at least 75% identity to any thereof; and a probe consisting of SEQ ID NO: 12 , or an oligonucleotide sequence having at least 75% identity thereof;
iii. a pair of primers comprising or consisting of SEQ ID NO: 13 and SEQ ID NO: 14, or an oligonucleotide sequence having at least 75% identity to any thereof; and a probe consisting of SEQ ID NO: 15, or an oligonucleotide sequence having at least 75% identity thereof; and
iv. optionally, an oligonucleotide specific to a normalizing gene;
v. optionally, further comprising instructions for the use of said reagents in determining the expression levels of said genes in a tumor sample isolated from a cancer patient.

In preferred embodiments, said sequence with at least 75% identity is a sequence with at least 80%, 85%, 90%, 95%, 96%, 97%, 98%, or at least 99% identity to the corresponding sequence. Identity percentage between the two sequences can be determined by any means known in the art, for example the Needleman and Wunsch global alignment algorithm.

Also, said kit may further comprise an oligonucleotide specific to a control gene. Preferably, said oligonucleotide is a primer and/or probe specific to said control gene. This is typically a housekeeping gene and preferably is one or more, such as two, three, four, five, six, seven, eight, nine, ten, eleven or twelve, selected from the group consisting of 18S, ACTB, B2M, GAPDH, GUSB2, HPRT, PPIA, RPLP, TBP, TUBB, UBC, and YWHAZ. For instance, these 12 genes can be amplified by using the primers and probes in the commercially available human Reference Gene Panels from TATAA Biocenter.

Said kit may comprise additional reagents. In a particular embodiment, said kit comprises reagents to perform a real-time PCR reaction, which typically contain a DNA polymerase, such as Taq DNA polymerase {e.g., hot- start Taq DNA polymerase), buffer, magnesium, dNTPs, and optionally other agents (e.g., stabilizing agents such as gelatin and bovine serum albumin). In addition, real-time PCR reaction mixtures also contain reagents for real time detection and quantification of amplification products as described above herein.

Another embodiment, refers a kit wherein said affinity reagent for the CRYAB gene encoded protein in i), said affinity reagent for the NNMT gene encoded protein in ii) and/or said affinity reagent for the SPRY4 gene encoded protein in iii) is a polypeptide, preferably an antibody, capable of specifically binding to CRYAB, NNMT or SPRY4, respectively.

Numerous methods known to those skilled in the art are available for obtaining antibodies or antigen-binding fragments thereof. Antibodies can be produced using recombinant DNA methods, see, e.g., Current Trends in Monoclonal Antibody Development (Steven Shire et al, Eds. Springer, 2010), the disclosures of which are incorporated herein by reference in their entirety. Monoclonal antibodies may also be produced by preparing immortalized cell lines capable of producing antibodies having the desired specificity. Such immortalized cell lines may be produced in a variety of ways. Conveniently, a small non-human animal, such as a mouse, is hyperimmunized with the desired immunogen. The vertebrate is then sacrificed, usually several days after the final immunization, the spleen cells removed, and the spleen cells immortalized. The most common technique is fusion with a myeloma cell fusion partner, as first described by Kohler and Milstein (1975) Nature 256:495-497. Other techniques, including EBV transformation, transformation with bare DNA, e.g., oncogenes, retroviruses, etc., or any other method which provides for stable maintenance of the cell line and production of monoclonal antibodies are also well known. Specific techniques for preparing monoclonal antibodies are described in Antibodies; A Laboratory Manual, Harlow and Lane, eds.. Cold Spring Harbor Laboratory, 1988, the full disclosure of which is incorporated herein by reference. Non-limiting examples of commercially available antibodies specifically binding to UNR protein are ab96124 (Abcam, Cambridge, UK), HPA018846 (Sigma-Aldrich) and HPA052221 (Sigma-Aldrich).

In a particular embodiment, said kit further comprises reagents to perform an immunohistochemistry (ICH) assay, which typically contain *interalia:* an enzyme-conjugated secondary antibody (e.g. conjugated to horseradish peroxidase or alkaline phosphatase), an enzyme substrate, and a counterstain such as hematoxylin. Kits for ICH are well known in the art and commercially available (http://www.sigmaaldrich.com/life-science/cell-biology/antibodies/antibodies-application/protocols/immunohistochemistry.html#reagents_equipment).

In a tenth aspect, the invention relates to the use of a kit as described above in any of the methods as described herein, such as for selecting a cancer patient which is likely to benefit from a treatment with a TGFβ signaling pathway inhibitor, for determining whether a patient's tumor microenvironment has a TGFβ -activated microenvironment, for predicting the tumor prognosis or for monitoring or evaluating the response to treatment with a TGFβ signaling pathway inhibitor.

**ITEMS OF THE INVENTION**

1. A method for determining whether a patient's tumor has a TGFβ-activated microenvironment, wherein said method comprises:

   a) determining the expression levels of a group of genes comprising CRYAB, NNMT, and SPRY4 in a tumor sample isolated from said patient;
   b) calculating a score from the expression levels of the genes determined in step a); and
   c) determining whether a patient's tumor has a TGFβ-activated microenvironment according to the score obtained in b).

2. The method according to item 1, wherein in step c) said method comprises comparing the score in the patient's sample with a reference value; and wherein an increase of the score in the patient's sample with regard to said reference value is indicative that said patient's tumor has a TGFβ-activated microenvironment.

3. The method according to any of items 1 or 2, wherein the expression levels of the genes CRYAB, NNMT, and SPRY4 have been normalized with respect to the expression levels of control genes in said isolated tumor sample.

4. The method according to item 3, wherein said control genes are selected from the group consisting of 18S, ACTB, B2M, GAPDH, GUSB2, HPRT, PPIA, RPLP, TBP, TUBB, UBC, and YWHAZ.

5. The method according to any of items 1 to 4, wherein the tumor sample isolated from said patient is a formalin-fixed, paraffin-embedded (FFPE) sample.

6. The method according to any of items 1 to 5, wherein the expression levels are mRNA expression levels, preferably determined by qRT-PCR.

7. The method according to any of items 1 to 6, wherein the score calculated in b) is proportional to the expression levels of CRYAB, NNMT, and SPRY4.

8. The method according to any of items 1 to 7, wherein said method is useful in the determination of a TGFβ-activated microenvironment in at least two, preferably three, four, five, six or seven different cancer types.

9. The method according to any of items 1 to 8, wherein the cancer is selected from the group consisting of colorectal cancer, stomach adenocarcinoma, bladder cancer, pancreas carcinoma, prostate cancer, lung cancer and breast cancer.

10. The method according to any of items 1 to 7, wherein the cancer is colorectal cancer (CRC).

11. The method according to any of items 1 to 10, wherein said method identifies a TGFβ-activated microenvironment in any of microsatellite instable (MSI) or microsatellite stable (MSS) tumors.

12. The method according to any of items 1 to 10, wherein said method identifies a TGFβ-activated microenvironment in any of CMS1, CMS2, CMS3 or CMS4 molecular subtype groups tumors.

13. The method according to any of items 1 to 12, wherein said patient is a human patient

14. The method according to any of items 1 to 13, wherein said method is a computer-implemented method.

15. A data-processing apparatus comprising means for carrying out the steps of a method of item 14.

16. A computer program comprising instructions which, when the program is executed by a computer, cause the computer to carry out the steps of the method of item 14.

17. A computer-readable storage medium having stored thereon a computer program according to item 16.

18. A method for selecting a cancer patient which is likely to benefit from a treatment with an inhibitor of the TGFβ signaling pathway, wherein said method comprises:

i. determining whether a patient's tumor has a TGFβ-activated microenvironment according to a method of any of items 1 to 14; and
ii. selecting said cancer patient for the treatment with an inhibitor of the TGFβ signaling pathway when said patient's tumor has a TGFβ-activated microenvironment.

19. An inhibitor of the TGFβ signaling pathway for use in a method for treatment of a cancer patient, wherein said patient's tumor has a TGFβ-activated microenvironment according to a method of any of items 1 to 14.

20. The inhibitor of the TGFβ signaling pathway for use according to item 19, wherein said inhibitor is administered to said patient in combination with another anti-cancer treatment.

21. The inhibitor of the TGFβ signaling pathway for use according to any of items 19 or 20, wherein said inhibitor is administered to said patient in combination with another anti-cancer agent or radiotherapy, preferably with a cytotoxic agent or immune checkpoint inhibitor.

22. A anti-cancer agent other than a TGFβ signaling pathway inhibitor, preferably a cytotoxic agent or immune checkpoint inhibitor, or radiotherapy for use in a method for treatment of a cancer patient in combination with an inhibitor of the TGFβ signaling pathway, wherein said patient's tumor has a TGFβ-activated microenvironment according to a method of any of items 1 to 14.

23. An anti-cancer agent other than a TGFβ signaling pathway inhibitor, preferably a cytotoxic agent or immune checkpoint inhibitor, or radiotherapy for use in a method for treatment of a cancer patient selected by a method of any of items 1 to 14 as failing to have a tumor with a TGFβ-activated microenvironment, wherein said other anti-cancer agent or radiotherapy is not administered in combination with an inhibitor of the TGFβ signaling pathway.

24. A method for determining the prognosis of a cancer patient, wherein said method comprises:

i. determining whether a patient's tumor has a TGFβ-activated microenvironment according to a method of any of items 1 to 14; and
ii. classifying the patient as having poor prognosis when said patient's tumor has a TGFβ-activated microenvironment.

25. A kit suitable for determining the expression levels of the genes CRYAB, NNMT, and SPRY4 in an isolated tumor sample, wherein said kit comprises:

i. an oligonucleotide specific to CRYAB mRNA or an affinity reagent for the CRYAB gene encoded protein;
ii. an oligonucleotide specific to NNMT mRNA or an affinity reagent for NNMT gene encoded protein;
iii. an oligonucleotide specific to SPRY4 mRNA or an affinity reagent for the SPRY4 gene encoded protein; and
iv. optionally, an oligonucleotide specific to a control gene mRNA or an affinity reagent for the control gene encoded protein;
v. optionally, further comprising instructions for the use of said reagents in determining the expression levels of said genes in a tumor sample isolated from a cancer patient.

26. Use of a kit according to item 25, in a method for determining whether a patient tumor's has a TGFβ-activated microenvironment according to any of items 1 to 14, in a method for selecting a cancer patient which is likely to benefit from a treatment with an inhibitor of the TGFβ signaling pathway according to item 18, or in a method for determining the prognosis of a cancer patient according to item 24.

It is contemplated that any features described herein can optionally be combined with any of the embodiments of any method, second medical use, kit, use of a kit, or computer program of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%). The following examples serve to illustrate the present invention and should not be construed as limiting the scope thereof.

## EXAMPLES

### Example 1.- Material and Methods

#### FFPE Cohort

FFPE colorectal cancer samples were obtained retrospectively from patients with colon or rectum adenocarcinoma who underwent surgical resection at 3 local Hospitals based in Barcelona, Spain: Hospital Clinic, Hospital del Mar, and Hospital Bellvitge. The study was approved by the Hospital Clinic Ethical Committee which acts as the reference Ethical Committee for research carried out at IRB Barcelona. All patients provided written informed consent, and the study was conducted in accordance with the Declaration of Helsinki. Retrospective CRC cases were collected according to the following guidelines: a) patients with stage 1-3 colon or rectum adenocarcinoma with no residual disease (with an emphasis on stage 2 and 3), b) patients aged 45 years or older at time of primary surgery with no family history of colorectal cancer in order to exclude potential hereditary cases; c) No preoperative cancer therapy.

Table 1. Demographics for patients included in the FFPE cohort.

| **MOMTGFB FFPE Cohort** | **Disease Free** n=194 | **Relapse** n=75 |
|---|---|---|
| Age at time of diagnosis (Years; Mean) | 73,6 | 73,7 |
| Follow up (Mean) | 5 | 5 |
| Gender<br>Female<br>Male | <br>92<br>102 | <br>33<br>42 |

(continued)

| | | |
|---|---|---|
| Localization of tumor | | |
| Caecum/Right | 76 | 33 |
| Transverse | 12 | 3 |
| Left + Sigma | 94 | 35 |
| Rectum | 11 | 3 |
| n/d | 1 | 1 |
| Stage | | |
| I* | 10 | 2 |
| II | 117 | 33 |
| III | 67 | 40 |
| T-stage | | |
| T1/T2 | 19 | 3 |
| T3/T4 | 175 | 72 |
| Differentiation | | |
| Well/Moderate (G1/G2) | 169 | 66 |
| Low (G3/G4) | 25 | 9 |
| Lymphovascular Invasion (LVI) | | |
| Yes | 56 | 40 |
| No | 139 | 35 |
| Microsatellite Status | | |
| MSI | 22 | 8 |
| MSS | 168 | 66 |
| n/d | 4 | 1 |
| Chemotherapy | | |
| Yes | 36 | 16 |
| No | 158 | 59 |

**Macrodissection**

H&E histological sections from FFPE preserved tumors from the patients described above were examined under a microscope in order to perform a quality control over the samples provided. Upon approval, the equivalent (depending on sample size) of 3x 10 μm paraffin sections of 4x2 cm area were placed in a tube for further processing, avoiding the addition of non-invaded tissues or normal mucosa.

**RNA extraction, reverse transcription and qRT-PCR analysis**

The FFPE samples were extracted using RNeasy FFPE Kit #73504 (Qiagen). The relative expression of 41 biomarkers was measured including MOMTGFB genes, TGFβ genes and the normalizing genes in commercially available human Reference Gene Panels (TATAA Biocenter) listed in Table 2. The specific primers and probes used for the quantification of MOMTGFB genes and TGFβ genes are as described in Table 3.

RNA was reverse transcribed in two 40 μl reactions with 2000 ng RNA per reaction. In the two 40 μl RT reactions reverse primers from the 41 genes of interest together with 12 normalization genes were used to reverse transcribe the RNA. The RT was performed using manufacturer's instructions with Qscript Flex cDNA kit (Quanta BioSciences cat #95049-100) to generate cDNA. After reverse transcription, the cDNA in the two 40 μl reactions were mixed and diluted 10X by adding additional 720 μl of RNase free $H_2O$ and stored in -20°C until further analysis.

QPCR analysis of the generated cDNA was performed on all 270 FFPE samples with qPCR assays designed to detect the genes. All qPCR analysis was performed in 20 μl reaction volume in duplicates on a LightCycler 480 instrument (Roche) with TATAA Probe® GrandMaster Mix # TA02-625 (TATAA Biocenter AB). The ValidPrimeTM (Kit#A105S25, TATAA Biocenter AB) concept was used to monitor and correct for contaminating gDNA (Laurell et al, Nucleic Acids Research, 2012, 1-10). All pipetting was performed by robot (EpMotion 5070, Eppendorf, Germany). The reaction conditions are described below (exception TGFB3_01, B2M and GUSB which had half the normal concentration of primers and probes to avoid primer dimer formation).

Table 2. MOMTGFB genes, TGFβ genes and normalizing genes

| Gene Name | NCBI Accession number (mRNA) |
|---|---|
| CRYAB | NM_001885.2 |
| NNMT | NM_006169.2 |
| SPRY4 | NM_001127496.1 |
| TGFB1 | NM_000660.5 |
| TGFB2 | NM_003238.3 |
| TGFB3 | NM_003239.2 |
| B2M | NM_004048 |
| GUSB | NM_000181 |
| GAPDH | NM_002046 |
| 18S | NR_003286 |
| UBC | NM_021009 |
| ACTB | NM_001101 |
| RPLP | NM_001002 |
| YWHAZ | NM_003406 |
| TBP | NM_003194 |
| HPRT | NM_000194.3 |
| PPIA | NM_021130.5 |
| TUBB | NM_001293212.1 |

Table 3. Primers and probes used for the quantification of CRYAB, NNMT, SPRY4, TGFβ1, TGFβ2 and TGFβ3 genes expression products by qRT-PCR

| Gene | Forward primer | Reverse primer | Probe |
|---|---|---|---|
| CRYAB | SEQ ID NO: 7: CCTTCTACCTTCGGCCAC | SEQ ID NO: 8: TTCTCCAGGCGCATCTCT | SEQ ID NO: 9: TCCTTCCTGCGGGCACCCAGC |
| NNMT | SEQ ID NO: 10: TGAAGGGAACAGAGTCAAGG | SEQ ID NO: 11: GTCACATCACACTTCAGCAC | SEQ ID NO: 12: ACCGCCTGTCTCAACTTCTCCTCCTTC |
| SPRY4 | SEQ ID NO: 13: CGGCTTCAGGATTTACACAG | SEQ ID NO: 14: TTCTAGGGGCCTTTGAGGAG | SEQ ID NO: 15: TGCAGGGTCACAAGCATCGCCC |
| TGFB1 | SEQ ID NO: 16: TCGCCAGAGTGGTTATCTTT | SEQ ID NO: 17: ATTTCCCCTCCACGGCTC | SEQ ID NO: 18: ACCACTGCCGCACAACTCCGGTG |
| TGFB2 | SEQ ID NO: 3419: TTGCTTTAGAAATGTGCAGG | SEQ ID NO: 20: CCATTTCCACCCTAGATCCC | SEQ ID NO: 21: ATTGCTGCCTACGTCCACTTTACATTGATTTC |
| TGFB3 | SEQ ID NO: 22: GCTGTTGAGAAGAGAGTCCA | SEQ ID NO: 23: CATTGGGCTGAAAGGTGTG | SEQ ID NO: 24: CTTAGGTCTAGAAATCAGCATTCACTGTCCATG |

**Master Mix protocol (per reaction)**

| | |
|---|---|
| Primers (forward/reverse) and probe: | 2.0 μl (4μM primers,2μM probe) |
| TATAA Probe GrandMaster Mix: | 10.0 μl (2x) |
| ddH2O: | 2.0 μl |
| Template: | 6.0 μl |
| Total reaction volume: | 20.0 μl |

**Temperature protocol**

| | | | |
|---|---|---|---|
| Step 1 | 60s | 95°C | 1 cycle (Activation) |
| Step 2 | 5 sec | 95°C | 45 cycles (Amplification) |
| | 45 sec | 60°C | (fluorescence measurement) |

**Microsatellite Instability testing**

Microsatellite instability (MSI) status of the CRC adenocarcinomas from FFPE cohort was evaluated through immunohistochemistry staining with antibodies to detect the presence of MLH1, MSH2, MSH6 and PMS2 following standard routine protocols from each hospital.

**F-/T-/Ma-TBRS and TGFB scores**

Response to TGFβ in cancer fibroblasts (F-TBRS), T-cells (T-TBRS) and macrophages (Ma-TBRS) was assessed in whole tumors samples using the gene expression signature previously published (Calon et al., Cancer Cell 2012, 22, 571-584). For doing so, expression values were centered and scaled gene-wise to produce z-scores, which were then averaged across all genes included in a given gene signature. The resulting scores were in turn centered and scaled across samples that were included in the dataset. The same procedure was carried out to obtain scores for TGFβ family genes (TGFB1+TGFB2+TGFB3). These calculations were carried out after correcting the expression data by potential sources of bias due to technical variability (section Transcriptomic datasets of human CRC samples).

**MOMTGFB score**

The levels of expression of CRYAB, NNMT and SPRY4 (the MOMTGFB genes) were used by the inventors for the calculation of a score (referred as MOMTGFB score) as the sum of the product between the normalized MOMTGFB genes expression values and their estimated regression coefficients obtained in a regression analysis. To normalize measurements in the paraffin cohort measured by qRT-PCR, the gene expression values of each patient were normalized by subtracting the mean expression of the control genes in that patient. In particular, the following 12 genes were used as control genes: 18S, ACTB, B2M, GAPDH, GUSB, HPRT, PPIA, RPLP, TBP, TUBB, UBC, YWHAZ. Normalization of samples from transcriptomic cohorts was performed as described below (please see section on pre-processing of datasets)

The following formula was used for the calculation of the score in the data presented herein:

$$\text{MOMTGFB score} = 0.30 * \text{CRYAB} + 0.36 * \text{NNMT} + 0.19 * \text{SPRY4}$$

**Transcriptomic datasets**

**Colorectal Cancer**

Seven public high-throughput datasets were used for transcriptomic analyses in human tumors [see summary in table below], including five Affymetrix microarray series publicly available in the NCBI GEO repository (Barrett et al., Nucleic Acids Res (2013), 41 (Database issue):D991-5) and the TCGA RNAseq datasets for Colon and Rectum tumors (TCGA Research Network: http://cancergenome.nih.gov/). Consensus Molecular Subtypes (CMS) information was available for most of these samples and was retrieved from (Guinney et al., Nature Medicine (2015), 21:1350-1356).

| DATABASE | N | Link |
|---|---|---|
| GSE14333 | 290 | https://www.ncbi.nlm.nih.gov/pubmed/19996206 |
| GSE33113 | 90 | https://www.ncbi.nlm.nih.gov/pubmed/22496204 |
| GSE39582 | 562 | https://www.ncbi.nlm.nih.gov/pubmed/23700391 |
| GSE38832 | 122 | https://www.ncbi.nlm.nih.gov/pubmed/25320007 |
| GSE44076 | 98 | https://www.ncbi.nlm.nih.gov/pubmed/25253512 |
| TCGA-COAD | 393 | https://www.cancer.gov/about-nci/organization/ccg/research/structural-genomics/tcga |
| TCGA-READ | 150 | https://www.cancer.gov/about-nci/organization/ccg/research/structural-genomics/tcga |

**Other Cancer Types**

The datasets below were used for the indicated cancer types.

| DATABASE | Cancer Type | N | Link |
|---|---|---|---|
| TCGA-STAD | Stomach Adenocarcinoma | 402 | https://www.cancer.gov/about-nci/organization/ccg/research/structural-genomics/tcga |
| TCGA-BLCA | Bladder Cancer | 300 | https://www.cancer.gov/about-nci/organization/ccg/research/structural-genomics/tcga |
| TCGA-PAAD | Pancreas Adenocarcinoma | 155 | https://www.cancer.gov/about-nci/organization/ccg/research/structural-genomics/tcga |
| GSE21034 | Prostate | 131 | https://www.ncbi.nlm.nih.gov/pubmed/20579941 |
| GSE31210 | Lung | 226 | https://www.ncbi.nlm.nih.gov/pubmed/22080568 |
| Metabric | Breast | 1974 | https://www.nature.com/articles/nature10983 |

**Pre-processing of TCGA CRC data**

RNA-Seq (version 2) data from the The Cancer Genome Atlas (TCGA) project was retrieved from the legacy version of the GDC commons repository (Grossman et al., New England Journal of Medicine (2016), 375: 1109-1112). Clinical and follow-up information was retrieved from the TCGA-Clinical Data Resource (CDR) [https://gdc.cancer.gov/about-data/publications/PanCan-Clinical-2018]. Expression measures were expressed in RSEM in the legacy version, which were log2-transformed and quantile normalized. Samples from different tumors (colon and rectum) and from different platforms (Genome Analyzer and HiSeq) were processed separately. Non-primary tumor samples were filtered out and duplicated samples measured in different platforms were excluded from the HiSeq subset. Only primary tumors from patients with no previous cancer diagnosis were kept for analyses. An exploratory analysis was carried out using Principal Component Analyses in order to identify samples with abnormal expression values in each dataset. Samples TCGA-A6-2679-01A and TCGA-AA-A004-01A were excluded as their gene expression showed an anomalous distribution compared to the rest of samples in their dataset, even after quantile normalization. Finally, each dataset was corrected by technical variation using a mixed-effect linear model, in which sample's center of origin and plate identifiers were modeled as a fixed and a random effect, respectively. Coefficients from the fixed effects and random effects' imputations provided by the models were then used to correct these technical effects.

**Pre-processing of microarray CRC datasets**

Microarray datasets were separately processed using R packages affy (Gautier et al., Bioinformatics (2004), 3: 307-315) and affyPLM (Bolstad et al., Gentleman R, Carey V, Huber W, Irizarry R, and Dudoit S. (Eds.), (2005) Springer, New York) from Bioconductor (Gentleman et al., Genome Biology (2004), 5: R80). Raw cel files data were processed using RMA (Irizarry et al., Biostatistics (2003), 4:249-264) and annotated using the information available in the Affymetrix web page (Affmetrix - Thermofisher web page. https://www.thermofisher.com/es/en/home/life-science/microarray-analysis/affymetrix.html).

Standard quality controls were performed in order to identify abnormal samples (Gentleman et al., Bioinformatics and Computational Biology Solutions Using R and Bioconductor (2005) (Springer, New York)) regarding: a) spatial artefacts in the hybridization process (scan images and pseudo-images from probe level models); b) intensity dependences of differences between chips (MvA plots); c) RNA quality (RNA digest plot); d) global intensity levels (boxplot of perfect match log-intensity distributions before and after normalization and RLE plots); e) anomalous intensity profile compared to the rest of samples (NUSE plots, Principal Component Analyses). Technical information concerning samples processing and hybridization was retrieved from the original CEL files: date of scanning were collected in order to define scan batches in each dataset separately; technical metrics described by Eklund AC and Szallasi Z in (Eklund and Szallasi, Genome Biology (2008), 9, R26) were computed and recorded as additional features for each sample. Microarray expression values were summarized at the gene level (entrez) using the first principal component of the probesets mapping to the same gene. This component was centered and scaled to the weighted mean of the means and standard deviations of the probesets using the corresponding contribution to the component as weight. The sign of this gene-based expression summary was imputed so that it was congruent to the sign of the probeset contributing the most to the original first component.

Previously to gene-level summarization, each microarray dataset was corrected a priori by Eklund metrics (Eklund and Szallasi, Genome Biology (2008), 9, R26) effects estimated from a standard linear model. In addition, mixed-effect linear models were used to correct microarray expression by the sample's center of origin (if more than one) and date of scanning (microarrays). Expression values were corrected using the coefficients from the fixed effects and the imputations of the random effects provided by the models.

**Final CRC transcriptomic pooled cohort (TCGA + GEO)**

Datasets were merged after gene-wise standardization by median and median absolute deviation to the GSE39582 dataset. As a result, this CRC pooled cohort (TCGA + GEO) included a total of 1.705 primary tumor samples.

**MSI imputation in transcriptomic CRC samples**

When not available in the published clinical information, MSI status was imputed in each dataset separately based on the expression of genes included in a published transcriptomic signature (Jorissen et al., Clinical Cancer Research (2008), 14, 8061-8069). For doing so, Pearson's correlation coefficients were computed between each sample's profile and an artificial MSI profile consisting on "one" values for genes included in the signature that were over-expressed in MSI samples and a "zero" values for genes over-expressed in MSS samples in (Jorissen et al., Clinical Cancer Research (2008), 14, 8061-8069). Assignation to MSI or MSS was performed according to results of a cluster analysis based on non-parametric density estimation (Azzalini A., Torelli N.S., Statistics and Computing (2007), 17, 71-80; Azzalini A., Menardi G., Journal of Statistical Software (2014), 57(11), 1-26) on these correlation coefficients. This imputation procedure was performed at the gene level using the entrez identifier and Affymetrix annotation (Affmetrix - Thermofisher web page) before correction by tecnical effects (see section Transcriptomic datasets of human tumors); for doing so, not-corrected microarray datasets were summarized at the gene level by the most variable probeset mapping to the same gene as measured by the median absolute deviation. In each case, the same number of top MSI and MSS over-expressed genes were used in order to avoid biases towards the group that included more genes (MSI). Accuracy of this imputation was evaluated in dataset GSE39582 and TCGA colon dataset, which included annotation of microsatellite-stable (MSS) and -instable (MSI) samples in their clinical information (GSE39582: 97% and 77% accuracy for MSS and MSI samples, respectively; TCGA colon: 97% and 92% accuracy for MSS and MSI, respectively).

**Cell Population Scores**

Three data sets publicly available in the NCBI GEO repository (Barrett et al., Nucleic Acids Res (2013), 41 (Database issue):D991-5) were used to characterize the subtype gene profiles according to specific gene expression in tumor cell subpopulations: GSE39395, GSE39396 [Calon A, et al., Cancer Cell (2012) 22(5), 571-84] and GSE35602 [Nishida Net al., Clin Cancer Res (2012) 18, 3054-70]. In GSE39395 and GSE39396, FACS was used to separate the following populations from 14 fresh CRC samples: CD45+EpCAM-CD31-FAP-, CD45-EpCAM+CD31-FAP-, CD45-EpCAM-CD31 +FAP- and CD45-EpCAM- CD31-FAP+. GSE35602 contains transcriptomic data for epithelial and stromal cells microdissected from 13 CRC tissue samples and 4 adjacent morphologically normal colorectal mucosae (>5 cm from the tumor). GSE39395 and GSE39396 were processed using RMA [Irizarry RA, et al., Biostatistics (2003) 4, 249-64] (see section Pre-processing of microarray CRC datasets) while the series matrix version of GSE35602 was used in the analyses.

Cell population signatures were derived after differential expression analysis using moderated t-statistics by empirical Bayes shrinkage (limma) [Ritchie, M.E. et al., Nucleic Acids Research 43(7), e47.]. For doing so, stromal genes were selected as they showed differential expression (2 fold-change, FDR < 1%) between epithelial and stromal compartments of samples in dataset GSE35602, and simultaneously over-expressed in the target population compared to the rest of cell types (5 fold-change, FDR < 1%). The Benjamini-Hochberg method was used for multiple comparison adjustment [Controlling the False Discovery Rate: A Practical and Powerful Approach to Multiple Testing Yoav Benjamini and Yosef Hochberg Journal of the Royal Statistical Society. Series B (Methodological) Vol. 57, No. 1 (1995), pp. 289-300]. Cell population signatures in the CRC transcriptomic meta-cohort were obtained as described in the previous section (F-/T-/Ma-TBRS and TGFB scores). Finally, estimations of the immune infiltration in the tumors of the CRC transcriptomic meta-cohort shown in Figure 7 were computed as the difference of the CD45 signature and the sum of the stromal signatures (CD45 + FAP + CD31) obtained in this way. The resulting score was centered and scaled across samples in the dataset.

**Trancriptomic datasets - other cancer types**

TCGA RNASeq data for stomach (STAD), bladder (BLCA) and pancreatic (PAAD) adenocarcinoma were downloaded from the GDC commons repository (Grossman et al., New England Journal of Medicine (2016), 375: 1109-1112) and processed in an analogous way to the colon and rectum TCGA datasets. Likewise, microarray datasets of prostate - GSE21034 (Taylor et al., Cancer Cell (2010), 18: 11-22) and Lung - GSE31210 (Okayama et al., Cancer Research (2011), 72: 100-111) were downloaded from the NCBI GEO repository and processed as described previously (see sections on Pre-processing of microarray CRC datasets). Unlike the rest of microarray data, dataset GSE21034 was processed using the oligo R package [Carvalho BS, Irizarry RA (2010). "A Framework for Oligonucleotide Microarray Preprocessing." Bioinformatics, 26(19), 2363-7. ISSN 1367-4803, doi: 10.1093/bioinformatics/btq431], as their samples were hybridized in a different Affymetrix platform. (Human Exon 1.0 ST Array). Finally, Metabric breast cancer data (Curtis et al., Nature (2012), 486, 346-352) was downloaded from the cBioportal for Cancer Genomics [http://cancerdiscovery.aacrjournals.org/content/2/5/401.abstract]; Metabric's expression values were a-priori corrected using a linear model in which PAM50 subtype was included as a covariate.

**Association analyses with clinical outcome in human datasets**

Association with relapse was evaluated using a Cox proportional hazards model. For the rest of association analyses with gene expression levels, linear models were carried out. In all cases, sample's dataset of origin was included in the models for statistical adjustment. In addition and for signatures evaluation, a global signature was computed using all the genes in the expression matrix and used as adjusting variable in the models. This strategy has been proved to be useful to avoid systematic biases due to the gene-correlation structure present in the data and to adjust by the expectation under gene randomization, i.e., the association expected for a signature whose genes have been chosen at random [Adjusting for systematic technical biases in risk assessment of gene signatures in transcriptomic cancer cohorts. Adrià Caballé Mestres, Antonio Berenguer-Llergo, Camille Stephan-Otto Attolini doi: https://doi.org/10.1101/360495; On Testing the Significance of Sets of Genes. Bradley Efron and Robert Tibshirani. The Annals of Applied Statistics. Vol. 1, No. 1 (Jun., 2007), pp. 107-129].

Statistical significance was assessed by means of a Log-likelihood Ratio Test (LRT), while Wald tests were used for pair-wise comparisons. Sample groups of low, medium and high expression levels were defined using the mean and -1 standard deviation as cutoffs. Hazard Ratios (HR) were computed as measures of association. For visualization purposes, Kaplan-Meier survival curves and boxplots were estimated for groups of samples showing low, medium and high gene or signature expression levels. Also, Cox proportional hazard ratios smoothed by continuous signature expression levels were graphically represented using smoothing splines with a p-spline basis (Eilers, Paul H. and Marx, Brian D., Statistical Science 11 (1996) 89-121) as implemented in the R package phenoTest (Evarist Planet (2018). phenoTest: Tools to test association between gene expression and phenotype in a way that is efficient, structured, fast and scalable. R package version1.30.0.). In addition, a heatmap was built to graphically show the correlation between multiple gene expression signatures simultaneously. In this heatmap, centered and scaled signature values were showed using a white to black color gradation, where black indicated the highest expression and white corresponded to the lowest expression values. For clarity, the most extreme expression values were truncated to -1.5 and 1.5.

Only samples from patients diagnosed in stages I, II and III were taken into consideration for analyses of time to relapse, for a total of 955 CRC samples; out of them 768 samples were Microsatellite Stable tumors (MSS) while 186 were Microsatellite Instable (MSI); TCGA's rectum samples were excluded from prognosis analyses accordingly to recommendations of the TCGA-Clinical Data Resource (CDR) [Liu J, Lichtenberg T, Hoadley KA, Poisson LM, Lazar AJ, Cherniack AD, Kovatich AJ, Benz CC, Levine DA, Lee AV, Omberg L, Wolf DM, Shriver CD, Thorsson V; Cancer Genome Atlas Research Network, Hu H. An Integrated TCGA Pan-Cancer Clinical Data Resource to Drive High-Quality Survival Outcome Analytics. Cell. 2018 Apr 5;173(2):400-416.e11. doi: 10.1016/j.cell.2018.02.052. PubMed PMID: 29625055; PubMed Central PMCID: PMC6066282.]. The threshold for statistical significance was set at 5%. All analyses were carried out using R (Team, R. C. R: A language and environment for statistical computing. R Foundation for Statistical Computing, Vienna, Austria (2018), at http://www.r-project.org).

**Generation and Treatment of Mouse Tumors**

Mouse tumor organoids (MTOs) mutant in four pathways involved in colorectal cancer progression were obtained from genetically modified mice (described in Tauriello et al., Nature, Vol. 554 (2018) 539 - 543). Briefly, we crossed mouse strains bearing engineered alleles for four of the most common genetic alterations found in human CRC - $Apc^{fl/fl}$, $Kras^{LSL-G12D}$, $Tgfbr2^{fl/fl}$ and $p53^{fl/fl}$- and recombined these mutations in intestinal stem cells by means of the Lgr5-creERT2 driver (LAKTP mice). Quadruple mutant mice developed metastatic intestinal tumors that reproduced several key features of human poor prognosis microsatellite stable CRCs including a stromal rich TGFβ-activated TME and T cell exclusion. From these mouse tumors, we derived a biobank of 3D MTOs (described in Tauriello et al., Nature, Vol. 554 (2018) 539 - 543). Upon transplantation into the caecum of wild-type syngeneic fully immunocompetent C57BL/6 mice, these tumor organoids (MTOs) generated primary CRCs that resemble those from the tumor of origin.

Three different MTOs derived from compound LAKTP mutant mice were orthotopically transplanted into syngeneic C57BL/6J mice. MTO1 organoids grown for 3 days were harvested from their 3D growth matrix (basement membrane extract, BME), counted and injected as intact spheroids in 30% BME below the serosa of the caecum of an anaesthetized mouse. Parental MTOs 2 and 3 were first injected subcutaneously and 1 $mm^3$ pieces of resulting tumors were transplanted onto the tip of the caecum of syngeneic mice; the caecum tip was then folded over to mitigate carcinomatosis. Mice were treated with 10mg of Galunisertib (GAL) (or placebo) twice per day by gavage, starting at day 11 after tumor implantation and lasting until sacrifice at 5 weeks. This treatment was associated to a strong reduction/prevention of liver metastasis, as well as a reduction of primary tumor (and local carcinomatosis) size (Tauriello et al., Nature, Vol. 554 (2018) 539 - 543).

**Analysis of Transcriptomic data from mouse tumors**

Gene expression data (GSE103562) obtained from mouse tumor samples (Tauriello et al., Nature (2018), 554,538-543) were processed similarly to Affymetrix human samples (Pre-processing of microarray CRC datasets). Previously to gene-level summarization, expression values were corrected a priori by metrics RMA.IQR and RNA.DEG described in Eklund AC et al., (Genome Biol. (2008), 9(2):R26). Next, genes were translated to their corresponding human homologous using the Mouse Genome Informatics Database. (14.353 genes) (Blake JA, et al., Nucleic Acids Res. (2017) 45(D1):D723-D729). Gene signatures scores were computed on this corrected and humanized expression matrix. Association of gene expression with treatment was assessed using a linear model in which sample's organoid of origin was included as a covariate.

**Example 2.- MOMTGFB score in FFPE samples of colorectal cancer (CRC) patients**

**2.1 Association of the MOMTGFB score with TGFβ in FFPE samples of colorectal cancer (CRC) patients**

RNA was extracted from retrospective FFPE samples from patients diagnosed with stage I-III colorectal cancer treated at three different hospitals in Barcelona ("FFPE cohort" (n=270)). Samples had clinical data associated.

As shown in **Figure 1,** the score computed from the predictor named MOMTGFB associated well with high overall TGFβ levels (TGFβ-1, -2 & -3) in these patients.

**2.2. Association of the MOMTGFB score with disease free survival (DFS) in FFPE samples of CRC patients**

In addition, MOMTGFB score was shown to segregate patients with poor prognosis in a cohort of CRC patients in FFPE samples (FFPE cohort) where the expression of MOMTGFB genes was measured by qRT-PCR, see **Figure 2.**

We found a linear association between the average expression of MOMTGFB score and the risk of relapse after therapy. Based on this finding we stratified patients on High or Low MOMTGFB score. Kaplan Meier curves evaluating disease-free survival invariably show that patients with high MOMTGFB tumors had worse prognosis. This was true for all patients (Stage 1-3) and also for patients when further stratified in stage II or stage III. Actually, MOMTGFB is a better predictor of survival than TGFβ itself, both in Cox univariate and multivariate analysis.

| | HR | CI | P-value |
|---|---|---|---|
| **Cox univariate** | | | |
| TGFB (+1SD) | 1.41 | (1.11 -1.78) | 0.0045103457 |
| momtgfb (+1SD) | 1.63 | (1.29 - 2.07) | 0.0000430262 |
| **Cox multivariate** | | | |
| TGFB (+1SD) | 1.43 | (1.12 - 1.83) | 3.769550e-03 |
| LVI.TRUEvsFALSE | 2.83 | (1.79 - 4.49) | 1.015781e-05 |
| momtgfb (+1SD) | 1.72 | (1.34 - 2.21) | 1.696988e-05 |
| LVI.TRUEvsFALSE | 2.96 | (1.87 - 4.69) | 4.775679e-06 |

**Example 3.- Elevated MOMTGFB score identifies a subset of patients with high TGFβ levels and high F-TBRS, T-TBRS and Ma-TBRS expression in transcriptomic cohorts**

The inventors obtained good data demonstrating that the group of patients with higher MOMTGFbeta scores are characterized by higher overall TGFβ levels, and also a higher degree of TGF-beta-activated TME, shown by higher levels of TGFβ response signatures from fibroblasts (F-TBRS), T-cells (T-TBRS) and macrophages (Ma-TBRS) in transcriptomic cohorts from various types of cancer compared to patients with medium and low MOMTGFB scores.

***3.1 Association of MOMTGFB score with TGFβ levels and F-/T-/Ma-TBRS in CRC transcriptomic cohorts (Pooled cohort GEO + TCGA):***

The results in **Figure 3** show that the 3 genes in the MOMTGFB score captured the essence of a TGFβ-activated stroma in a pooled transcriptomic cohort of CRC. In particular, this cohort contains n=1705 patients and pools together data from TCGA, as well as GEO datasets: GSE14333, GSE33113, GSE39582, GSE38832, and GSE44076.

MOMTGFB score was calculated by applying a specific coefficient to the expression values of each gene (see the MOMTGFB score formula in Example 1 above) and compared versus the F-/T-/Ma-TBRS (from Calon et al., Cancer Cell 22 (2012) 571-584) and overall TGFβ levels (average expression of TGFβ1,-β2 and -β3 levels). Sample groups of low, medium and high expression levels were defined using the mean and -1 standard deviation as cutoffs.

As referred above, MOMTGFB score can also be calculated without coefficients computing solely gene expression levels. These scores identified tumors with TGFβ-activated TME in a similar manner albeit with slightly poorer results (data not shown). This applies to all comparisons between MOMTGFB scores groups and expression of TGFβ or TBRS shown throughout this document.

Molecular classifications of colorectal cancer (CRC) based on global gene expression profiles have defined the CMS4 subtype as displaying resistance to therapy and poor prognosis (Guinney et al. Nat Med. 2015, 21(11), 1350-6). On the contrary, patients with tumors classified as CMS1, 2 or 3 are considered of good prognosis.

Patients with CMS4 tumors show overall worse prognosis compared to CMS1-3. **Figure 4A** provides Kaplan Meier curves showing disease free survival for patients with associated CMS data in the pooled transcriptomic cohort (GEO +TCGA). As depicted in **Figure 4B,** CMS4 tumors show a high degree of TGF-β-activated stroma according to their high overall expression levels of F-, T-, and Ma-TBRS. Accordingly, most CMS4 tumors fall into the MOMTGFB high category. Finally, the table in **Figure 4C** shows that MOMTGFB captures 96.1% of CMS4 patients. These patients are likely to benefit from therapies directed towards inhibition of TGFβ signaling.

Additionally, MOMTGFB score identifies patients with a high degree of TGFβ active stroma within other subgroups considered of good prognosis (CMS1-3), and particularly amongst CMS 1 and 3 (see **Figure 5A**). Kaplan Meier analysis (see **Figure 5B**) demonstrates that patients with high MOMTGFB levels within CMS1 and CMS3 have worse prognosis and therefore these patients may also benefit from therapies that inhibit TGF-β signaling. Importantly, patients with CMS1 tumors are likely candidates to checkpoint immunotherapies. Around 50% (**Figure 4C**) of these patients present tumors with a high degree of TGFβ-activated TME, and will likely not respond to such therapies alone (as described in Tauriello et al., Nature, Vol. 554 (2018) 539 - 543).

Therefore, MOMTGFB captures a large proportion of CMS4 tumors as well as bad prognosis tumors in other CMS that show a TGFβ-activated TME.

In addition, the inventors identified that high MOMTGFB scores associated with tumor exhibiting elevated overall TGFβ, F-TBRS, T-TBRS and Ma-TBRS in both MSS (microsatellite stable) and MSI (microsatellite instable) subgroups of patients (**Figure 6**). Accordingly, MOMTGFB signature performance in the identification of a TGFβ-activated stroma has been found to be independent from MSI status. Generally, MSI patients are considered as good prognosis patients and are therefore typically not selected for neoadjuvant and/or adjuvant treatments. The MOMTGFB signature would thus be capable of capturing a subset of MSI patients that have high levels of TGF-β, poor prognosis and would likely benefit from anti-TGFβ therapies. In particular, administration of TGFβ signaling pathway inhibitors would be particularly useful in combination with immune checkpoint inhibitors since these patients are likely to be no-responders to checkpoint immunotherapy alone. Again, under this classification, MSI patients are likely candidates to checkpoint immunotherapies. Around 48% (**Figure 6B**) of these patients present tumors with a high degree of TGFβ-activated TME, and will likely not respond to such therapies alone, unless in combination with TGFβ signaling inhibitory therapies.

The inventors further show heatmaps (**Figure 7**) illustrating the correlations between MOMTGFB score with average gene expression of overall F-TBRS, T-TBRS and Ma-TRBRS (from Calon et al., Nature Genetics 47:4 (2015) 320-332) and distribution of Consensus Molecular Subtypes (CMS) in CRC transcriptomic cohorts (TCGA+GEO: 1705 patients), including patients from stages I to IV.

The combination of the MOMTGFB genes shows a footprint of tumors that have a TGFβ-activated TME, as well as immune-excluded (see reverse pattern for expression of a signature of CD45 positive cells that captures the relative abundance of leukocytes compared to the other cell types present in the TME). With respect to the distribution of patients within CMS, it is observed that CMS4 patients cluster with MOMTGFB high and TGFβ-activated stromal signatures, whereas CMS2 patients show the reverse pattern. CMS1 patients are distributed all along the axis indicating that those with high TGFβ-active TME which segregates with bad prognosis (**Figure 5B**) also could potentially benefit form therapies inhibiting TGFβ signaling pathway. Importantly, these patients are likely candidates to receive immune checkpoint inhibitors therapy. The presence of a TGF-beta-activated TME in 50.9% of patients with CMS1 tumors will likely predict no-response to these therapies alone, unless in combination with TGFβ signaling inhibitory therapies.

### *3.2. Association of MOMTGFB score with overall TGFβ, F-TBRS, T-TBRS and Ma-TRBRS expression levels in transcriptomic cohorts of stomach adenocarcinoma (STAD)*

The combined 3 genes in the MOMTGFB test capture the essence of a TGFβ-activated microenvironment in the transcriptomic cohorts of STAD (from TCGA), as shown in Figure 8. MOMTGFB scores are shown for all patients or for patients according to their MSS or MSI status.

### *3.3. Association of MOMTGFB score with overall TGFβ, F-TBRS, T-TBRS and Ma-TRBRS expression levels in transcriptomic cohorts of bladder cancer*

The 3 genes in the MOMTGFB score captured the essence of a TGFβ-activated TME in the TCGA transcriptomic cohort of bladder adenocarcinoma, as shown **Figure 9.**

### *3.4. Association of MOMTGFB score with overall TGFβ, F-TBRS, T-TBRS and Ma-TRBRS expression levels in transcriptomic cohorts of pancreas adenocarcinoma (TCGA)*

The 3 genes in the MOMTGFB score captured the essence of a TGFβ-activated TME in the TCGA transcriptomic cohort of pancreas adenocarcinoma, as shown **Figure 10.**

### *3.5. Association of MOMTGFB score with overall TGF-β, F-TBRS, T-TBRS and Ma-TRBRS expression levels in transcriptomic cohorts of prostate carcinoma (GSE21034)*

The 3 genes in the MOMTGFB score captured the essence of a TGFβ-activated TME in a transcriptomic cohort of prostate carcinoma, as shown in **Figure 11.**

### *3.6. Association of MOMTGFB score with overall TGF-β, F-TBRS, T-TBRS and Ma-TRBRS expression levels in transcriptomic cohorts of lung cancer (GSE31210)*

The 3 genes in the MOMTGFB score captured the essence of a TGFβ-activated TME in a transcriptomic cohort of lung cancer, as shown in **Figure 12.**

### *3.7. Association of MOMTGFB score with with overall TGFβ, F-TBRS, T-TBRS and Ma-TRBRS expression levels in transcriptomic cohorts of breast cancer (Metabric cohort)*

The inventors obtained the MOMTGFB scores in the Metabric cohort of breast cancer patients (Curtis et al., Nature (2012) 486(7403): 346-52. From the data in **Figure 13,** association with TBRSs is good for various breast cancer subtypes, in particular Her2 and Basal. Indeed, MOMTGFB captures patients with a TGFβ-activated TME much better than TGFβ itself.

As described extensively for CRC patients, in all these additional examples (3.2-3.7) patients with high MOMTGFB scores, and thus, TGFβ-active TME could potentially benefit from therapies inhibiting TGFβ signaling pathway.

**Example 4.- MOMTGFB genes identify the microenvironment of tumors that respond to anti TGFβ therapies**

Galunisertib (GAL), a TGFBR1 inhibitor, was used to treat mice with TGFβ high metastatic CRCs. The graph in **Figure 14** shows that after therapy of metastatic CRC with GAL there is a reduction of MOMTGFB score. Thus, the presented data evidences that the three gene signature is a good biomarker of response to TGFβ inhibition therapies.

**Claims**

1. A method for determining whether a patient's tumor has a TGFβ-activated microenvironment, wherein said method comprises:

   a) determining the expression levels of a group of genes comprising CRYAB (Crystallin Alpha B), NNMT (Nicotinamide N-Methyltransferase), and SPRY4 (Sprouty RTK Signaling Antagonist 4) genes in a tumor sample isolated from said patient;
   b) calculating a score from the expression levels of the genes determined in step a); and
   c) comparing the score in the patient's sample with a reference value; wherein an increase of the score in the patient's sample with regard to said reference value is indicative that said patient's tumor has a TGFβ-activated microenvironment.

2. The method according to claim 1, wherein the expression levels of the genes in step a) have been normalized with respect to the expression levels of control genes in said isolated tumor sample.

3. The method according to any of claims 1 or 2, wherein the score calculated in b) is proportional to the expression levels of CRYAB, NNMT, and SPRY4.

4. The method according to any of claims 1 to 3, wherein said method is not tissue-specific.

5. The method according to any of claims 1 to 4, wherein said tumor is selected from the group consisting of colorectal cancer, stomach adenocarcinoma, bladder cancer, pancreas carcinoma, prostate cancer, lung cancer and breast cancer.

6. The method according to any of claims 1 to 5, wherein said method identifies a TGFβ-activated microenvironment in any of microsatellite instable (MSI) or microsatellite stable (MSS) tumors.

7. The method according to any of claims 1 to 6, wherein said method identifies a TGFβ-activated microenvironment in any of CMS1, CMS2, CMS3 or CMS4 molecular subtype groups tumors.

8. A method for selecting a cancer patient which is likely to benefit from a treatment with an inhibitor of the TGFβ signaling pathway, wherein said method comprises:

   i. determining whether a patient's tumor has a TGFβ-activated microenvironment according to a method of any of claims 1 to 7; and
   ii. selecting said cancer patient for the treatment with an inhibitor of the TGFβ signaling pathway when said patient's tumor has a TGFβ-activated microenvironment.

9. An inhibitor of the TGFβ signaling pathway for use in a method for treatment of a cancer patient, wherein said patient's tumor has a TGFβ-activated microenvironment according to a method of any of claims 1 to 7.

10. The inhibitor of the TGFβ signaling pathway for use according to claim 9, wherein said inhibitor is administered to said patient in combination with another anti-cancer agent or radiotherapy, preferably with a cytotoxic agent or immune checkpoint inhibitor.

11. An anti-cancer agent other than a TGFβ signaling pathway inhibitor, preferably a cytotoxic agent or immune check-

point inhibitor, or radiotherapy for use in a method for treatment of a cancer patient in combination with an inhibitor of the TGFβ signaling pathway, wherein said patient's tumor has a TGFβ-activated microenvironment according to a method of any of claims 1 to 7.

**12.** A method for determining the prognosis of a cancer patient, wherein said method comprises:

i. determining whether a patient's tumor has a TGFβ-activated microenvironment according to a method of any of claims 1 to 7; and
ii. classifying the patient as having poor prognosis when said patient's tumor has a TGFβ-activated microenvironment.

**13.** A method for monitoring or evaluating the response to treatment with an inhibitor of the TGFβ signaling pathway in a cancer patient, wherein said method comprises determining whether a patient's tumor has a TGFβ-activated microenvironment according to a method of any of claims 1 to 7, wherein a TGFβ-activated microenvironment is associated to lack of response.

**14.** A kit suitable for determining the expression levels of the genes CRYAB, NNMT, and SPRY4 in an isolated tumor sample, wherein said kit comprises:

i. a pair of primers comprising or consisting of SEQ ID NO: 7 and SEQ ID NO: 8, or an oligonucleotide sequence having at least 75% identity to any thereof; and a probe consisting of SEQ ID NO: 9, or an oligonucleotide sequence having at least 75% identity thereof;
ii. a pair of primers comprising or consisting of SEQ ID NO: 10 and SEQ ID NO: 11, °or an oligonucleotide sequence having at least 75% identity to any thereof; and a probe consisting of SEQ ID NO: 12 , or an oligonucleotide sequence having at least 75% identity thereof;
iii. a pair of primers comprising or consisting of SEQ ID NO: 13 and SEQ ID NO: 14, or an oligonucleotide sequence having at least 75% identity to any thereof; and a probe consisting of SEQ ID NO: 15, or an oligonucleotide sequence having at least 75% identity thereof; and
iv. optionally, an oligonucleotide specific to a normalizing gene;
v. optionally, further comprising instructions for the use of said reagents in determining the expression levels of said genes in a tumor sample isolated from a cancer patient.

**15.** Use of a kit suitable for determining the expression levels of the genes CRYAB, NNMT, and SPRY4 in an isolated tumor sample, in a method for determining whether a patient tumor's has a TGFβ-activated microenvironment according to any of claims 1 to 7, in a method for selecting a cancer patient which is likely to benefit from a treatment with a TGFβ signaling pathway inhibitor according to claim8, in a method for determining the prognosis of a cancer patient according to claim 12 or in a method for monitoring or evaluating the response to treatment with a TGFβ signaling pathway inhibitor according to claim 13;
wherein said kit comprises:

i. a reagent for the quantification of the CRYAB gene expression levels;
ii. a reagent for the quantification of the NNMT gene expression levels;
iii. a reagent for the quantification of the SPRY4 gene expression levels; and
iv. optionally, a reagent for the quantification of a control gene expression levels;
v. optionally, further comprising instructions for the use of said reagents in determining the expression levels of said genes in a tumor sample isolated from a cancer patient.

FIG.1

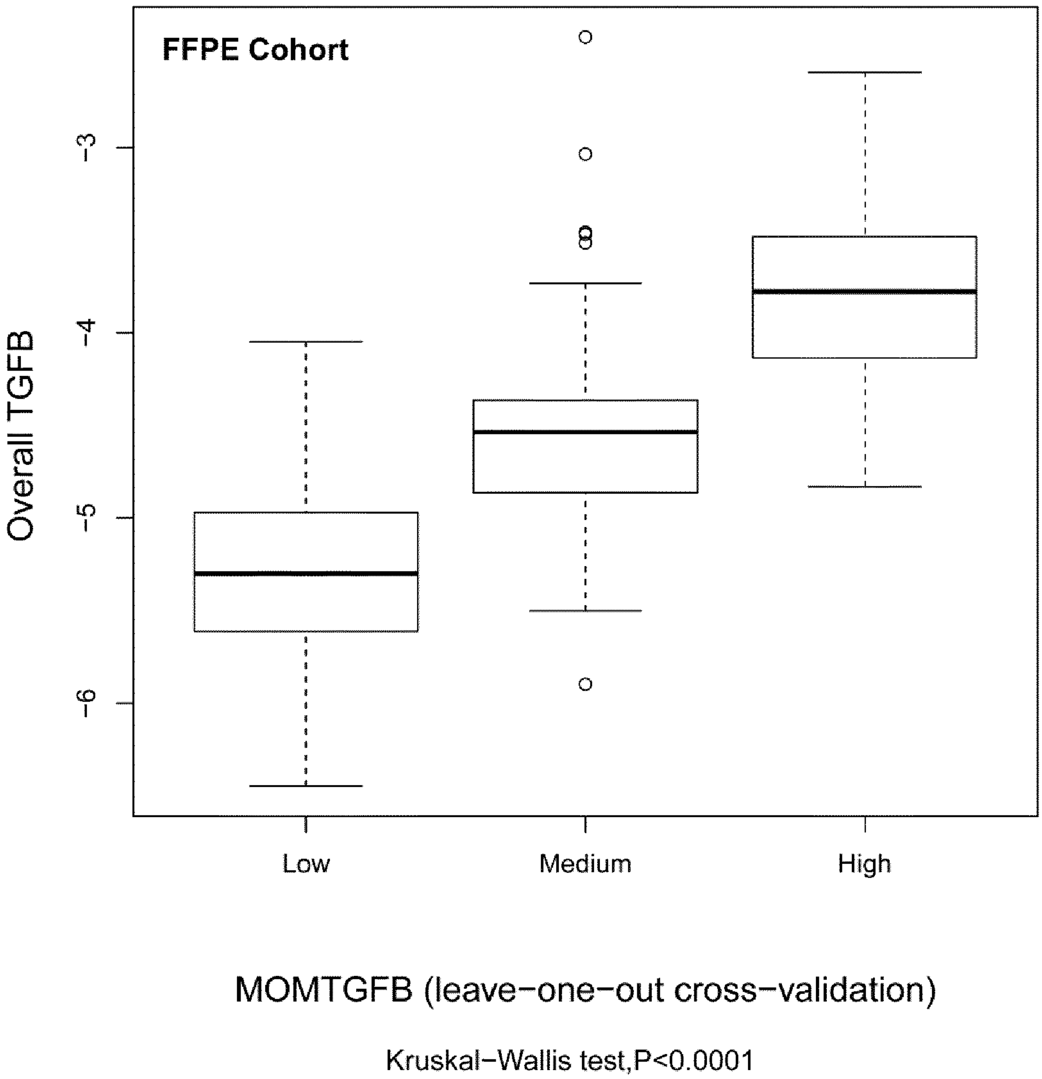
FFPE Cohort
Overall TGFB
-3
-4
-5
-6
Low
Medium
High
MOMTGFB (leave-one-out cross-validation)
Kruskal-Wallis test,P<0.0001

FIG. 2

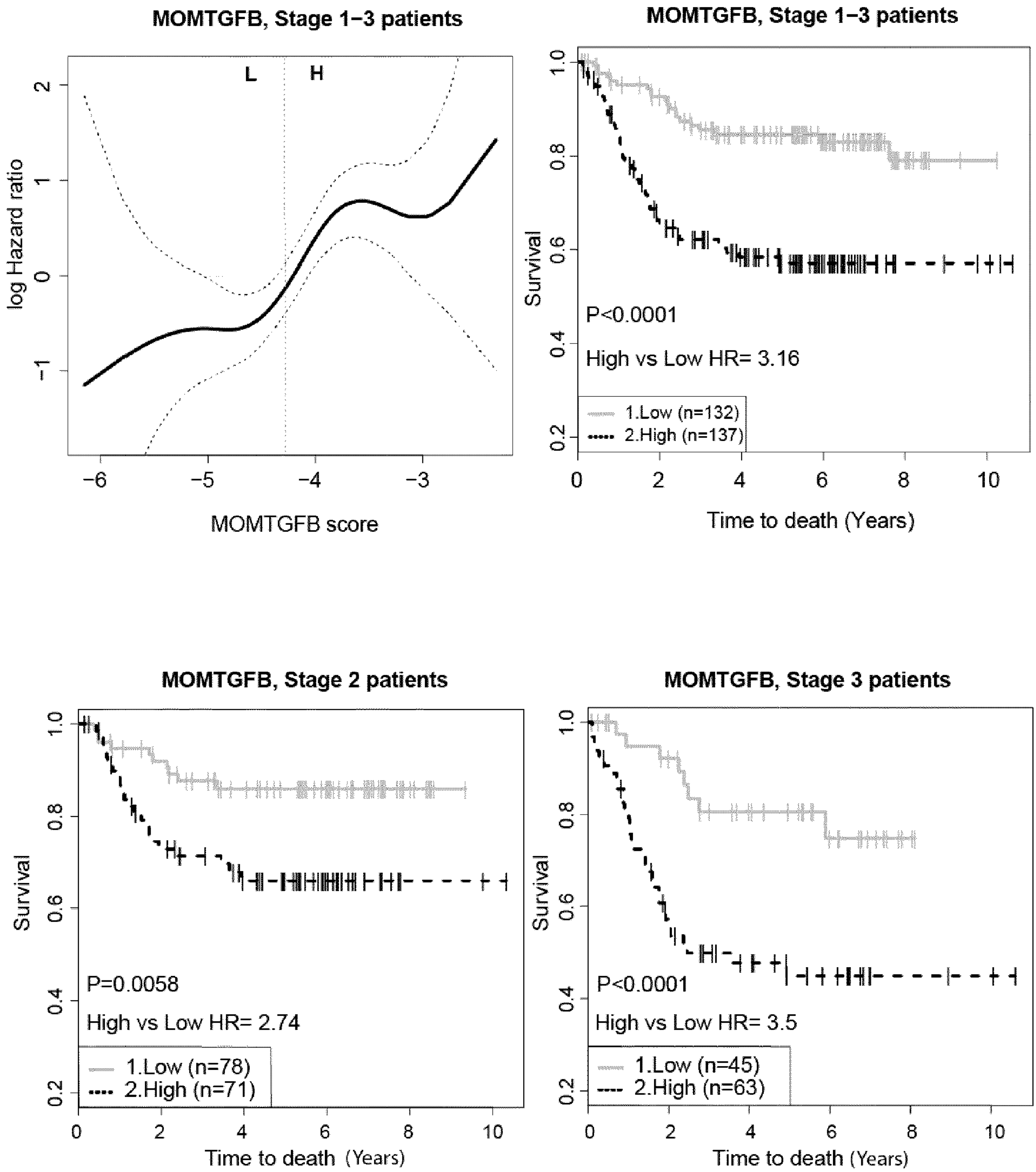
MOMTGFB, Stage 1−3 patients
L
H
log Hazard ratio
MOMTGFB score
MOMTGFB, Stage 1−3 patients
Survival
P<0.0001
High vs Low HR= 3.16
1.Low (n=132)
2.High (n=137)
Time to death (Years)
MOMTGFB, Stage 2 patients
Survival
P=0.0058
High vs Low HR= 2.74
1.Low (n=78)
2.High (n=71)
Time to death (Years)
MOMTGFB, Stage 3 patients
Survival
P<0.0001
High vs Low HR= 3.5
1.Low (n=45)
2.High (n=63)
Time to death (Years)

FIG.3

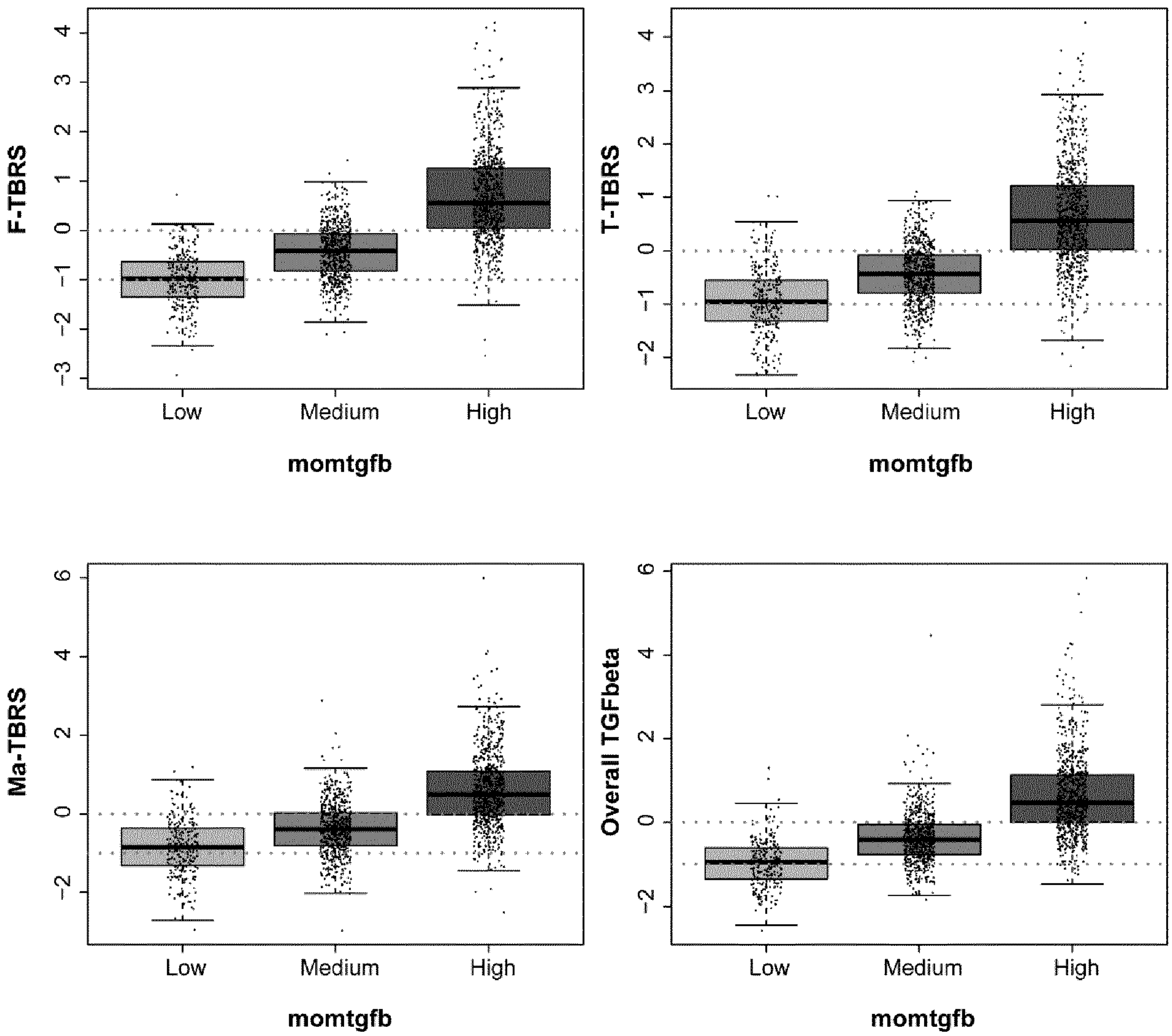
F-TBRS
4
3
2
1
0
−1
−2
−3
Low
Medium
High
momtgfb
T-TBRS
4
3
2
1
0
−1
−2
Low
Medium
High
momtgfb
Ma-TBRS
6
4
2
0
−2
Low
Medium
High
momtgfb
Overall TGFbeta
6
4
2
0
−2
Low
Medium
High
momtgfb

FIG.4

A

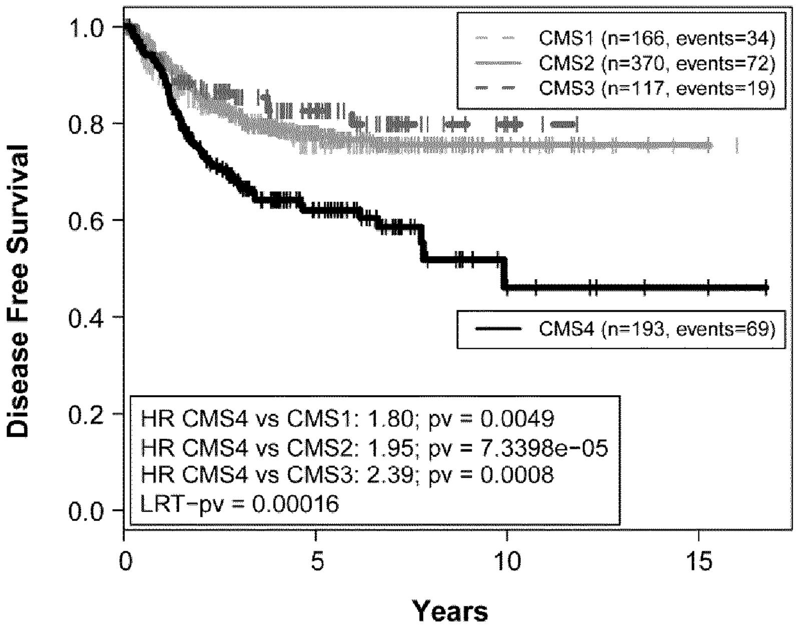


B

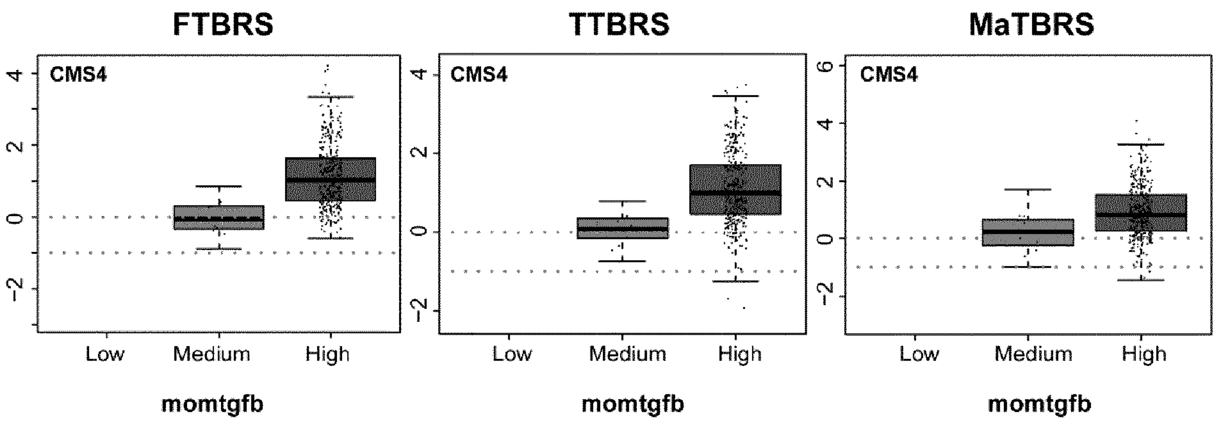


C

| MOMTGFb | CMS1 | CMS2 | CMS3 | CMS4 |
|---|---|---|---|---|
| Low | 20 (9.1%) | 106 (18.7%) | 86 (48.3%) | 0 (0.0%) |
| Medium | 88 (40%) | 328 (57.8%) | 69 (38.8%) | 13 (3.9%) |
| High | 112 (50.9%) | 133 (23.5%) | 23 (12.9%) | 322 (96.1%) |

FIG.5

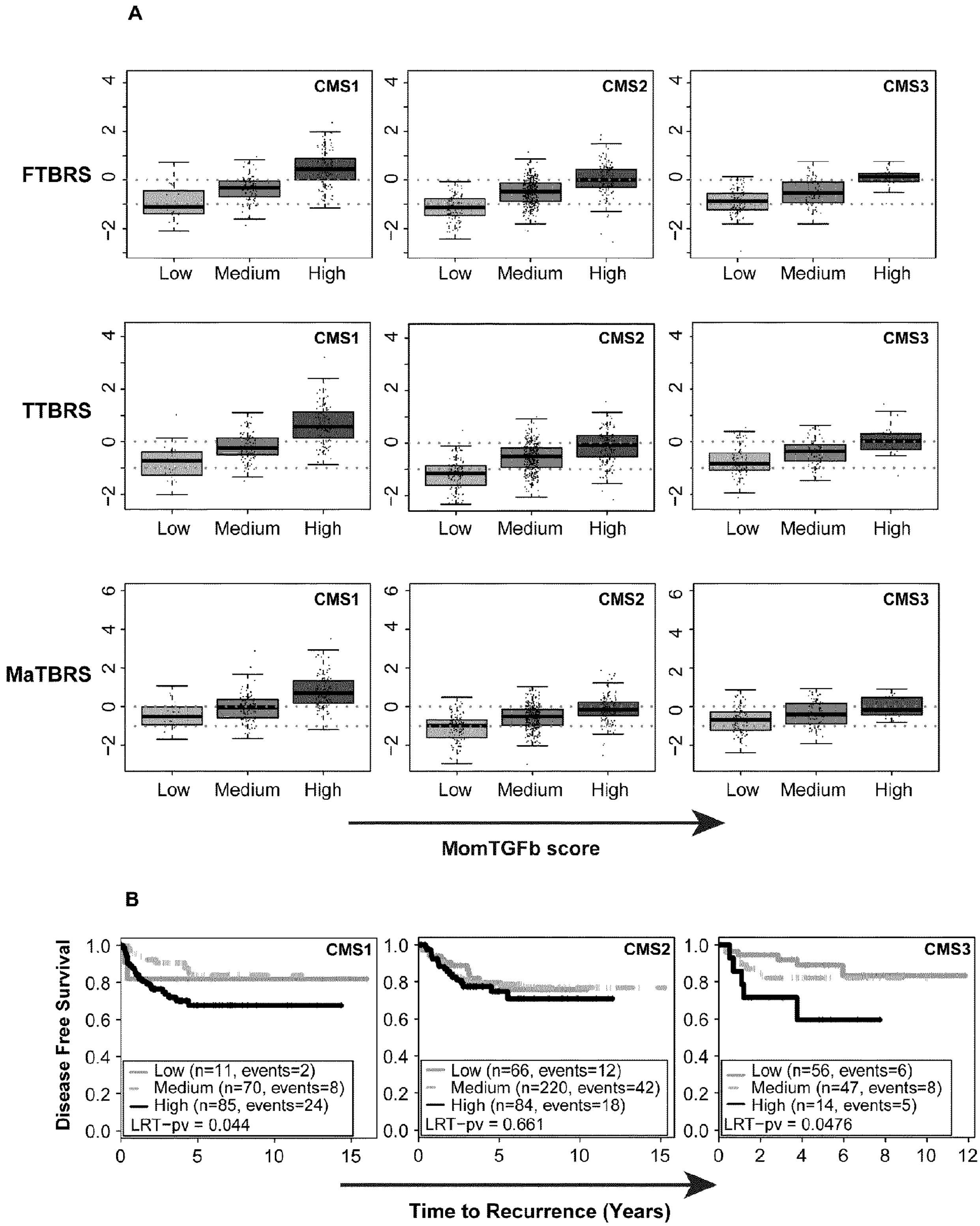
A
FTBRS
TTBRS
MaTBRS
CMS1
CMS2
CMS3
Low Medium High
MomTGFb score
B
Disease Free Survival
Low (n=11, events=2)
Medium (n=70, events=8)
High (n=85, events=24)
LRT−pv = 0.044
Low (n=66, events=12)
Medium (n=220, events=42)
High (n=84, events=18)
LRT−pv = 0.661
Low (n=56, events=6)
Medium (n=47, events=8)
High (n=14, events=5)
LRT−pv = 0.0476
Time to Recurrence (Years)

FIG.6

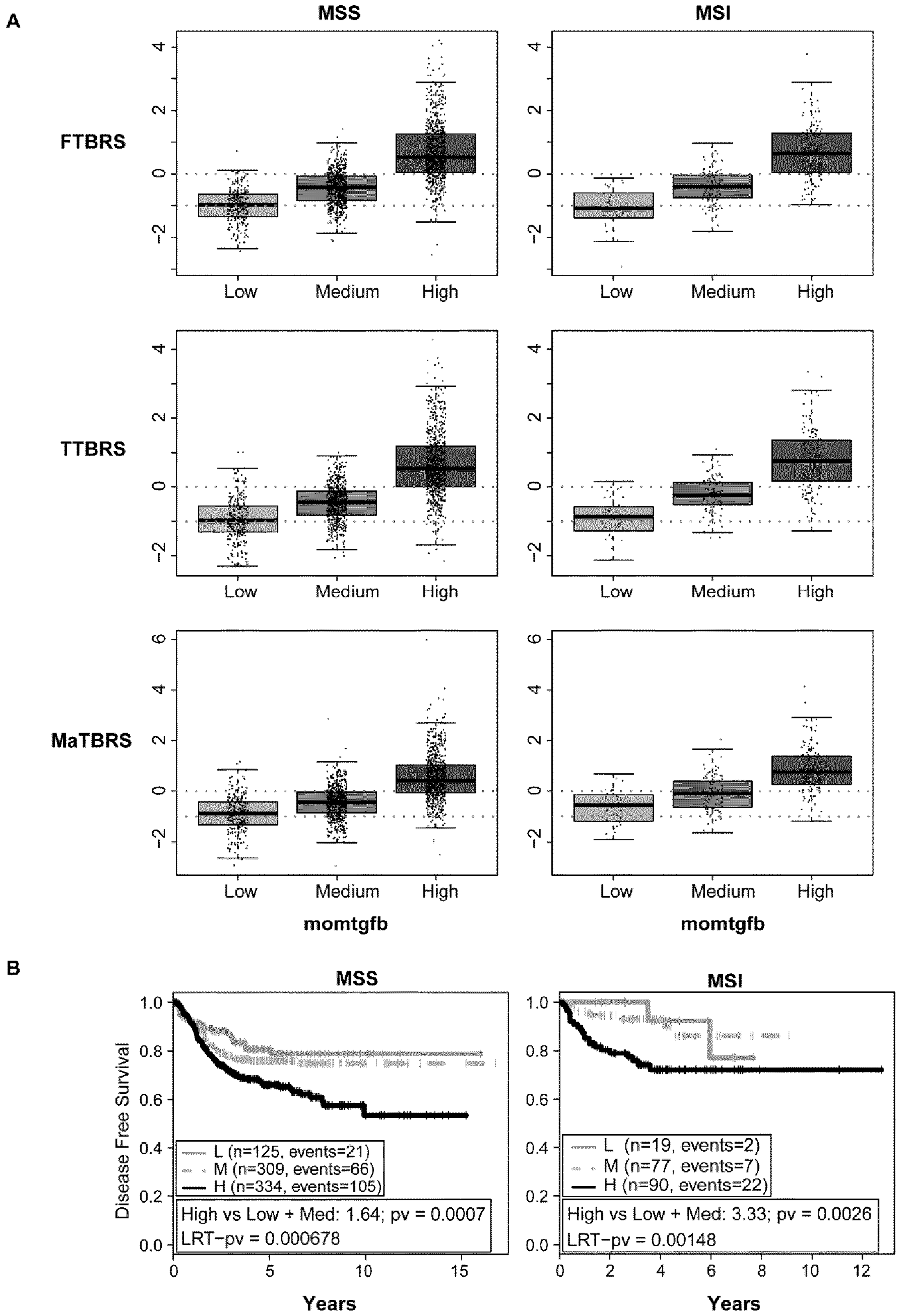
A
MSS
MSI
FTBRS
TTBRS
MaTBRS
Low
Medium
High
momtgfb
B
MSS
MSI
Disease Free Survival
L (n=125, events=21)
M (n=309, events=66)
H (n=334, events=105)
High vs Low + Med: 1.64; pv = 0.0007
LRT−pv = 0.000678
L (n=19, events=2)
M (n=77, events=7)
H (n=90, events=22)
High vs Low + Med: 3.33; pv = 0.0026
LRT−pv = 0.00148
Years
Years

FIG.7

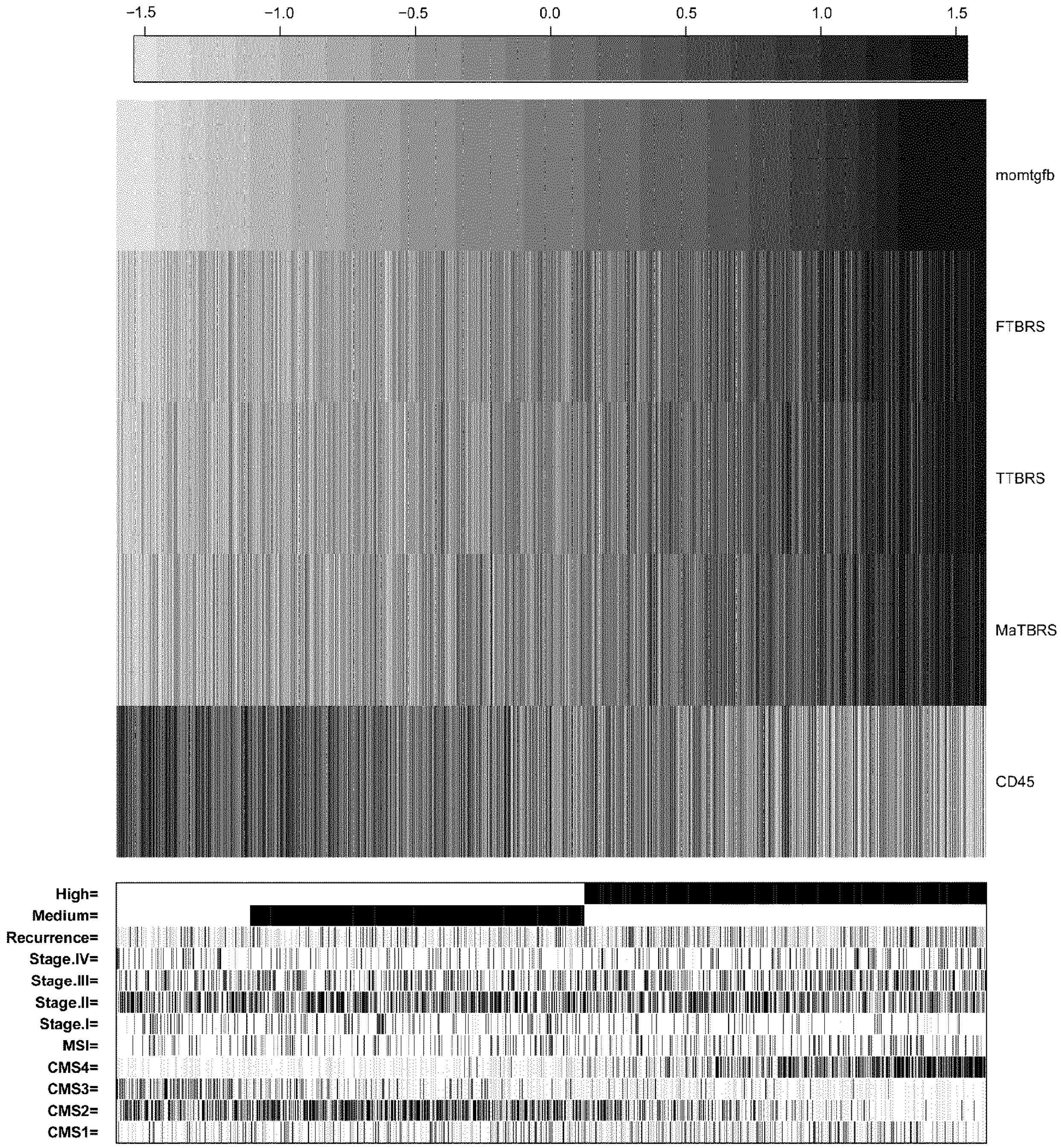
-1.5
-1.0
-0.5
0.0
0.5
1.0
1.5
momtgfb
FTBRS
TTBRS
MaTBRS
CD45
High=
Medium=
Recurrence=
Stage.IV=
Stage.III=
Stage.II=
Stage.I=
MSI=
CMS4=
CMS3=
CMS2=
CMS1=

FIG.8

Stomach Adenocarcinoma (STAD)

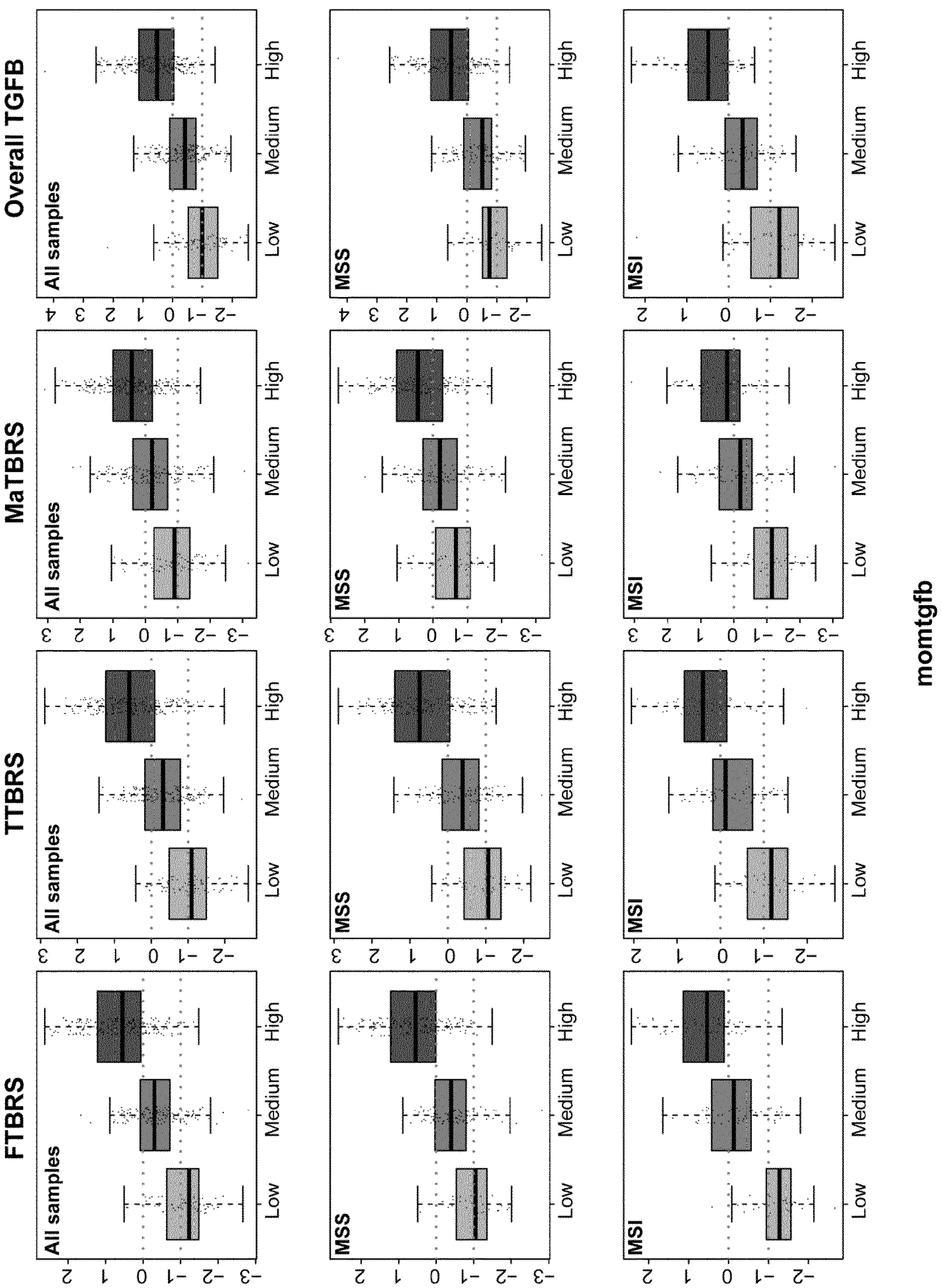

FIG.9

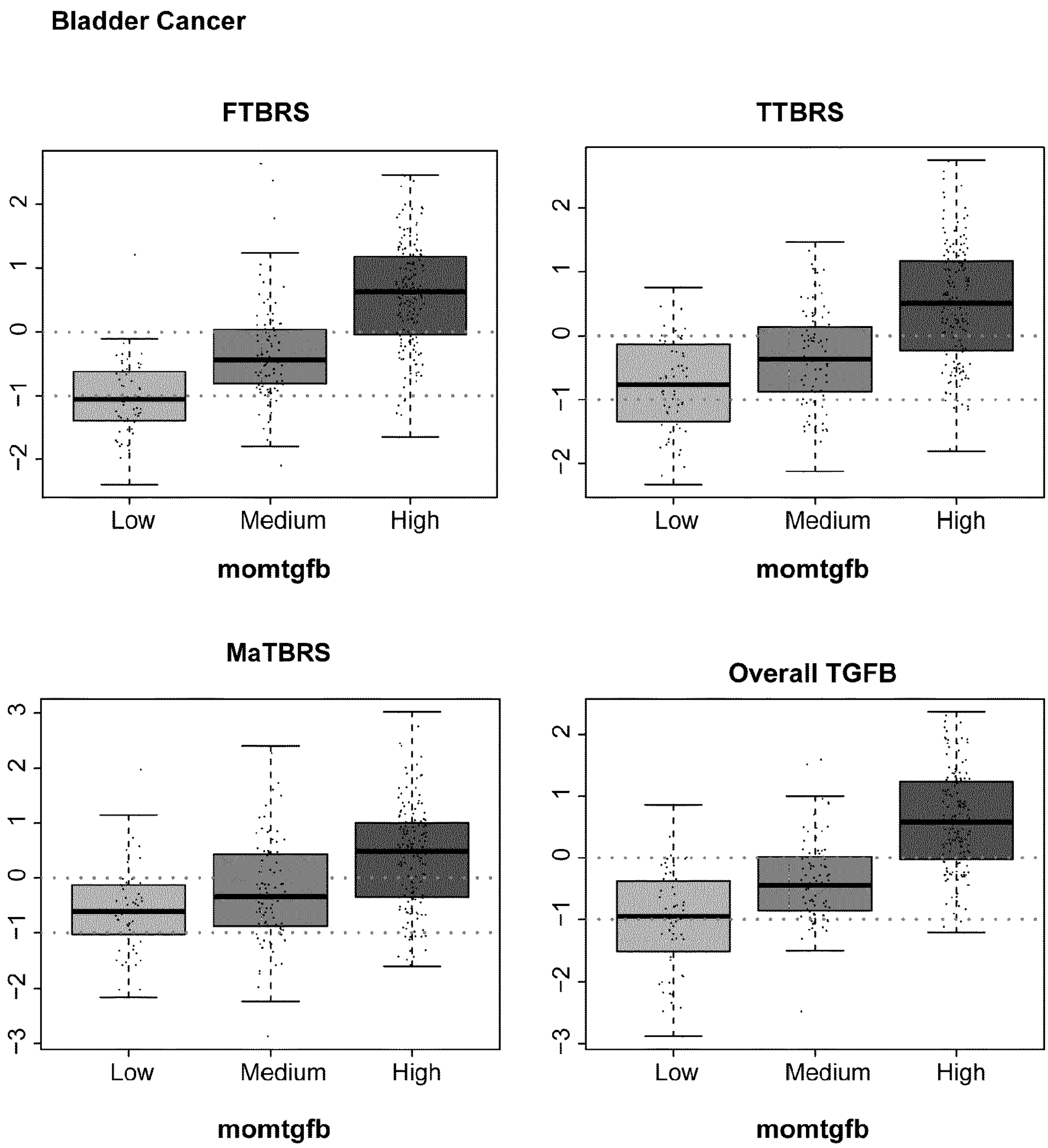
Bladder Cancer
FTBRS
TTBRS
MaTBRS
Overall TGFB
Low
Medium
High
momtgfb
3
2
1
0
−1
−2
−3

FIG. 10

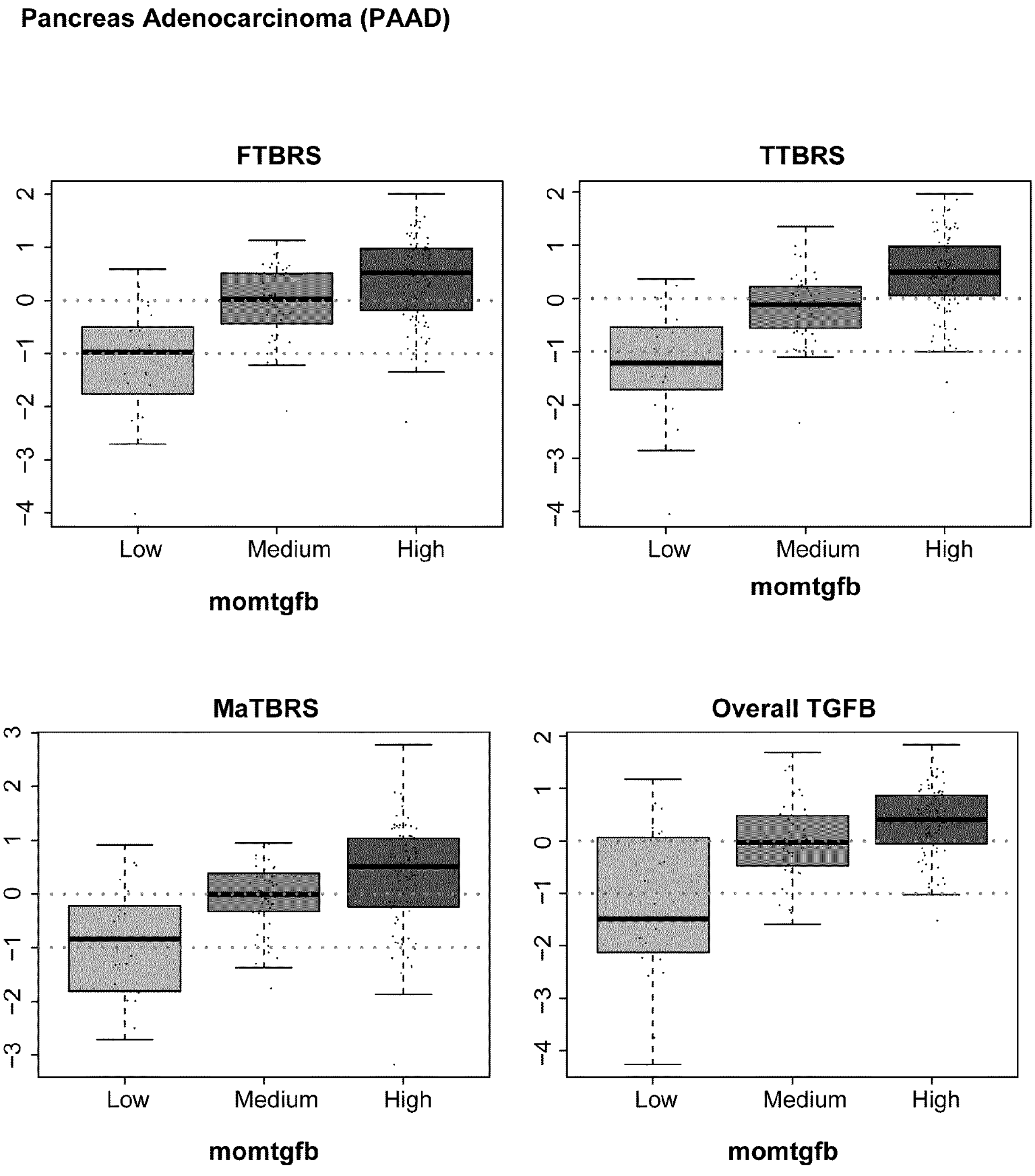
Pancreas Adenocarcinoma (PAAD)
FTBRS
TTBRS
MaTBRS
Overall TGFB
Low
Medium
High
momtgfb
2
1
0
−1
−2
−3
−4
3

FIG.11

Prostate Cancer

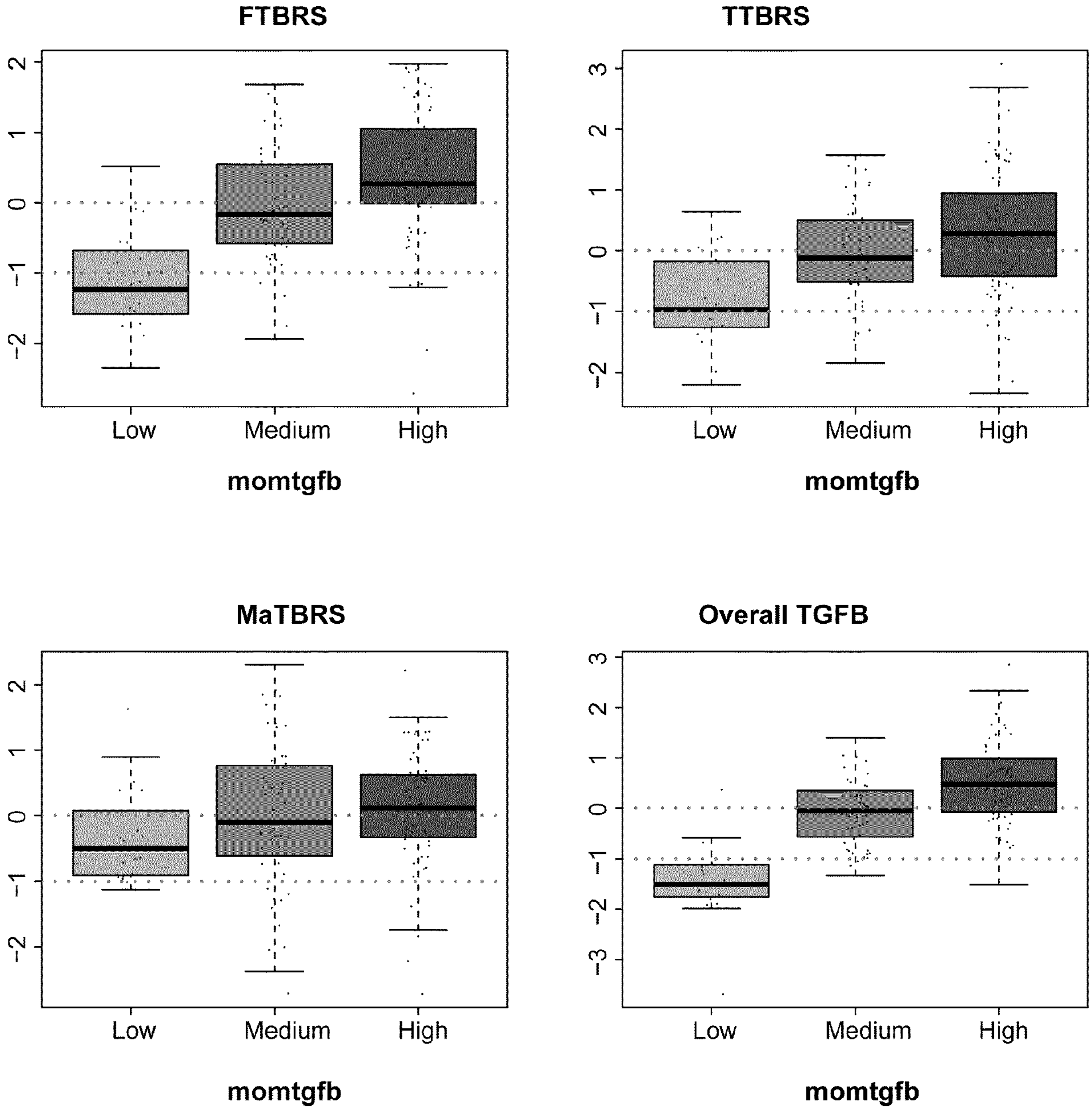

FIG.12

Lung Cancer

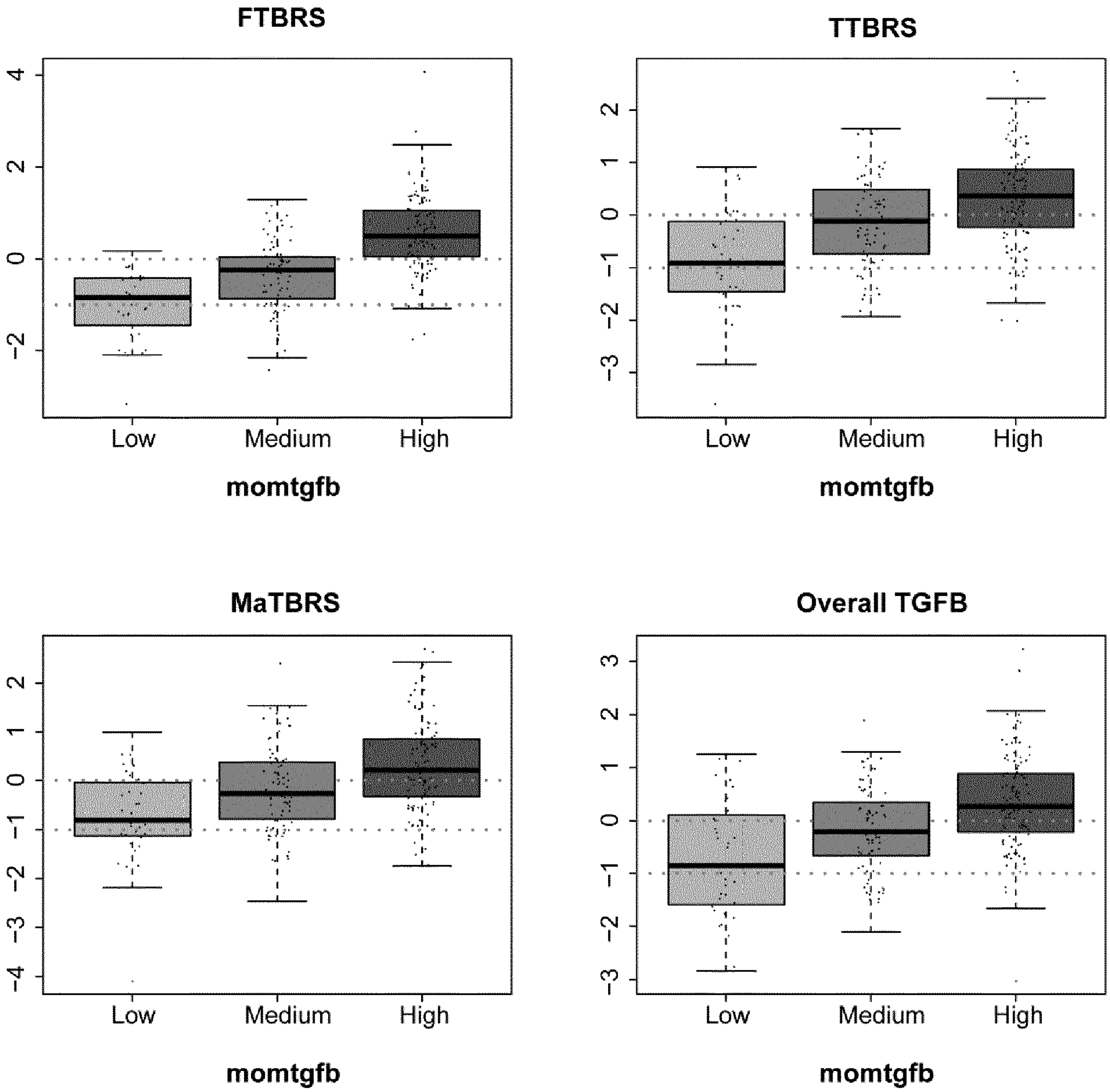

FIG.13

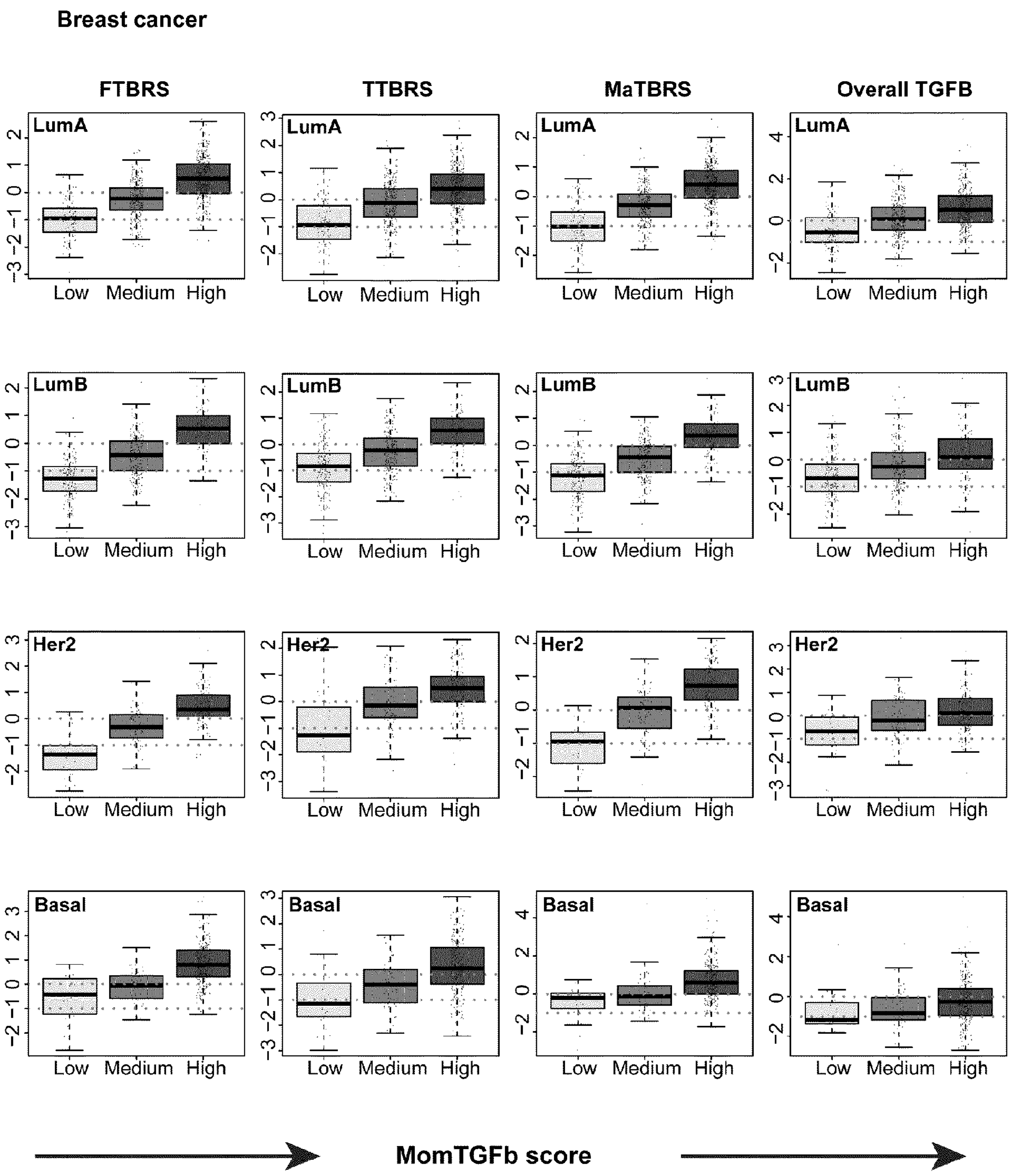
Breast cancer
FTBRS
TTBRS
MaTBRS
Overall TGFB
LumA
LumB
Her2
Basal
Low
Medium
High
MomTGFb score

FIG 14

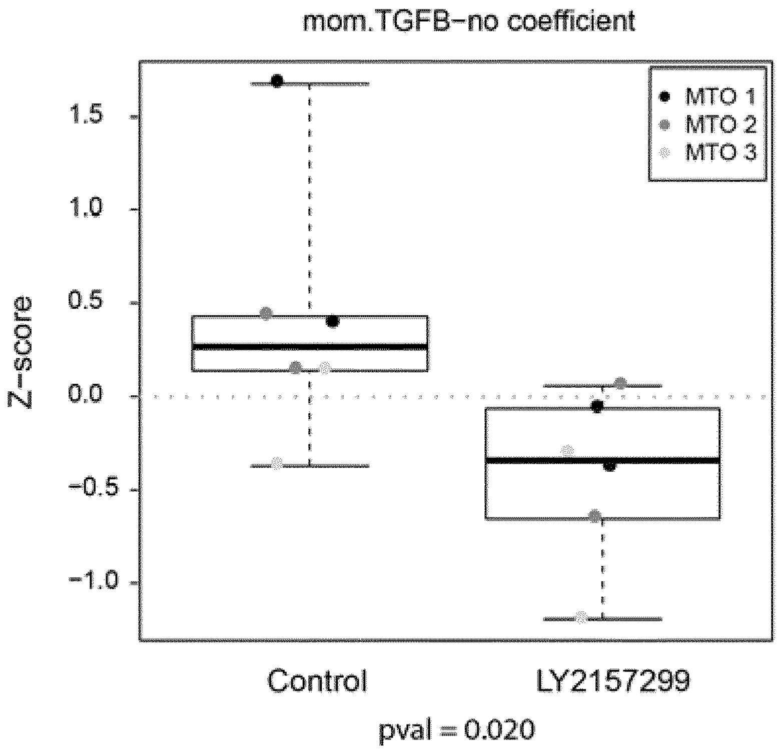
mom.TGFB−no coefficient
MTO 1
MTO 2
MTO 3
Z−score
1.5
1.0
0.5
0.0
−0.5
−1.0
Control
LY2157299
pval = 0.020

Europäisches Patentamt
European Patent Office
Office européen des brevets

# EUROPEAN SEARCH REPORT

Application Number
EP 19 38 2839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/072517 A1 (INSTITUCIÓ CATALANA DE RECERCA I ESTUDIS AVANÇATS [ES] ET AL.) 15 May 2014 (2014-05-15) * p14 last par; page 45 - page 46 * | 1-15 | INV. G01N33/574 |
| X | ALEXANDRE CALON ET AL: "Stromal gene expression defines poor-prognosis subtypes in colorectal cancer", NATURE GENETICS., vol. 47, no. 4, 23 February 2015 (2015-02-23), pages 320-329, XP055297456, NEW YORK, US ISSN: 1061-4036, DOI: 10.1038/ng.3225 * the whole document * * suppl info * | 1-15 | |

TECHNICAL FIELDS SEARCHED (IPC)

G01N

The present search report has been drawn up for all claims

1

| Place of search | Date of completion of the search | Examiner |
|---|---|---|
| The Hague | 18 March 2020 | Rosin, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 38 2839

This annex lists the patent family members relating to the patent documents cited in the above-mentioned European search report. The members are as contained in the European Patent Office EDP file on
The European Patent Office is in no way liable for these particulars which are merely given for the purpose of information.

18-03-2020

| Patent document cited in search report | Publication date | Patent family member(s) | Publication date |
|---|---|---|---|
| WO 2014072517 A1 | 15-05-2014 | EP 2917365 A1 | 16-09-2015 |
| | | US 2015289795 A1 | 15-10-2015 |
| | | WO 2014072517 A1 | 15-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100173786 A
- WO 2012155019 A
- US 20110039287 A


### Non-patent literature cited in the description


- **NEUZILLET et al.** *Pharmacology & Therapeutics,* 2015, vol. 147, 22-31
- **BATLLE ; MASSAGUE.** *Immunity,* 2019, vol. 50, 924-940
- **CALON et al.** *Cancer Cell,* 2012, vol. 22, 571-584
- **CALON et al.** *Nature Genetics,* 2015, vol. 47 (4), 320-332
- **GUINNEY et al.** *Nat Med.,* 2015, vol. 21 (11), 1350-6
- **GRAMONT et al.** *Oncoimmunology,* 2017, vol. 6 (1), e1257453
- **TAURIELLO et al.** *Nature,* 2018, vol. 554, 539-543
- **MARIATHASAN et al.** *Nature,* 2018, vol. 554, 544-548
- **LYONS et al.** *J.Cell.Biol.,* 1990, vol. 110 (4), 1361-1367
- **BATLLE ; MASSAGUÉ.** *Immunity,* 2019, vol. 50, 924-940
- **DAVID ; MASSAGUÉ.** *Nat Rev Mol Cell Biol.,* 2018
- **BATTAGLIN et al.** *Advances in Hematology and Oncology,* 2018, vol. 16 (11), 735-747
- **GALON et al.** *Science,* 2006, vol. 313, 1960-1964
- **CHAMBERS AF et al.** *Nat Rev Cancer,* 2002, vol. 2, 563-72
- **CUENDE et al.** *Science Translational Medicine,* 2015, vol. 7 (284), 284ra56
- **N. TAKASAKA et al.** *JCI Insight.,* 2018, vol. 3
- **BATLLE ; MASSAGUÉ.** *Immunity,* 2019, vol. 50 (4), 924-940
- Cell cycle regulation, oncogenes, and antineoplastic drugs. **MURAKAMI et al.** The Molecular Basis of Cancer. WB Saunders, 1995
- **WEIDLE et al.** *Cancer Genomics & Proteomics.,* 2013, vol. 10, 1-18
- **LOFBLOM, J. et al.** *Curr. Opin. Biotechnol.,* 2011, vol. 22, 843-848
- **BANTA, S. et al.** *Annu. Rev. Biomed. Eng.,* 2010, vol. 15, 93-113
- **NOLTE.** *Adv. Clin. Chem.,* 1998, vol. 33, 201-235
- **ANDOH et al.** *Current Pharmaceutical Design,* 2009, vol. 15, 2066-2073
- **GOODWIN S et al.** *Nat Rev Genet.,* 2016, vol. 17 (6), 333-51
- **WONG ML ; MEDRANO JF.** *Biotechniques,* 2005, vol. 39 (1), 75-85
- **E. NAVARRO.** *Clinica Chimica Acta,* 15 January 2015, vol. 439, 231-250
- **RODRIGUEZ A et al.** *Methods Mol Biol.,* 2015, vol. 1275, 31-56
- Molecular Cloning. **SAMBROOK ; FISCHER ; MANIATIS.** laboratory manual. Cold Spring Harbor Laboratory Press, 1989
- **M. RAUH.** *J Chromatogr B Analyt Technol Biomed Life Sci,* 01 February 2012, vol. 883-884, 59-67
- AJCC Cancer Staging Manual
- **UMAR, A. et al.** *J. Natl. Cancer Inst.,* 2004, vol. 96, 261-268
- **RATTI, M et al.** *Cell Mol Life Sci.,* 2018, vol. 75 (22), 4151-4162
- **VOGELSTEIN ; FEARON.** *Cell 1,* 1990, vol. 61 (5), 759-767
- **MANAVIS J. et al.** *Appl Immunohistochem Mol Morphol.,* March 2003, vol. 11 (1), 73-7
- **NOWAK J.A. et al.** *J Mol Diagn.,* 2017, vol. 19 (1), 84-91
- **DOTTI.** *Blood,* 2009, vol. 114 (8), 1457-58
- **BARBER et al.** *Nature,* 2006, vol. 439 (9), 682-687
- **CALON et al.** *Cancer cell,* 2012, vol. 22, 571-584
- **CALON et al.** *Nature genetics,* 2015, vol. 47 (4), 320-332
- Current Trends in Monoclonal Antibody Development. Springer, 2010
- **KOHLER ; MILSTEIN.** *Nature,* 1975, vol. 256, 495-497
- Antibodies; A Laboratory Manual. Cold Spring Harbor Laboratory, 1988

- **LAURELL et al.** *Nucleic Acids Research,* 2012, 1-10
- **BARRETT et al.** *Nucleic Acids Res,* 2013, vol. 41, D991-5
- **GUINNEY et al.** *Nature Medicine,* 2015, vol. 21, 1350-1356
- **GROSSMAN et al.** *New England Journal of Medicine,* 2016, vol. 375, 1109-1112
- **GAUTIER et al.** *Bioinformatics,* 2004, vol. 3, 307-315
- **GENTLEMAN et al.** *Genome Biology,* 2004, vol. 5, R80
- **IRIZARRY et al.** *Biostatistics,* 2003, vol. 4, 249-264
- **GENTLEMAN et al.** Bioinformatics and Computational Biology Solutions Using R and Bioconductor. Springer, 2005
- **EKLUND ; SZALLASI.** *Genome Biology,* 2008, vol. 9, R26
- **JORISSEN et al.** *Clinical Cancer Research,* 2008, vol. 14, 8061-8069
- **AZZALINI A. ; TORELLI N.S.** *Statistics and Computing,* 2007, vol. 17, 71-80
- **AZZALINI A. ; MENARDI G.** *Journal of Statistical Software,* 2014, vol. 57 (11), 1-26
- **CALON A et al.** *Cancer Cell,* 2012, vol. 22 (5), 571-84
- **NISHIDA N et al.** *Clin Cancer Res,* 2012, vol. 18, 3054-70
- **IRIZARRY RA et al.** *Biostatistics,* 2003, vol. 4, 249-64
- **RITCHIE, M.E. et al.** *Nucleic Acids Research,* vol. 43 (7), e47
- Controlling the False Discovery Rate: A Practical and Powerful Approach to Multiple Testing Yoav Benjamini and Yosef Hochberg Journal of the Royal Statistical Society. Series B. *Methodological,* 1995, vol. 57 (1), 289-300
- **TAYLOR et al.** *Cancer Cell,* 2010, vol. 18, 11-22
- **OKAYAMA et al.** *Cancer Research,* 2011, vol. 72, 100-111
- **CARVALHO BS ; IRIZARRY RA.** A Framework for Oligonucleotide Microarray Preprocessing. *Bioinformatics,* 2010, vol. 26 (19), ISSN 1367-4803, 2363-7
- **CURTIS et al.** *Nature,* 2012, vol. 486, 346-352
- **ADRIÀ CABALLÉ MESTRES ; ANTONIO BERENGUER-LLERGO ; CAMILLE STEPHAN-OTTO ATTOLINI.** *Adjusting for systematic technical biases in risk assessment of gene signatures in transcriptomic cancer cohorts*
- **BRADLEY EFRON ; ROBERT TIBSHIRANI.** On Testing the Significance of Sets of Genes. *The Annals of Applied Statistics,* June 2007, vol. 1 (1), 107-129
- **EILERS, PAUL H. ; MARX, BRIAN D.** *Statistical Science,* 1996, vol. 11, 89-121
- **EVARIST.** *Planet,* 2018
- An Integrated TCGA Pan-Cancer Clinical Data Resource to Drive High-Quality Survival Outcome Analytics. **LIU J ; LICHTENBERG T ; HOADLEY KA ; POISSON LM ; LAZAR AJ ; CHERNIACK AD ; KOVATICH AJ ; BENZ CC ; LEVINE DA ; LEE AV.** Cell. Cancer Genome Atlas Research Network, 05 April 2018, vol. 173, 400-416
- **TAURIELLO et al.** *Nature,* 2018, vol. 554, 538-543
- **EKLUND AC et al.** *Genome Biol.,* 2008, vol. 9 (2), R26
- **BLAKE JA et al.** *Nucleic Acids Res.,* 2017, vol. 45 (D1), D723-D729
- **CURTIS et al.** *Nature,* 2012, vol. 486 (7403), 346-52